(19) Europäisches Patentamt
European Patent Office
Office européen des brevets

(11) **EP 3 455 342 B1**

(12) **FASCICULE DE BREVET EUROPEEN**

(45) Date de publication et mention
de la délivrance du brevet:
**25.10.2023 Bulletin 2023/43**

(21) Numéro de dépôt: **17727301.8**

(22) Date de dépôt: **11.05.2017**

(51) Classification Internationale des Brevets (IPC):
**C12M 3/00** (2006.01)

(52) Classification Coopérative des Brevets (CPC):
**C12M 23/16; C12M 21/08; C12M 25/00; C12M 25/04**

(86) Numéro de dépôt international:
**PCT/FR2017/051148**

(87) Numéro de publication internationale:
**WO 2017/194894 (16.11.2017 Gazette 2017/46)**

(54) **PUCE DE CO-CULTURE CELLULAIRE ET SON PROCEDE DE FABRICATION**

ZELLCOKULTUR UND VERFAHREN ZUR HERSTELLUNG DAVON

CELL CO-CULTURE CHIP AND PROCESS FOR THE PRODUCTION THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.05.2016 FR 1654213**

(43) Date de publication de la demande:
**20.03.2019 Bulletin 2019/12**

(73) Titulaire: **UNIVERSITE GRENOBLE ALPES**
**38400 Saint Martin d'Hères (FR)**

(72) Inventeurs:
  • **MARTIN, Donald, Keith**
    **38610 Gieres (FR)**
  • **PICOLLET-D'HAHAN, Nathalie**
    **38580 La Ferriere (FR)**

(74) Mandataire: **Hautier IP**
    **20, rue de la Liberté**
    **06000 Nice (FR)**

(56) Documents cités:
**WO-A2-2011/014674     WO-A2-2011/094486**

• HYUN JUNG KIM ET AL: "Gut-on-a-Chip microenvironment induces human intestinal cells to undergo villus differentiation", INTEGRATIVE BIOLOGY, vol. 5, no. 9, 1 janvier 2013 (2013-01-01), page 1130, XP055326242, UK ISSN: 1757-9694, DOI: 10.1039/c3ib40126j
• Jose Manuel ET AL: "Delft Institute for Microsystems and Nanoelectronics Fabrication of a microporous PDMS membrane for an Organ-on-Chip device", , 1 juillet 2014 (2014-07-01), XP055326354, Extrait de l'Internet: URL:http://repository.tudelft.nl/assets/uu id:9f9874cd-91ec-47ab-abf4-70bc871aaa22/MS c_thesis_JM_Rosas_4254872.pdf [extrait le 2016-12-06]
• MANDY BRIGITTE ESCH ET AL: "On chip porous polymer membranes for integration of gastrointestinal tract epithelium with microfluidic 'body-on-a-chip' devices", BIOMEDICAL MICRODEVICES, KLUWER ACADEMIC PUBLISHERS, BO, vol. 14, no. 5, 31 juillet 2012 (2012-07-31), pages 895-906, XP035113965, ISSN: 1572-8781, DOI: 10.1007/S10544-012-9669-0
• ROLAND THUENAUER ET AL: "Microfluidic approaches for epithelial cell layer culture and characterisation", THE ANALYST, vol. 139, no. 13, 1 janvier 2014 (2014-01-01), pages 3206-3218, XP055320530, GB ISSN: 0003-2654, DOI: 10.1039/C4AN00056K

## Description

La présente invention concerne une puce microfluidique de culture cellulaire comprenant plusieurs parties, désignées chacune par le terme « module », et susceptibles d'être assemblées deux à deux.

La présente invention concerne également un procédé de fabrication de ladite puce, ainsi que ses utilisations dans le domaine du diagnostic.

De nombreux dispositifs actuels développés dans le but de mimer des organes sur une puce, sont constitués de membranes en 2D.

Dans le brevet US 8 647 861 B2 de Ingber et al., déposé le 13 Octobre 2011, les auteurs ont mis au point un dispositif organomimétique permettant une co-culture de cellules adhérentes de part et d'autre d'une membrane, doté de moyens permettent de créer une pression différentielle aux abords de la membrane provoquant ainsi l'expansion ou la rétraction de celle-ci, afin de reproduire les contraintes mécaniques existant in vivo à une interface tissu-tissu. Ce dispositif, basé sur un système microfluidique comprenant une membrane poreuse flexible en polydiméthylsiloxane (PDMS), à l'interface de deux canaux microfluidiques permettant la circulation de fluides le long de ladite membrane, permet notamment de mimer les alvéoles pulmonaires pendant la respiration.

Dans la demande US 2014/0038279 A1 de Ingber et al., déposée le 28 février 2012, les auteurs proposent un système de co-culture cellulaire permettant reproduire les structures épithéliales intestinales naturelles et mimer leur comportement. Ce dispositif organomimétique, basé sur un système microfluidique, comporte une membrane poreuse en PDMS, positionnée à l'interface de deux canaux microfluidiques permettant la circulation de fluides le long de ladite membrane, et sur laquelle est cultivée une couche de cellules épithéliales intestinales sur au moins l'un des côtés. Le couplage de la membrane à des éléments de support entraine un mouvement d'étirement de la membrane dans au moins une dimension, et permet ainsi de reproduire le mouvement des tissus *in vivo.*

Même si ces dispositifs permettent de recréer des interfaces tissu-tissu ainsi que les contraintes mécaniques existant *in vivo,* pour mimer la microarchitecture des organes, la co-culture cellulaire demeure sur un milieu en 2D, et ne permettent pas de reproduire la topographie de certains organes.

En 2013, Kim et al. , dans leur publication intitulée « Gut-on-a-Chip microenvironment induces human intestinal cells to undergo villus differenciation », Integr. Biol., 5, 1130, proposent une puce microfluidique organomimétiques permettant ainsi de reproduire les villosités intestinales en 3D, à l'image de celles formées *in vivo* dans l'intestin. Cette puce, basée sur un système microfluidique, comporte une membrane poreuse en PDMS, positionnée à l'interface de deux canaux microfluidiques permettant la circulation de fluides le long de ladite membrane, et sur laquelle sont cultivées des cellules épithéliales intestinales Caco-2 qui forment spontanément des villosités au-delà de deux jours de culture en présence d'un flux constant de milieu de 30 μL/h.

Tous ces dispositifs proposent cependant une co-culture cellulaire sur une membrane poreuse en PDMS, qui n'est pas optimale en termes de biocompatibilité (inertie, porosité). Le PDMS peut absorber des petits composants organiques, aussi des drogues et a une perméabilité aux gaz pouvant gêner certaines applications. Par ailleurs, les membranes en PDMS peuvent avoir des propriétés de transport, mécaniques et structurales intrinsèques qui sont différentes de celles la membrane basale naturelle du tissu. En recréant ce type d'interfaces, les auteurs privilégient la reproduction de la microarchitecture de l'organe.

En 2014, March et al. , dans leur publication intitulé « Differentiating Intestinal Stem Cells in a 3D Niche », proposent un modèle d'intestin *in vitro* utilisant des structures en acide glycolique poly-lactique (PLGA) ayant la morphologie 3D des microvillosités et aptes à supporter une co-culture de différents types de cellules épithéliales à leur surface, telles qu'une co-culture de cellules épithéliales intestinales Caco-2 et HT29-MTX. Ce modèle permet d'obtenir une différenciation cellulaire à la surface des microvillosités d'une manière similaire aux intestins in vivo.

Ainsi, même si certains dispositifs actuels présentent des structures, en PDMS ou en PGLA, permettant de mimer les microvillosités des organes, la détection de marqueurs connus se fait par immunofluorescence après fixation des cellules et marquage à l'aide d'un anticorps, ce qui implique donc la lyse des cellules pour l'analyse.

De nombreuses publications font état que des cellules en culture sur des structures 3D n'ont pas les mêmes propriétés que les mêmes cellules cultivées en 2D ; des différences sont en effet observées dans le profil d'expression de gènes et de protéines, l'adhésion des cellules, la vitesse de prolifération et la différenciation cellulaire. De plus, la réponse métabolique aux drogues d'un type cellulaire est différente selon que les cellules sont cultivées en 2D et en 3D, comme l'ont montré Kim et al. en 2013 dans leur publication intitulée « Gut-on-a-Chip microenvironment induces human intestinal cells to undergo villus differenciation », Integr. Biol., 5, 1130, où ils réalisent une comparaison, entre le modèle de culture 2D Transwell® et un modèle de culture comportant des structures 3D mimant les microvillosités, de l'activité de l'enzyme cytochrome P450 métabolisant les drogues, et montrent qu'en 2D l'activité reste inchangée au cours de la culture, tandis qu'en 3D cette activité augmente lors de la formation des microvillosités puis se stabilise.

La topographie du milieu cellulaire ayant un impact non négligeable sur le comportement des cellules, il est donc important de reproduire le plus précisément possible les conditions in vivo des cellules, afin de mettre au point des modèles fiables de culture cellulaire.

Dans la présente invention, les Inventeurs ont mis au point une puce microfluidique de co-culture cellulaire, mimant les microvillosités d'organes avec une structure acinaire/tubullaire et le microenvironnement luminal.

La puce microfluidique selon l'invention comprend une membrane en biomatériaux poreux qui comporte des replis formant ainsi des protubérances creuses en 3D sur lesquelles sont cultivées des cellules adhérentes de part et d'autre de ladite membrane au niveau des protubérances. Le couplage de cette membrane à un système microfluidique permet de récupérer les sécrétions cellulaires au cours de leur culture pour en analyser la composition.

Ainsi, contrairement à l'art antérieur, où les marqueurs sont détectés par un immunomarquage sur cellules fixées impliquant donc la mort des cellules (Kim *et al.,* 2013), la puce selon l'invention permet de réaliser ces détections dans les sécrétions des cellules cultivées sur la protubérance, qui sont récupérées par le système micro fluidique.

La puce selon l'invention permet ainsi de collecter des sécrétions sur cellules vivantes et par conséquent de collecter les sécrétions à des temps différents au cours de la culture, voire sur plusieurs jours pour la réalisation d'études cinétiques, tout en maintenant la viabilité et la fonctionnalité des cellules au cours du temps.

Ces sécrétions sont constituées de l'ensemble des molécules pouvant être sécrétées dans le milieu de culture cellulaire tel que les peptides, les protéines, des acides aminés, des miARN, ADN, ARN.

Le profil d'analyse de ces sécrétions permet d'obtenir le sécrétome, c'est-à-dire le profil qualitatif et quantitatif des composants des sécrétions.

La présente invention concerne une puce microfluidique de culture cellulaire qui contient un module central (104) comprenant :

- une unité centrale (105), laquelle contient

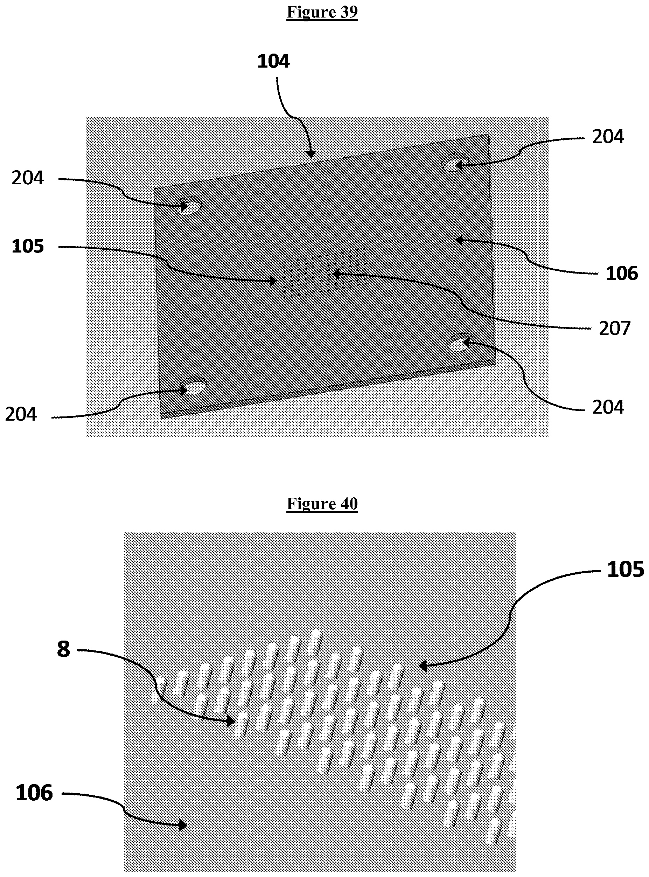

  - un support constitué d'une membrane non résorbable (1), comportant une face supérieure (2) et une face inférieure (3), perforé par au moins une perforation (4),
  - une membrane poreuse nanostructurée en 3D (5), comportant une face supérieure (6) et une face inférieure (7), et comportant au moins une protubérance (8), ladite au moins une protubérance comportant une face externe (9) et une face interne (10) et formant une structure en relief du coté de la face supérieure (6) de la membrane nanostructurée en 3D (5) **(Figure 40),**

    ladite face inférieure (7) de ladite membrane poreuse nanostructurée en 3D (5) étant positionnée de façon solidaire sur ladite face supérieure (2) dudit support (1),

ou ladite face supérieure (6) de la membrane nanostructurée en 3D (5) étant positionnée de façon solidaire sur ladite face inférieure (3) dudit support (1),

et ladite membrane poreuse nanostructurée en 3D (5) et la au moins une protubérance (8) étant composées de matériaux appropriés pour la culture de deux types de cellules distincts ;

- un socle (106),

ladite unité centrale (105) étant intégrée dans ledit socle (106), et formant un tout avec ledit socle (106) **(Figures 39 et 40).**

L'expression « **membrane non résorbable** » signifie que la membrane ne peut être éliminée ni par un procédé physique ni par un procédé chimique dans un solvant aqueux.

L'expression « **perforation** » qui se rapporte au support, désigne un évidement traversant le support de part en part, c'est-à-dire une ouverture à travers le support entre la face inférieure et la face supérieure, permettant ainsi la communication entre ces deux faces.

Cette perforation dudit support crée un espace vide à travers ledit support, et est caractérisée par une section au niveau de la face supérieure dudit support et une section au niveau de la face inférieure dudit support.

L'expression « **protubérance** » qui se rapporte à la membrane poreuse nanostructurée en 3D désigne une saillie proéminente formant une structure en relief, du côté de la face supérieure de ladite membrane poreuse nanostructurée en 3D.

L'expression « **poreuse** » qui se rapporte à la membrane poreuse nanostructurée en 3D signifie que ladite membrane comporte des pores continus interconnectés entre eux, d'un diamètre compris entre 2 et 10 nm, s'étendant de la face supérieure à la face inférieure de ladite membrane. Ces pores permettant les échanges de gaz à travers la membrane, ainsi que de petites molécules contenues dans le milieu de culture (facteurs de croissance, protéines sériques, éléments nutritifs assurant la viabilité des cellules teks que les ions $Ca^{2+}$, $K^+$, $Na^+$...). Ces pores permettent également de laisser passer des molécules pharmacologiques telles que des inhibiteurs ou des anti-cancéreux, des petits ARN (par exemple des ARN interférents), des hormones (par exemple la dihydrotestostérone).

L'expression « **nanostructurée en 3D** » qui se rapporte à la membrane poreuse désigne la présence d'au moins une structure en relief, soit en 3 dimensions, sur la face supérieure de ladite membrane poreuse, l'échelle de ladite structure étant de l'ordre du nanomètre.

L'expression « **de façon solidaire** » qui désigne la nature de la liaison entre ledit support et ladite membrane, signifie que des liaisons chimiques ainsi que des interactions hydrophobes et électrostatiques sont établies entre ledit support et ladite membrane afin de lier ladite membrane et ledit support de manière cohésive par une connexion étanche.

Le terme « **socle** » désigne la partie solide dans laquelle est intégrée ladite unité.

L'expression « **formant un tout** » utilisée pour caractériser l'agencement entre le socle et l'unité, signifie que le socle et l'unité forme ensemble une pièce d'un seul tenant, et ne sont pas dissociables l'un de l'autre.

Selon un mode de réalisation particulier, l'invention concerne une puce microfluidique de culture cellulaire qui contient un module central (104) comprenant :

- une unité centrale (105), laquelle contient

  - un support constitué d'une membrane non résorbable (1), comportant une face supérieure (2) et une face inférieure (3), perforé par au moins une perforation (4),
  - une membrane poreuse nanostructurée en 3D (5), comportant une face supérieure (6) et une face inférieure (7), et comportant au moins une protubérance (8), ladite au moins une protubérance comportant une face externe (9) et une face interne (10) et formant une structure en relief du coté de la face supérieure (6) de la membrane nanostructurée en 3D (5) **(Figure 40)**,

    ladite face inférieure (7) de ladite membrane poreuse nanostructurée en 3D (5) étant positionnée de façon solidaire sur ladite face supérieure (2) dudit support (1),

    et ladite membrane poreuse nanostructurée en 3D (5) et la au moins une protubérance (8) étant composées de matériaux appropriés pour la culture de deux types de cellules distincts ;

- un socle (106),

ladite unité centrale (105) étant intégrée dans ledit socle (106), et formant un tout avec ledit socle (106) **(Figures 39 et 40)**.

Selon un mode de réalisation particulier, l'invention concerne une puce microfluidique de culture cellulaire qui contient un module central (104) comprenant :

- une unité centrale (105), laquelle contient

  - un support constitué d'une membrane non résorbable (1), comportant une face supérieure (2) et une face inférieure (3), perforé par au moins une perforation (4),
  - une membrane poreuse nanostructurée en 3D (5), comportant une face supérieure (6) et une face inférieure (7), et comportant au moins une protubérance (8), ladite au moins une protubérance comportant une face externe (9) et une face interne (10) et formant une structure en relief du coté de la face supérieure (6) de la membrane nanostructurée en 3D (5),

    ladite face supérieure (6) de la membrane nanostructurée en 3D (5) étant positionnée de façon solidaire sur ladite face inférieure (3) dudit support (1),

    et ladite membrane poreuse nanostructurée en 3D (5) et la au moins une protubérance (8) étant composées de matériaux appropriés pour la culture de deux types de cellules distincts ;

- un socle (106),

ladite unité centrale (105) étant intégrée dans ledit socle (106), et formant un tout avec ledit socle (106).

Selon un mode de réalisation particulier, l'invention concerne une puce microfluidique de culture cellulaire dans laquelle ladite au moins protubérance (8) de la membrane poreuse nanostructurée de ladite puce microfluidique de culture cellulaire, est en forme de dôme creux et présente une base circulaire (13).

L'expression « **en forme de dôme**» qui se rapporte à la forme de la protubérance signifie que ladite protubérance a une structure arrondie en forme de voûte avec une base circulaire, formant une convexité du côté de la face supérieure dudit support.

Dans un autre mode de réalisation, ladite protubérance peut avoir une structure arrondie en forme de voûte avec une base ovale, formant une convexité du côté de la face supérieure dudit support.

L'expression « **en forme de dôme creux**» signifie que la face interne de ladite protubérance délimite un volume vide car ladite protubérance correspond à un renfoncement de ladite membrane poreuse nanostructurée en 3D du côté de la face inférieure de celle-ci, ledit renfoncement formant ainsi une concavité du côté de la face inférieure de ladite membrane, et donc un volume vide.

L'expression « **base circulaire** » qui se rapporte au dôme creux, désigne la surface inférieure vide sur laquelle repose la protubérance en forme de dôme et délimitée par la face interne de ladite protubérance.

Selon un mode de réalisation particulier, l'invention concerne une puce microfluidique de culture cellulaire dans laquelle ladite au moins une perforation du support est caractérisée par une section circulaire au niveau de la face supérieure dudit support et une section circulaire au niveau de la face inférieure dudit support.

Selon un mode de réalisation particulier, l'invention concerne une puce microfluidique de culture cellulaire dans laquelle ladite au moins une perforation (4) du support (1) présente

une section (11) au niveau de la face supérieure (2) du support (1) (section supérieure de la perforation) présentant un axe (x) passant par le centre de ladite section supérieure (11) de la perforation (4) perpendiculaire audit support (1),

et une section (12) au niveau de la face inférieure (3) du support (1) (section inférieure de la perforation) présentant un axe (w) passant par le centre de ladite section inférieure (12) de la perforation (4) et perpendiculaire audit support (1),

et dans laquelle lesdits axes (x) et (w) sont confondus.

Selon un mode de réalisation particulier, l'invention concerne une puce microfluidique de culture cellulaire dans laquelle le diamètre d2 de la section circulaire de ladite perforation au niveau de la face inférieure du support, est supérieure ou égal au diamètre d1 de la section circulaire de ladite perforation au niveau de la face supérieure du support.

Selon un mode de réalisation particulier, l'invention concerne une puce microfluidique de culture cellulaire dans laquelle ladite au moins une perforation (4) du support (1) présente

une section (11) de forme circulaire au niveau de la face supérieure du support (1) (section supérieure de la perforation) présentant un diamètre d1 (diamètre supérieur d1 de la perforation) et un axe (x) passant par le centre de ladite section supérieure (11) de la perforation (4) et perpendiculaire audit support (1),

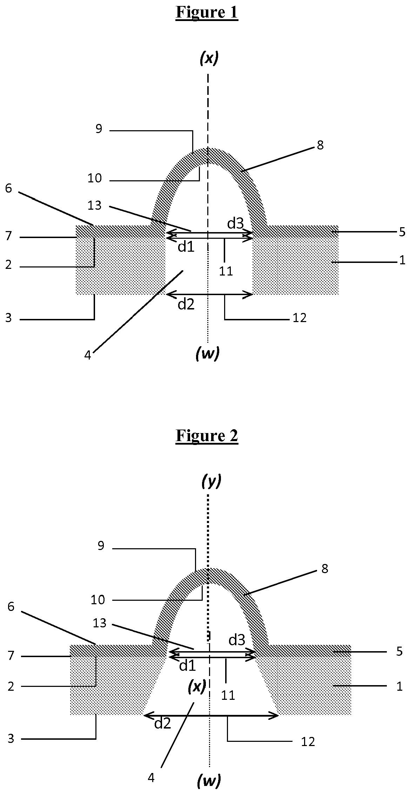

et une section (12) de forme circulaire au niveau de la face inférieure (3) du support (1) (section inférieure de la perforation) présentant un diamètre d2 (diamètre inférieur d2 de la perforation) et un axe (w) passant par le centre de ladite section inférieure de la perforation et perpendiculaire audit support (1),

le diamètre d1 étant d'une valeur supérieure ou égale à 10 μm à une valeur inférieure ou égale à 500 μm, préférablement d'une valeur de 150 μm, et le diamètre d2 étant d'une valeur supérieure ou égale à 10 μm à une valeur inférieure ou égale à 500 μm, préférablement d'une valeur de 150 μm,

tel que la valeur du diamètre d2 est supérieure ou égale à la valeur du diamètre d1,

et dans laquelle lesdits axes (x) et (w) sont confondus **(Figures 1 et 2)**.

Selon un mode de réalisation particulier, l'invention concerne une puce microfluidique de culture cellulaire dans laquelle le diamètre d2 de la section circulaire de ladite perforation au niveau de la face inférieure du support, est supérieur au diamètre d1 de la section circulaire de ladite perforation au niveau de la face supérieure du support.

Selon un mode de réalisation particulier, l'invention concerne une puce microfluidique de culture cellulaire dans laquelle le diamètre d2 de la section circulaire de ladite perforation au niveau de la face inférieure du support, est supérieur au diamètre d1 de la section circulaire de ladite perforation au niveau de la face supérieure du support, tel que le ratio (valeur de d1/ valeur de d2) est compris d'une valeur de 0.3 à une valeur inférieure à 1.

Selon un mode de réalisation particulier, l'invention concerne une puce microfluidique de culture cellulaire dans laquelle le diamètre d2 de la section circulaire de ladite perforation au niveau de la face inférieure du support, est supérieur au diamètre d1 de la section circulaire de ladite perforation au niveau de la face supérieure du support, et ladite perforation dudit support est en forme de cône tronqué.

Selon un mode de réalisation particulier, l'invention concerne une puce microfluidique de culture cellulaire dans laquelle la valeur du diamètre supérieur d1 de ladite au moins une perforation (4) est inférieure à la valeur du diamètre inférieur d2 de ladite au moins une perforation (4), et ladite perforation (4) est en forme de cône tronqué (Figure 2).

Selon un mode de réalisation particulier, l'invention concerne une puce microfluidique de culture cellulaire dans laquelle le diamètre d2 de la section circulaire de ladite perforation au niveau de la face inférieure du support, est égal au diamètre d1 de la section circulaire de ladite perforation au niveau de la face supérieure du support.

Selon un mode de réalisation particulier, l'invention concerne une puce microfluidique de culture cellulaire dans laquelle le diamètre d2 de la section circulaire de ladite perforation au niveau de la face inférieure du support, est égal au diamètre d1 de la section circulaire de ladite perforation au niveau de la face supérieure du support, et ladite perforation dudit support est de forme cylindrique.

Dans ce mode de réalisation, la perforation dudit support est donc de forme régulière c'est-à-dire que les diamètres de l'ensemble de ses sections sont de longueurs égales, et que lesdites sections sont de forme circulaire.

Selon un mode de réalisation particulier, l'invention concerne une puce microfluidique de culture cellulaire dans laquelle la valeur du diamètre supérieur d1 de ladite au moins une perforation (4) est égale à la valeur du diamètre inférieur d2 de ladite au moins une perforation (4), et ladite perforation (4) est de forme cylindrique **(Figure 1)**.

Selon un mode de réalisation particulier, l'invention concerne une puce microfluidique de culture cellulaire dans laquelle ladite protubérance (8) présente une base circulaire (13) présente de diamètre d3 (diamètre d3 de la base circulaire de la protubérance), ledit diamètre d3 de la protubérance (8) étant d'une valeur de 10 $\mu$m à une valeur de 500 $\mu$m.

Selon un mode de réalisation particulier, l'invention concerne une puce microfluidique de culture cellulaire dans laquelle la valeur du diamètre d3 de ladite au moins une protubérance (8) est égale à la valeur du diamètre supérieur d1 de ladite au moins une perforation (4) **(Figures 1 et 2)**.

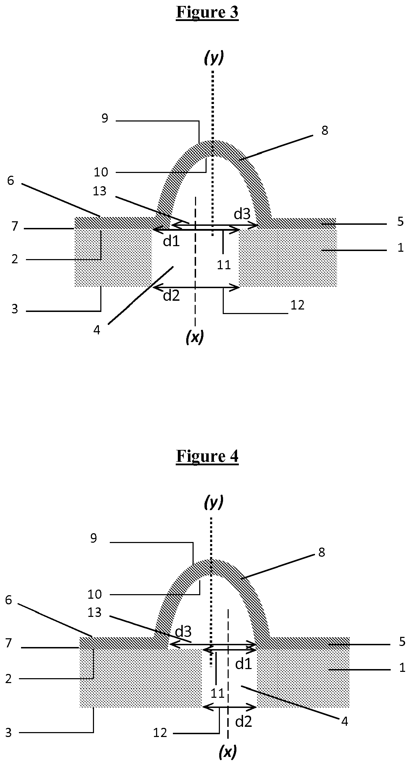

Selon un mode de réalisation particulier, l'invention concerne une puce microfluidique de culture cellulaire dans laquelle la valeur du diamètre d3 de ladite au moins une protubérance (8) est égale à la valeur du diamètre supérieur d1 de ladite au moins une perforation et est égale à la valeur du diamètre inférieur d2 de ladite au moins une perforation (4) **(Figures 1 et 3)**.

Selon un mode de réalisation particulier, l'invention concerne une puce microfluidique de culture cellulaire dans laquelle la valeur du diamètre d3 de ladite au moins une protubérance (8) est supérieure à la valeur du diamètre supérieur d1 de ladite au moins une perforation (4) **(Figures 4, 5 et 6)**.

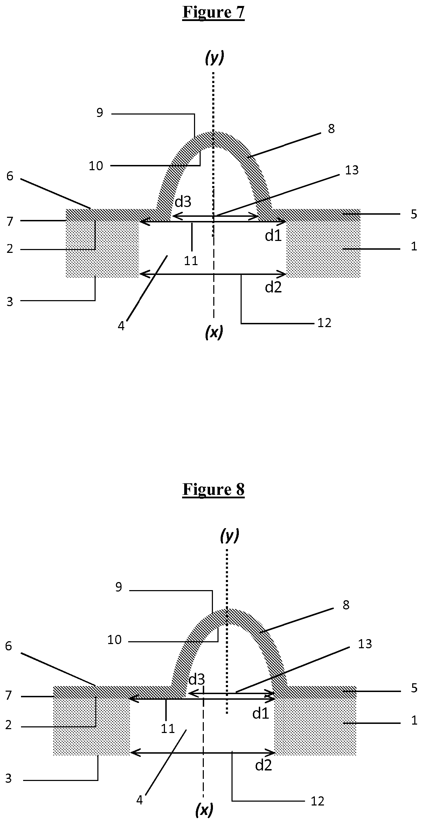

Selon un mode de réalisation particulier, l'invention concerne une puce microfluidique de culture cellulaire dans laquelle la valeur du diamètre d3 de ladite au moins une protubérance (8) est inférieure à la valeur du diamètre supérieur d1 de ladite au moins une perforation (4) **(Figures 7, 8, 9, 10 et 11)**.

Selon un mode de réalisation particulier, l'invention concerne une puce microfluidique de culture cellulaire dans laquelle ladite face interne (10) de ladite au moins une protubérance (8) est en totalité ou en partie en regard de ladite au moins une perforation (4) dudit support (1).

La membrane nanostructurée en 3D comportant au moins une protubérance et le support perforé par au moins une perforation sont positionnés l'un par rapport de façon à ce que la face interne de la dite protubérance soit en totalité ou en partie en regard de la section circulaire de ladite perforation au niveau de la face supérieure du support.

Selon un mode de réalisation particulier, l'invention concerne une puce microfluidique de culture cellulaire dans laquelle ladite face interne (10) de ladite au moins une protubérance (8) est **en totalité en regard de ladite au moins une perforation** (4) dudit support (1) :

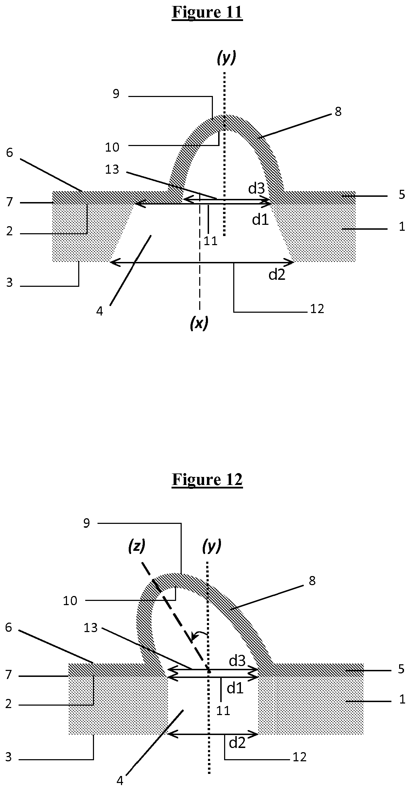

-   lorsque la valeur du diamètre supérieur d1 de ladite au moins une perforation (4) est **supérieure ou égale** à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus (Figures 1, 2, 7 et 9)**; ou
-   lorsque la valeur du diamètre supérieur d1 de ladite au moins une perforation (4) est **supérieure** à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure ou égale à [(valeur de d1 - valeur de d3)/2] **(Figures 8 et 11)**.

Le terme « **confondus** » utilisé pour caractériser les axes (x) et (y) signifie que la distance entre ces deux axes est égale à zéro.

Le terme « **distincts** » utilisé pour caractériser les axes (x) et (y) signifie que la distance entre ces deux axes est strictement supérieure à zéro.

Selon un mode de réalisation particulier, l'invention concerne une puce microfluidique de culture cellulaire dans laquelle ladite face interne (10) de ladite au moins une protubérance (8) est **en partie en regard de ladite au moins une perforation (4)** dudit support (1) :

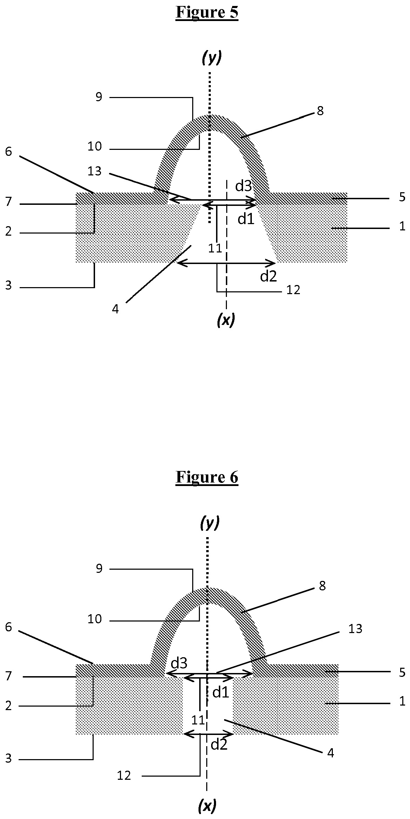

-   lorsque la valeur du diamètre supérieur d1 de ladite au moins une perforation (4) est **inférieure** à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus (Figure 6)**; ou

-   lorsque la valeur du diamètre supérieur d1 de ladite perforation (4) est **égale ou inférieure à** la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure à la valeur de d3 **(Figures 3, 4 et 5)**; ou

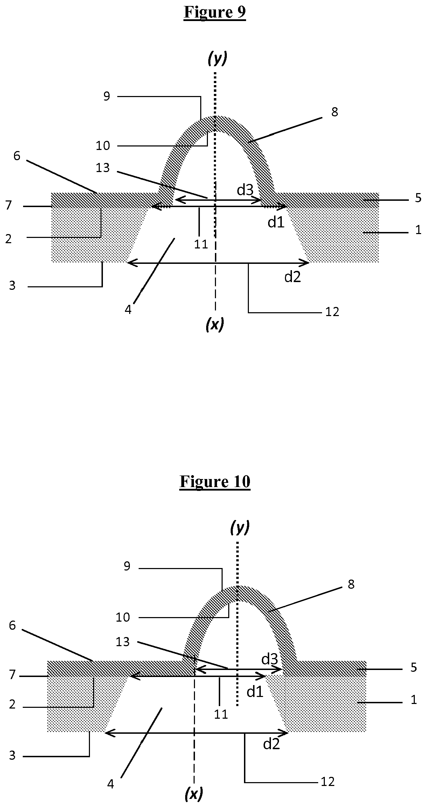

-   lorsque la valeur du diamètre supérieur d1 de ladite perforation (4) est **supérieure** à la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur supérieure à [(valeur de d1 - valeur de d3)/2] à une valeur inférieure à [[valeur de d1 + valeur de d3] /2] **(Figure 10)**.

Selon un mode de réalisation particulier, l'invention concerne une puce microfluidique de culture cellulaire dans laquelle la protubérance (4) présente une déformation oblique, ladite déformation oblique étant définie par l'angle $\alpha$ formé entre

- l'axe (z) passant par le centre de la base circulaire (13) de diamètre d3, délimitée par ladite protubérance (8) au niveau de la face supérieure (2) dudit support (1), et par le sommet de ladite protubérance (8),
- et l'axe (y) passant par le centre de ladite base circulaire (13) et qui est perpendiculaire audit support (1) **(Figure 12).**

Selon un mode de réalisation particulier, l'invention concerne une puce microfluidique de culture cellulaire dans laquelle l'angle $\alpha$ formé entre l'axe (y) et l'axe (z) est compris entre 0 et 45°.

Selon un mode de réalisation particulier, l'invention concerne une puce microfluidique de culture cellulaire dans laquelle l'angle $\alpha$ formé entre l'axe (y) et l'axe (z) est compris entre 0 et 60°.

Si l'angle $\alpha$ formé entre l'axe (y) et l'axe (z) est supérieur à 45°, l'inclinaison de la protubérance bloque partiellement la récupération des sécrétions. La valeur maximale théorique que peut prendre l'angle $\alpha$ est de 90°, mais dans ce mode de réalisation, la protubérance est complètement rabattue le long de la face supérieure de ladite membrane nanostructurée en 3D, et la récupération des sécrétions est bloquée.

Selon un mode de réalisation particulier, l'invention concerne une puce microfluidique de culture cellulaire dans laquelle la face externe (9) de ladite protubérance (8) supporte au moins une cellule adhérente à ladite face externe, et dans laquelle la face interne (10) de ladite protubérance (8) supporte au moins une cellule adhérente à ladite face interne (10), les au moins deux cellules appartenant à deux types de cellules distincts.

Le terme de « **cellules adhérentes** » désigne tout type de cellules dont la croissance nécessite une adhésion à un support et pour lesquelles le détachement audit support nécessite un traitement mécanique ou enzymatique (par exemple avec de la trypsine).

Le terme de « **types de cellules distincts** » est utilisé pour désigner des types de cellules de nature, de fonction différentes ou provenant de tissus différents.

Selon un mode de réalisation particulier, l'invention concerne une puce microfluidique de culture cellulaire dans laquelle ladite cellule adhérente à ladite face externe est une cellule stromale, et ladite cellule adhérente à ladite face interne est une cellule épithéliale.

Selon un mode de réalisation particulier, l'invention concerne une puce microfluidique de culture cellulaire dans laquelle ladite au moins une cellule adhérente à ladite face externe (9) est une cellule stromale, plus particulièrement un fibroblaste, et ladite au moins une cellule adhérente à ladite face interne (10) est une cellule épithéliale.

Selon un mode de réalisation particulier, l'invention concerne une puce microfluidique de culture cellulaire dans laquelle ladite cellule adhérente à ladite face externe est une cellule épithéliale, et ladite cellule adhérente à ladite face interne est une cellule stromale.

Les cellules épithéliales peuvent être des lignées cellulaires commerciales non tumorigènes de prostate, vessie ou rein, ou des cultures primaires commerciales.

Les cellules stromales peuvent être des fibroblastes (cultures primaires commerciales ou lignées), des cellules mésenchymateuses (cultures commerciales ou lignées), ou d'autres cellules stromales telles que les cellules endothéliales.

Les deux types de cellules cultivés sur les faces internes et externes de ladite protubérance, sont dites « neutres » ou saines, c'est-à-dire qu'elles sont non tumorigènes.

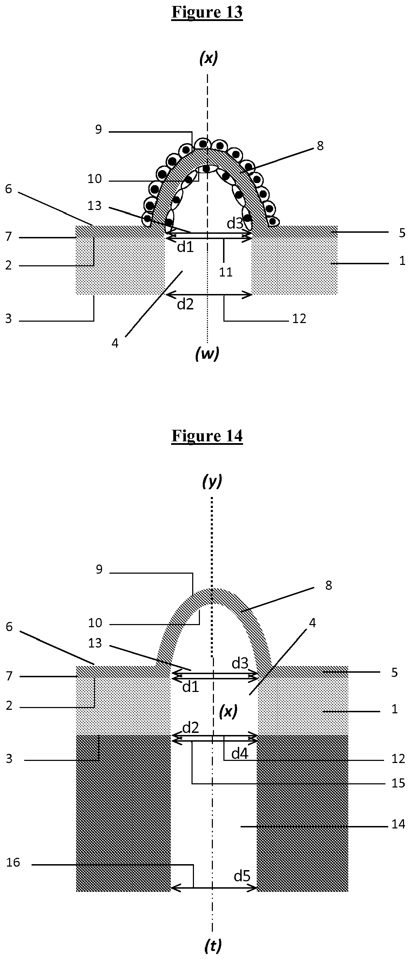

Selon un mode de réalisation particulier, l'invention concerne une puce microfluidique de culture cellulaire dans laquelle la face externe (9) de ladite protubérance (8) supporte un premier ensemble de cellules adhérentes au stade de la confluence, et dans laquelle la face interne (10) de ladite protubérance (8) supporte un deuxième ensemble de cellules adhérentes au stade de la confluence, les cellules du premier et du deuxième ensemble de cellules adhérentes appartenant à deux types cellulaires distincts **(Figure 13).**

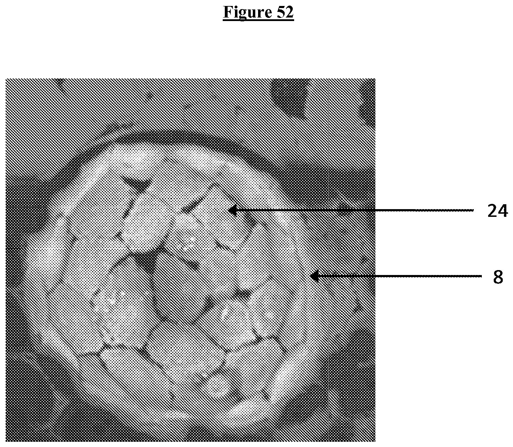

Selon un mode de réalisation particulier, l'invention concerne une puce microfluidique de culture cellulaire dans laquelle la face externe (9) de ladite protubérance (8) supporte un premier ensemble de cellules adhérentes avantageusement au stade de la confluence, ledit premier ensemble étant un ensemble de cellules stromales, plus particulièrement de fibroblastes, et dans laquelle la face interne (10) de ladite protubérance (8) supporte un deuxième ensemble de cellules adhérentes au stade de la confluence, ledit deuxième ensemble étant un ensemble de cellules épithéliales **(Figure 52).**

Selon un mode de réalisation particulier, l'invention concerne une puce microfluidique de culture cellulaire dans laquelle la face externe (9) de ladite protubérance (8) supporte un premier ensemble de cellules adhérentes, ledit premier ensemble étant un ensemble de cellules stromales, plus particulièrement de fibroblastes, et dans laquelle la face interne (10) de ladite protubérance (8) supporte un deuxième ensemble de cellules adhérentes au stade de la confluence, ledit deuxième ensemble étant un ensemble de cellules épithéliales.

Selon un mode de réalisation particulier, l'invention concerne une puce microfluidique de culture cellulaire dans laquelle la face externe et la face interne des protubérances, sont recouvertes d'une préparation de matrice extracellulaire (ECM) (Matrigel®, collagène, fibronectine, acide hyaluronique), avant l'introduction des cellules sur ces surfaces, soit avant la culture cellulaire. Plus particulièrement, ladite préparation ECM est composée de Matrigel® ou d'un mélange Matrigel® /collagène.

Selon un mode de réalisation particulier, l'invention concerne une puce microfluidique de culture cellulaire dans laquelle ladite membrane poreuse nanostructurée en 3D a une épaisseur de 2 à 300 nm, plus particulièrement de 30 nm.

Selon un mode de réalisation particulier, l'invention concerne une puce microfluidique de culture cellulaire dans laquelle ladite membrane poreuse nanostructurée en 3D a une épaisseur de 2 à 300 nm.

Selon un mode de réalisation particulier, l'invention concerne une puce microfluidique de culture cellulaire dans laquelle ladite membrane poreuse nanostructurée en 3D a une épaisseur de 30 nm.

La protubérance possédant une section à sa base de forme circulaire, la surface de cette section peut être calculée en fonction du rayon.

Selon un mode de réalisation particulier, l'invention concerne une puce microfluidique de culture cellulaire dans laquelle ladite protubérance en forme de dôme creux a une hauteur de 1 à 600 $\mu$m, plus particulièrement de 1 à 300 $\mu$m, plus particulièrement de 50 à 300 $\mu$m, plus particulièrement de 50 $\mu$m et une surface délimitée par la base du dôme de 78,5 $\mu$m$^2$ à 200 000 $\mu$m$^2$, plus particulièrement de 2 000 à 70 000 $\mu$m$^2$, encore plus particulièrement de 17 500 $\mu$m$^2$, et encore plus particulièrement de 7850 $\mu$m$^2$.

Le terme « **hauteur** » désigne la distance entre le centre du sommet de la protubérance et le centre de l'ouverture de diamètre d3.

Selon un mode de réalisation particulier, l'invention concerne une puce microfluidique de culture cellulaire dans laquelle ledit support comporte au moins deux perforations de forme et de dimensions identiques, et ladite membrane poreuse nanostructurée en 3D comporte au moins deux protubérances de forme et de dimensions identiques, le nombre de perforations étant égal au nombre de protubérances.

Lorsque que ladite membrane comporte au moins deux protubérances, il convient de définir la distance entre ces au moins deux protubérances.

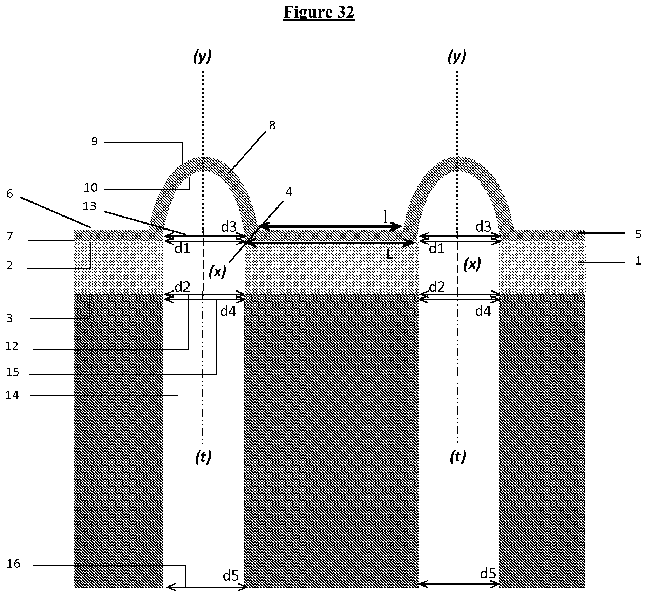

La définition de l'expression « **distance entre deux protubérances contiguës** » doit être comprise comme étant la distance entre deux points, situés respectivement sur la circonférence formée par l'intersection de la face externe de ladite protubérance et de la face supérieure de ladite membrane nanostructurée en 3D de deux protubérances contiguës, et formant la distance la plus courte parmi l'ensemble des distances possibles entre les couples de points situés respectivement sur lesdites circonférences de deux protubérances contiguës **(Figure 32)**.

Lorsque que ladite membrane comporte au moins deux protubérances, celles-ci doivent être séparées par une distance appropriée.

Selon un mode de réalisation particulier, l'invention concerne une puce microfluidique de culture cellulaire dans laquelle **la distance entre deux protubérances contiguës** est d'une valeur 1, ladite valeur 1 étant de 10 à 100 $\mu$m.

Selon un mode de réalisation particulier, l'invention concerne une puce microfluidique de culture cellulaire dans laquelle la **distance entre deux protubérances contiguës** est d'une valeur 1, ladite valeur 1 étant de 10 à 100 $\mu$m, plus particulièrement de 50 à 100 $\mu$m.

Selon un mode de réalisation particulier, l'invention concerne une puce microfluidique de culture cellulaire dans laquelle la **distance entre deux protubérances contiguës** est d'une valeur 1, ladite valeur 1 étant de 50 à 100 $\mu$m.

Dans chacun des paragraphes ci-dessous, l'expression « **distance entre deux protubérances contiguës** » peut être remplacée par la définition donnée ci-dessus.

Selon un mode de réalisation particulier, l'invention concerne une puce microfluidique de culture cellulaire dans laquelle ledit support comporte au moins deux perforations, et ladite membrane poreuse nanostructurée comporte au moins deux protubérances

tel que le ratio $\dfrac{d1}{d2}$ varie de 1 à $\dfrac{d1}{(d1+l)}$, 1 étant la valeur de la distance deux protubérances contiguës,

où le diamètre d1 est d'une valeur supérieure ou égale à 10 $\mu$m à une valeur inférieure ou égale à 500 $\mu$m, et le diamètre d2 est d'une valeur supérieure ou égale à 10 $\mu$m et inférieure ou égale à 500 $\mu$m.

Ainsi, le diamètre inférieur d2 de ladite perforation au niveau de la face inférieure du support, est toujours supérieur ou égal au diamètre supérieur d1 de ladite perforation au niveau de la face supérieure du support.

Selon un mode de réalisation particulier, l'invention concerne une puce microfluidique de culture cellulaire dans laquelle ladite membrane poreuse nanostructurée en 3D comporte 1 à 100 protubérances pour une surface de l'unité centrale de 1 cm$^2$.

Selon un mode de réalisation particulier, l'invention concerne une puce microfluidique de culture cellulaire dans laquelle ladite membrane poreuse nanostructurée en 3D comporte un nombre maximal de protubérances pour une surface de l'unité centrale de 1 cm$^2$, égal à la formule

$$\frac{\pi\,(d3/2)^2}{(l+d3)-\pi\,(d3/2)^2}$$

où 1 est la distance entre deux protubérances contiguës, ladite valeur 1 étant de 10 à 100 $\mu$m, plus particulièrement de 50 à 100 $\mu$m,
et où d3 est le diamètre de ladite protubérance.

La membrane poreuse nanostructurée en 3D est composée d'un nombre de couches successives de polyélectrolytes de 1 à 150, étant entendu que la successivité des couches s'applique à partir d'au moins deux couches.

Le nombre de couches permet de faire varier la tenue mécanique de la protubérance. En effet, plus le nombre de couches sera élevé et plus la résistance mécanique de la protubérance sera élevée afin qu'elle puisse conserver sa forme tout en soutenant la culture cellulaire sur ces faces externes et internes et en résistant au flux (par exemple de milieu de culture) de part et d'autre de celle-ci.

Selon un mode de réalisation particulier, l'invention concerne une puce microfluidique de culture cellulaire dans laquelle le nombre de couches successives de polyélectrolytes est de 1 à 150, en particulier de 15.

L'utilisation de polyélectrolyte pour constituer la membrane poreuse nanostructurée en 3D fournit un substitut biocompatible et fonctionnel pour mimer la lame basale sur laquelle viennent se fixer les cellules adhérentes dans les tissus.

La membrane poreuse nanostructurée en 3D peut être constituée d'une couche d'un polyélectolyte.

Selon un mode de réalisation particulier, l'invention concerne une puce microfluidique de culture cellulaire dans laquelle la membrane poreuse nanostructurée en 3D est constituée d'une couche d'un polyélectolyte choisi parmi le poly(sodium 4-styrènesulphonate) (PSS) , le poly(éthylèneimine), le poly(chlorure de diallyldimethylammonium), le poly(chlorure de acrylamide-co-diallyldimethylammonium), le chlorure de diallyldiméthyllammonium, le poly(hydrochlorure d'allylamine) (PAH), l'acide polyanetholesulfonique, l'acide polyacrylique, l'acide polystyrène-alt-maléique, le sulfate de polyvinyle, l'acide polyvinylsulfonique, l'acide poly(2-acrylamido-2-méthyl-1-propanesulfonique), le poly(acide 2-acrylamido-2-méthyl-1-propanesulfonique -co-acrylonitrile), l'acide poly 4-styrènesulfonique, le poly(acide 4-styrènesulfonique-co-acide maléique), le sel de sodium de 4-styrènesulfonique hydraté.

La membrane poreuse nanostructurée en 3D, peut être constituée d'au moins deux couches successives d'un polyélectolyte.

Lorsque ladite membrane comprend au moins deux couches, la construction de cette membrane couche par couche est indispensable.

La au moins une protubérance étant une partie intégrante de ladite la membrane poreuse nanostructurée en 3D, la composition de ladite la membrane poreuse nanostructurée en 3D sera identique à la composition de ladite au moins une protubérance.

Selon un mode de réalisation particulier, l'invention concerne une puce microfluidique de culture cellulaire dans laquelle la membrane poreuse nanostructurée en 3D est constituée de couches successives d'un polyélectrolyte choisi parmi le poly(sodium 4-styrènesulphonate) (PSS) , le poly(éthylèneimine), le poly(chlorure de diallyldimethylammonium), le poly(chlorure de acrylamide-co-diallyldimethylammonium), le chlorure de diallyldiméthyllammonium, le poly(hydrochlorure d'allylamine) (PAH), l'acide polyanetholesulfonique, l'acide polyacrylique, l'acide polystyrène-alt-maléique, le sulfate de polyvinyle, l'acide polyvinylsulfonique, l'acide poly(2-acrylamido-2-méthyl-1-propanesulfonique), le poly(acide 2-acrylamido-2-méthyl-1-propanesulfonique -co-acrylonitrile), l'acide poly 4-styrènesulfonique, le poly(acide 4-styrènesulfonique-co-acide maléique), le sel de sodium de 4-styrènesulfonique hydraté.

Lorsque ladite membrane comprend au moins deux couches successives, lesdites couches peuvent correspondre à au moins deux polyélectrolytes de nature différente : au moins un polyélectrolyte chargé négativement et au moins un polyélectrolyte chargé positivement.

Dans ce cas, la construction couche par couche de cette membrane doit être effectuée de telle manière qu'une couche de polyélectrolyte chargé positivement soit en alternance avec une couche de polyélectrolyte chargé négativement.

La membrane est ainsi fortement cohésive en raison des interactions électrostatiques entre les couches de polyélectrolytes chargées positivement et les couches de polyélectrolytes chargées négativement. Cela permet d'obtenir une membrane ayant un module de Young de l'ordre du kiloPascal.

Il est à noter que la couche inférieure et la couche supérieure de la membrane poreuse, peuvent être constituées, indépendamment l'une de l'autre, de l'un quelconque des polyélectrolytes.

Cependant, la charge du polyélectrolyte constituant la dernière couche, et constituant donc la face supérieure de ladite membrane, a un impact sur l'hydrophobicité de la membrane. Ainsi, lorsque la dernière couche est constituée par un polyélectrolyte chargée négativement, la face supérieure de la membrane est de nature relativement hydrophile en comparaison à une membrane dont la face supérieure est chargée positivement. A l'inverse, lorsque la dernière couche est constituée par un polyélectrolyte chargée positivement, la face supérieure de la membrane est de nature relativement hydrophobique en comparaison à une membrane dont la face supérieure est chargée négativement.

La rugosité de la face supérieure de la membrane est régie par les groupements portés par sur le polyélectrolyte constituant la dernière couche de la membrane, soit la couche constituant la face supérieure de ladite membrane. Par exemple, s'il s'agit d'une couche de PSS, ce polyélectrolyte possède un noyau benzène pendant attaché et un groupement sulfate lié, qui fournissent ainsi une surface plus rugueuse qu'une couche de PAH, électrolyte qui ne possède pas de noyau benzène attaché.

La rugosité de la membrane poreuse nanostructurée en 3 D est de l'ordre du nanomètre.

Selon un mode de réalisation particulier, l'invention concerne une puce microfluidique de culture cellulaire dans laquelle le nombre de couches successives est de 1 à 150, en particulier de 15, d'un polyélectrolyte chargé positivement et d'un polyélectrolyte chargé négativement, ladite couche de polyélectrolyte chargé négativement et ladite couche de polyélectrolyte chargé positivement étant en alternance l'une par rapport à l'autre.

Selon un mode de réalisation particulier, l'invention concerne une puce microfluidique de culture cellulaire dans laquelle la membrane poreuse nanostructurée en 3D est constituée de couches successives d'au moins deux polyélectrolytes, dont au moins un polyélectrolyte est chargé positivement et au moins un polyélectrolyte est chargé négativement,

     ledit polyélectrolyte chargé positivement étant choisi parmi : le poly(sodium 4-styrènesulphonate) , le poly(éthylèneimine), le poly(chlorure de diallyldimethylammonium), le poly(chlorure de acrylamide-co-diallyldimethylammonium), le chlorure de diallyldiméthyllammonium,

     et ledit polyélectrolyte chargé négativement étant choisi parmi le poly(hydrochlorure d'allylamine), l'acide polyanetholesulfonique, l'acide polyacrylique, l'acide polystyrène-alt-maléique, le sulfate de polyvinyle, l'acide polyvinylsulfonique, l'acide poly(2-acrylamido-2-méthyl-1-propanesulfonique), le poly(acide 2-acrylamido-2-méthyl-1-propanesulfonique -co-acrylonitrile), l'acide poly 4-styrènesulfonique, le poly(acide 4-styrènesulfonique-co-acide maléique), le sel de sodium de 4-styrènesulfonique hydraté.

ladite couche de polyélectrolyte chargé négativement et ladite couche de polyélectrolyte chargé positivement étant en alternance l'une par rapport à l'autre.

Selon un mode de réalisation particulier, l'invention concerne une puce microfluidique de culture cellulaire dans laquelle la membrane poreuse nanostructurée en 3D est constituée de couches successives d'un polyélectrolyte chargé positivement et d'un polyélectrolyte chargé négativement, ledit polyélectrolyte chargé positivement étant le poly(sodium 4-styrènesulphonate) (PSS) et ledit polyélectrolyte chargé négativement étant le poly(hydrochlorure d'allylamine) (PAH), ladite couche de polyélectrolyte chargé négativement et ladite couche de polyélectrolyte chargé positivement étant en alternance l'une par rapport à l'autre.

Selon un mode de réalisation particulier, l'invention concerne une puce microfluidique de culture cellulaire dans laquelle le nombre de couches successives de poly(sodium 4-styrenesulphonate) (PSS) et/ou de poly(allylamine hydrochloride) (PAH), est de 1 à 150 , en particulier de 15.

Selon un mode de réalisation particulier, l'invention concerne une puce microfluidique de culture cellulaire dans laquelle chaque couche de polyélectrolyte a une épaisseur de 2 à 300 nm, plus particulièrement d'environ 2 nm.

Selon un mode de réalisation particulier, l'invention concerne une puce microfluidique de culture cellulaire dans laquelle le nombre de couches successives est de 15, d'un polyélectrolyte chargé positivement et d'un polyélectrolyte chargé négativement, ladite couche de polyélectrolyte chargé négativement et ladite couche de polyélectrolyte chargé positivement étant en alternance l'une par rapport à l'autre, chaque couche ayant une épaisseur de 2 nm.

Selon un mode de réalisation particulier, l'invention concerne une puce microfluidique de culture cellulaire dans laquelle la valeur de l'épaisseur de la totalité des couches successives de polyélectrolytes est comprise de la valeur de l'épaisseur d'une couche à une valeur inférieure à la moitié du diamètre d3 de ladite protubérance.

Selon un mode de réalisation particulier, l'invention concerne une puce microfluidique de culture cellulaire selon RX dans laquelle la membrane poreuse nanostructurée en 3D (5) est constituée d'au moins une couche d'un polyélectolyte choisi parmi le poly(sodium 4-styrènesulphonate) (PSS) , le poly(éthylèneimine), le poly(chlorure de diallyldimethylammonium), le poly(chlorure de acrylamide-co-diallyldimethylammonium), le chlorure de diallyldiméthyllammonium, le poly(hydrochlorure d'allylamine) (PAH), l'acide polyanetholesulfonique, l'acide polyacrylique, l'acide polystyrène-alt-maléique, le sulfate de polyvinyle, l'acide polyvinylsulfonique, l'acide poly(2-acrylamido-2-méthyl-1-propanesulfonique), le poly(acide 2-acrylamido-2-méthyl-1-propanesulfonique -co-acrylonitrile), l'acide poly 4-styrènesulfonique, le poly(acide 4-styrènesulfonique-co-acide maléique), le sel de sodium de 4-styrènesulfonique hydraté,

     et dans laquelle lorsque ladite membrane poreuse nanostructurée en 3D (5) est constituée d'au moins deux couches successives, une couche constituée d'un un polyélectrolyte chargé positivement est en alternance d'une couche constituée d'un polyélectrolyte chargé négativement,

     ladite membrane poreuse nanostructurée en 3D (5) étant en particulier constituée 1 à 150 couches successives de polyélectrolyte,

     et plus particulièrement ladite membrane poreuse nanostructurée en 3D (5) étant constituée de 15 couches successives de poly(sodium 4-styrènesulphonate) (PSS) et/ou de poly(allylamine hydrochloride) (PAH), en alternance l'une de l'autre.

La membrane poreuse nanostructurée en 3D est donc composée de couches successives de polyélectrolytes et comporte ainsi comme paramètres variables :

-      le nombre de couches,

-      l'épaisseur de chacune des couches,

-      la charge du ou des polyélectrolytes utilisés.

En faisant varier le nombre de couches, la rugosité, l'épaisseur et la rigidité de ladite membrane, et donc de ladite protubérance peuvent être modifiées.

En faisant varier le nombre de couches ou le type de charge des polyélectrolytes utilisés, l'hydrophobicité de ladite membrane et de ladite protubérance peut également être modifiée.

Selon un mode de réalisation particulier, l'invention concerne une puce microfluidique de culture cellulaire dans laquelle ledit support a une épaisseur de 2 $\mu$m à 1000 $\mu$m plus particulièrement de 20 $\mu$m.

Selon un mode de réalisation particulier, l'invention concerne une puce microfluidique de culture cellulaire dans laquelle ladite perforation a une surface délimitée par ladite membrane non résorbable de 78,5 $\mu$m$^2$ à 200 000 $\mu$m$^2$, plus particulièrement de 2 000 à 70 000 $\mu$m$^2$, encore plus particulièrement de 17 500 $\mu$m$^2$, et encore plus particulièrement de 7850 $\mu$m$^2$.

Le support comporte au moins une perforation. Lorsque que ledit support comporte au moins deux perforations, il convient de définir la distance entre ces au moins deux perforations.

La « **distance entre deux perforations contigües** » doit être comprise comme étant la distance entre deux points situés respectivement sur la circonférence de chacune des sections circulaires supérieures desdites perforations contigües et formant la distance la plus courte, parmi l'ensemble des distances possibles entre les couples de points situés respectivement la circonférence de chacune des sections circulaires supérieures desdites perforations contigües.

Lorsque que ledit support comporte au moins deux perforations, celles-ci doivent être séparées par une distance appropriée.

Selon un mode de réalisation particulier, l'invention concerne une puce microfluidique de culture cellulaire dans laquelle ladite distance entre deux perforations contigües est d'une valeur L, ladite valeur L étant de 10 à 100 $\mu$m, plus particulièrement de 50 à 100 $\mu$m **(Figure 32).**

Selon un mode de réalisation particulier, l'invention concerne une puce microfluidique de culture cellulaire dans laquelle ledit support comporte 1 à 100 perforations pour une surface de l'unité centrale de 1 cm$^2$.

Selon un mode de réalisation particulier, l'invention concerne une puce microfluidique de culture cellulaire dans laquelle le support est composé de plastique bioimprimé, de polycarbonate, de plastique de culture tissulaire, de verre ou de résine SU-8.

La résine SU-8 est une résine polymérique photosensible négative à base d'époxyde novolac possédant une fonctionnalité moyenne en groupes époxyde d'environ 8. La résine SU-8 est bien connue de l'homme du métier et couramment utilisée dans la fabrication de micro-système. Le terme négatif signifie que les parties exposées aux UV deviennent réticulées, tandis que le reste du film reste soluble et peut être éliminé par lavage.

Les plastiques bioimprimés correspondent aux plastiques connus de l'homme de métier qui sont adaptés et compatibles aux équipements d'impression 3D (extrusion, contraintes de température).

Les plastiques de culture tissulaire correspondent aux plastiques, constituant les boites de Pétri, qui comportent un traitement en fond (traitement effectué par décharge électrique ou plasma pour rendre la surface hydrophile c'est-à-dire avec une charge nette négative) et permettent la fixation et la croissance de cellules adhérentes eucaryotes (à la différence des boites de culture plastiques non traitées pour la bactériologie).

Selon un mode de réalisation particulier, l'invention concerne une puce microfluidique de culture cellulaire dans laquelle le support est composé de plastique bioimprimé, de polycarbonate, de plastique de culture tissulaire, de verre ou de résine SU-8, et ledit support est transparent.

## MODULE INFERIEUR

Selon un mode de réalisation particulier, l'invention concerne une puce microfluidique de culture cellulaire qui contient un module inférieur (107) solide, comportant

- une unité inférieure (108)
  laquelle contient une face supérieure, une face inférieure et au moins une face latérale, et comprenant au moins un canal (14) de forme tubulaire comportant un orifice supérieur (15) et un orifice inférieur (16),

- un socle (109),

  ladite unité inférieure (108) étant intégrée dans ledit socle (109), et formant un tout avec ledit socle (109),

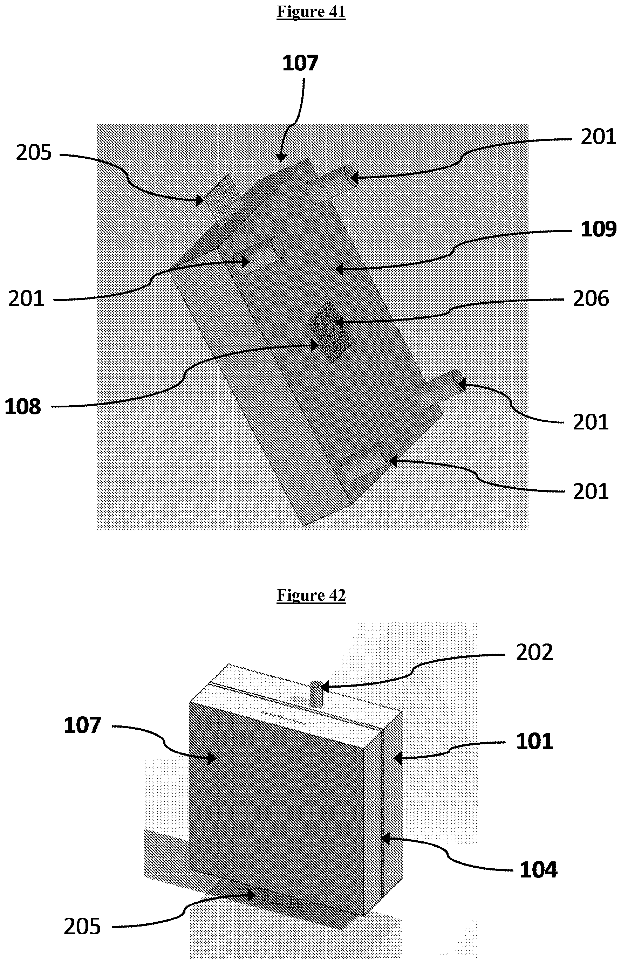

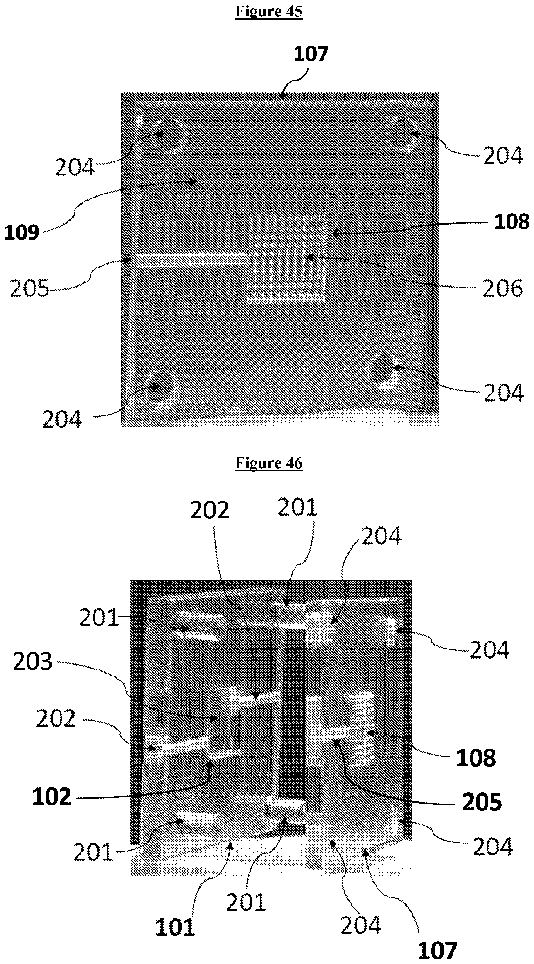

  et ladite face supérieure de ladite unité inférieure comportant ledit orifice supérieur (15) du au moins un canal, et ledit orifice inférieur (16) débouchant, lui-même ou par des moyens intermédiaires (17), à l'extérieur dudit socle (109) **(Figures 41 et 45).**

L'orifice supérieur du au moins un canal est situé au sein de l'unité inférieure, le au moins un canal s'étend à travers l'unité inférieure puis à travers le socle du module inférieur, ladite unité et ledit socle formant une pièce d'un seul tenant, de telle sorte que l'orifice inférieur du au moins un canal débouche à l'extérieur dudit socle.

L'orifice inférieur du au moins un canal débouche soit lui-même à l'extérieur dudit socle, soit débouche dans un moyen intermédiaire tel qu'un réservoir, qui lui débouche à l'extérieur dudit socle.

Selon un mode de réalisation particulier, l'invention concerne une puce microfluidique de culture cellulaire dans laquelle ledit module inférieur (107) et ledit module central (104) sont assemblés tel que l'orifice supérieur (15) du au moins un canal (14), de ladite face supérieure de ladite unité inférieure (108), s'ouvre sur au moins une desdites perforations (4) dudit support (1) de ladite unité centrale (105), et l'orifice inférieur (16) du au moins un canal (14), débouche sur l'extérieur de la puce, ladite face supérieure de ladite unité inférieure (108) et ladite face inférieure dudit support (3) de ladite unité centrale (105) ayant une forme et une surface identiques entre elles.

L'expression « **à l'extérieur de la puce »** doit s'interpréter comme « **à l'extérieur du socle »**, et vice-versa.

Lorsque le module inférieur est assemblé au module central, l'orifice supérieur (15) du au moins un canal (14), de ladite face supérieure de ladite unité inférieure (108), s'ouvre sur au moins une desdites perforations (4) dudit support (1). Ce au moins un canal permet de récupérer les sécrétions des cellules cultivées sur la face interne de la au moins une protubérance.

Ce au moins canal permet également d'introduire du milieu de culture et éventuellement les cellules qui sont cultivées sur la face interne de ladite protubérance.

Selon un mode de réalisation particulier, l'invention concerne une puce microfluidique de culture cellulaire dans laquelle ledit module inférieur (107) et ledit module central (104) sont assemblés tel que l'orifice supérieur (15) du au moins un canal (14), de ladite face supérieure de ladite unité inférieure (108), s'ouvre sur au moins une desdites perforations (4) dudit support (1) de ladite unité centrale (105), et l'orifice inférieur (16) du au moins un canal (14), débouche sur l'extérieur de la puce, via un moyen intermédiaire constitué par un réservoir (17) apte à récupérer des liquides et permettant un acheminement à l'extérieur de la puce via un canal de sortie (18) débouchant à l'extérieur du socle (109) dudit module inférieur (107),

ladite face supérieure de ladite unité inférieure (108) et ladite face inférieure dudit support de ladite unité centrale (105) ayant une forme et une surface identique entre elles.

Selon un mode de réalisation particulier, l'invention concerne une puce microfluidique de culture cellulaire, dans laquelle ledit orifice inférieur (16) du au moins un canal (14), débouche à l'extérieur de la puce sur un dispositif permettant l'analyse de composés en solution.

Ce dispositif d'analyse peut être constitué par tout dispositif analytique connu de l'Homme du métier tel qu'un spectromètre de masse, un dispositif RMN, un dispositif de chromatographie (en phase liquide ou gazeuse), un dispositif basé sur les interactions immunologiques (ELISA, immunoprécipitation), un dispositif PCR ou RT-PCR.

En effet, les sécrétions des cellules sont composées à la fois de protéines et peptides mais également d'ADN et d'ARN. Ainsi, le ou les dispositifs choisis d'analyse doivent permettre d'analyser l'ensemble de ces molécules.

Selon un mode de réalisation particulier, l'invention concerne une puce microfluidique de culture cellulaire, dans laquelle ledit orifice inférieur (16) du au moins un canal (14), débouche à l'extérieur de la puce sur un dispositif permettant l'analyse de composés en solution, ledit dispositif étant un spectromètre de masse.

La puce microfluidique selon l'invention peut être couplée directement à une puce intégrant un spectromètre de masse afin de réaliser une analyse en temps réel et en continu des sécrétions.

Selon un mode de réalisation particulier, l'invention concerne une puce microfluidique de culture cellulaire dans laquelle ladite unité inférieure (108) et ladite unité centrale (105) sont assemblées par des éléments de fixation (204) situés respectivement sur chacun des socles du module inférieur (109) et du module central (106), de façon à assembler de manière étanche ladite face supérieure de ladite unité inférieure (108) et ladite face inférieure dudit support (3) de ladite unité centrale (105).

Selon un mode de réalisation particulier, l'invention concerne une puce microfluidique de culture cellulaire, dans laquelle la valeur du diamètre d4 dudit orifice supérieur (15) dudit canal (14) est supérieure à la valeur du diamètre d5 dudit orifice inférieur (16) dudit canal (14).

Selon un mode de réalisation particulier, l'invention concerne une puce microfluidique de culture cellulaire, dans laquelle la valeur du diamètre d4 dudit orifice supérieur (15) dudit canal (14) est égale à la valeur du diamètre d5 dudit orifice inférieur (16) dudit canal (14), ledit au moins un canal étant de forme cylindrique.

Selon un mode de réalisation particulier, l'invention concerne une puce microfluidique de culture cellulaire, dans laquelle ledit orifice supérieur (15) dudit au moins un canal (14) présente un diamètre d4 et un axe (t) passant par le centre dudit orifice supérieur (15) et perpendiculaire audit support (1),

tel que la valeur du diamètre d4 est **supérieure ou égale** à la valeur du diamètre inférieur d2 de ladite au moins une perforation (4).

Selon un mode de réalisation particulier, l'invention concerne une puce microfluidique de culture cellulaire, dans laquelle ledit orifice supérieur (15) dudit au moins un **canal (14)** débouche sur **une seule perforation** (4) dudit support (1).

Selon un mode de réalisation particulier, l'invention concerne une puce microfluidique de culture cellulaire, dans laquelle ladite au moins une perforation (4) dudit support (1) est en **totalité en regard** dudit orifice supérieur (15) dudit au moins un canal (14) :

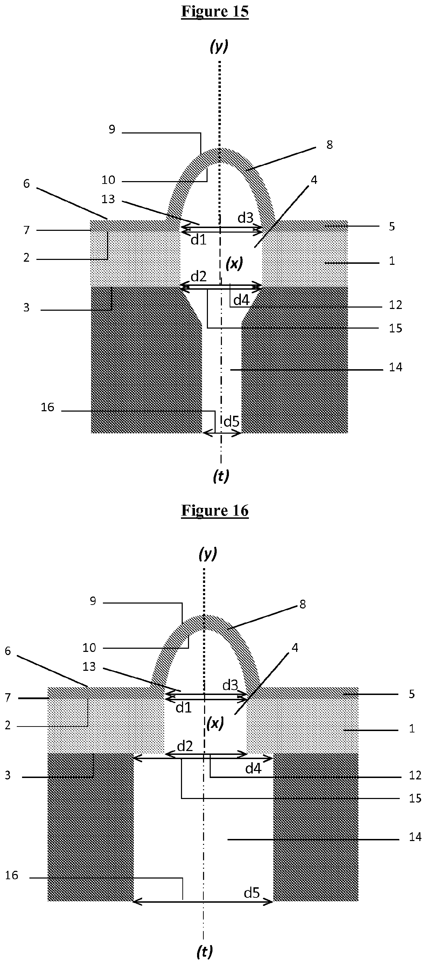

- lorsque la valeur du diamètre inférieure d2 de ladite perforation (4) est **égale** à la valeur du diamètre d4 dudit orifice supérieur (15) dudit canal (14), et que l'axe (x), passant par le centre de ladite section supérieure (11) de la perforation (4) et perpendiculaire audit support (1), et l'axe (t), passant par le centre dudit orifice supérieur (15) dudit canal (14) et perpendiculaire audit support (1), sont **confondus (Figures 14 et 15),** ou

- lorsque la valeur du diamètre inférieure d2 de ladite perforation (4) est **inférieure** à la valeur du diamètre d4 dudit orifice supérieur (15) dudit canal (14), et que l'axe (x), passant par le centre de ladite section supérieure (11) de la perforation (4) et perpendiculaire audit support (1), et l'axe (t), passant par le centre dudit orifice supérieur (15) dudit canal (14) et perpendiculaire audit support (1), sont **confondus, ou distincts l'un par rapport à l'autre** d'une distance d'une valeur inférieure ou égale à [ (valeur de d4 - valeur de d2) /2] (Figures 16, 17 et 18).

Selon un mode de réalisation particulier, l'invention concerne une puce microfluidique de culture cellulaire, dans laquelle ladite au moins une perforation (4) dudit support (1) est **en partie en regard** dudit orifice supérieur (15) dudit au moins un canal (14) :

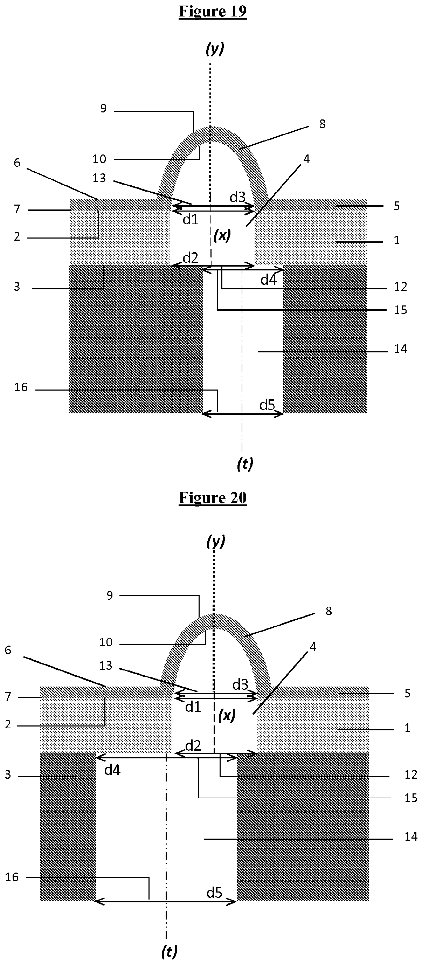

- lorsque la valeur du diamètre inférieure d2 de ladite perforation (4) est **égale** à la valeur du diamètre d4 dudit orifice supérieur (15) dudit canal (14), et que l'axe (x) passant par le centre de ladite section supérieure (11) de la perforation (4) et perpendiculaire audit support (1), et l'axe (t), passant par le centre dudit orifice supérieur (15) dudit canal (14) et perpendiculaire audit support (1), sont **distincts** l'un par rapport à l'autre d'une distance d'une valeur inférieure ou égale à [valeur de d4 / 2] **(Figures 19, 21, 22, 24, 29 et 30),** ou
- lorsque la valeur du diamètre inférieure d2 de ladite perforation (4) est **inférieure** à la valeur du diamètre d4 dudit orifice supérieur (15) dudit canal (14), et que l'axe (x) passant par le centre de ladite section supérieure (11) de la perforation (4) et perpendiculaire audit support (1), et l'axe (t), passant par le centre dudit orifice supérieur (15) dudit canal (14) et perpendiculaire audit support (1), sont **distincts** l'un par rapport à l'autre d'une distance d'une valeur inférieure ou égale à [valeur de d4 / 2] **(Figures 20, 23, 25, 26, 27 et 28)**.

Selon un mode de réalisation particulier, l'invention concerne une puce microfluidique de culture cellulaire, dans laquelle ledit module inférieur (107) comporte **au moins deux canaux (14).**

Selon un mode de réalisation particulier, l'invention concerne une puce microfluidique de culture cellulaire, dans laquelle chacun des orifices supérieurs (15) des au moins deux canaux (14) débouche sur une perforation (4) et les au moins deux orifices inférieurs (16) des au moins deux canaux (14) débouchent à l'extérieur de la puce respectivement à au moins deux emplacements distincts **(Figure 32)**.

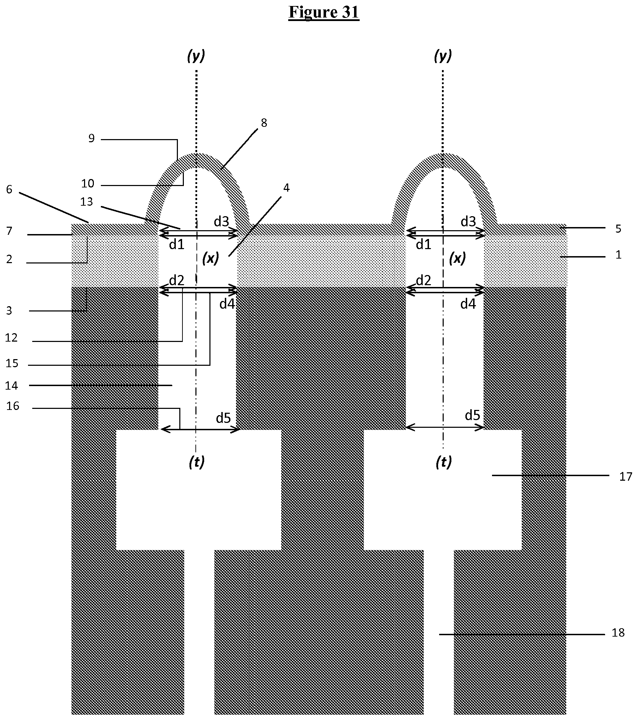

Selon un mode de réalisation particulier, l'invention concerne une puce microfluidique de culture cellulaire, dans laquelle chacun des orifices supérieurs (15) des au moins deux canaux (14) débouche sur une perforation (4) et les au moins deux orifices inférieurs (16) des au moins deux canaux (14) débouchent respectivement sur un moyen intermédiaire constitué par un réservoir (17) apte à récupérer des liquides et permettant un acheminement à l'extérieur de la puce via un canal de sortie (18) débouchant à l'extérieur du socle (109) dudit module inférieur (107) **(Figure 31)**.

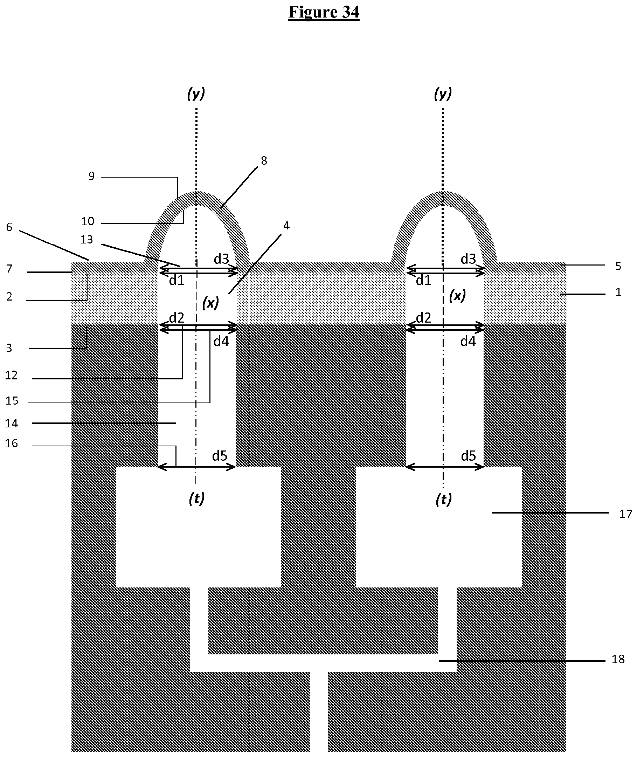

Selon un mode de réalisation particulier, l'invention concerne une puce microfluidique de culture cellulaire, dans laquelle chacun des orifices supérieurs (15) des au moins deux canaux (14) débouche sur une perforation (4) et les au moins deux orifices inférieurs (16) des au moins deux canaux (14) débouchent respectivement sur un moyen intermédiaire constitué par un réservoir (17) apte à récupérer des liquides et permettant un acheminement à l'extérieur de la puce via un canal de sortie (18) débouchant à l'extérieur du socle (109) dudit module inférieur (107), lesdits canaux de sortie de chacun desdits réservoirs étant connectés entre eux pour déboucher à l'extérieur de la puce au même emplacement **(Figure 34)**.

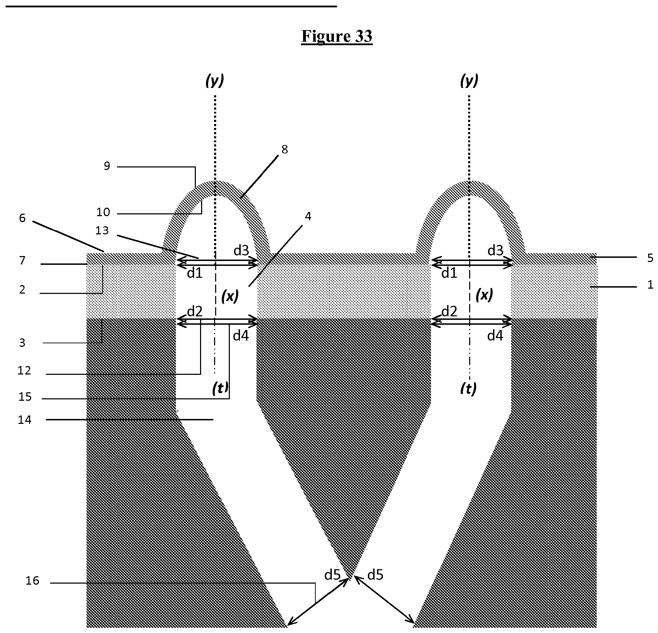

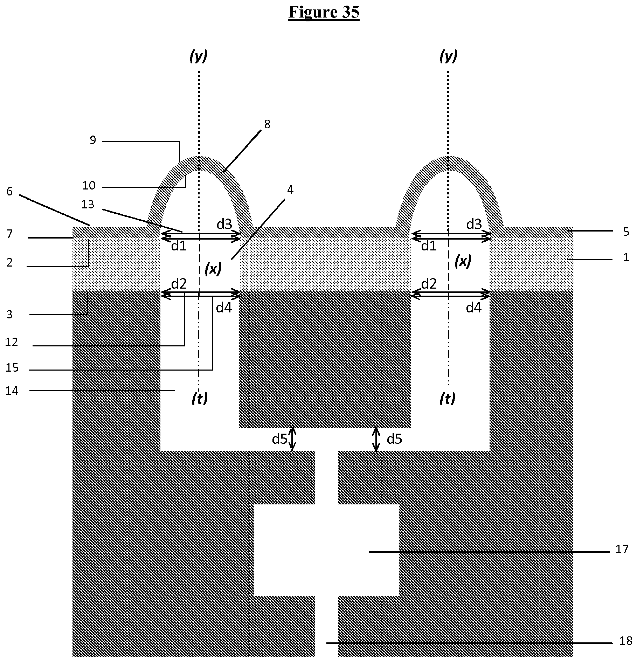

Selon un mode de réalisation particulier, l'invention concerne une puce microfluidique de culture cellulaire, dans laquelle les au moins deux canaux (14) sont connectés entre eux pour former un réseau, tel que chacun des orifices supérieurs (15) des au moins deux canaux (14) débouche sur une perforation (4) et l'orifice inférieur (16) de chacun des au moins deux canaux (14) débouchent eux-même à l'extérieur de la puce au même emplacement ou via un moyen intermédiaire constitué d'un réservoir (17) apte à récupérer des liquides et permettant un acheminement à l'extérieur de la puce via un canal de sortie (18) débouchant à l'extérieur du socle (109) dudit module inférieur (107) **(Figures 33 et 35)**.

Selon un mode de réalisation particulier, l'invention concerne une puce microfluidique de culture cellulaire, dans laquelle chacun des orifices supérieurs (15) des au moins deux canaux (14) débouche sur une perforation (4)

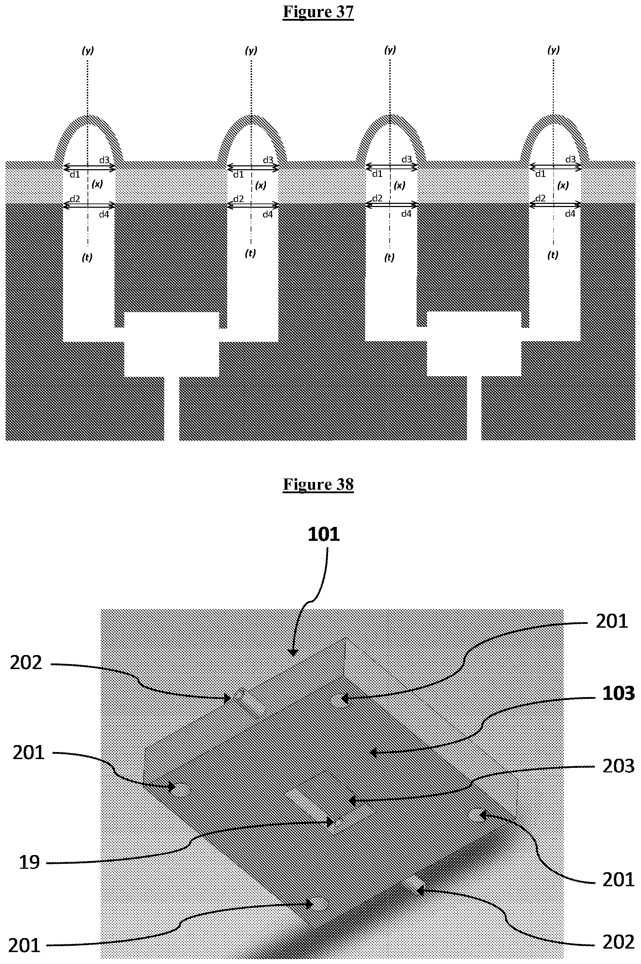

et un premier ensemble d'au moins deux orifices inférieurs (16) des au moins deux canaux (14) débouchent sur un premier moyen intermédiaire constitué par un réservoir (17),

et un au moins deuxième ensemble d'au moins deux orifices inférieurs (16) des au moins deux canaux (14) débouchent sur au moins un deuxième moyen intermédiaire constitué par un réservoir (17),

lesdits premier et au moins deuxième réservoirs (17) étant aptes à récupérer des liquides et permettant chacun un acheminement à l'extérieur de la puce via des canaux de sortie (18) respectifs débouchant à l'extérieur du socle (109) dudit module inférieur (107), à des emplacements différents **(Figure 37)**.

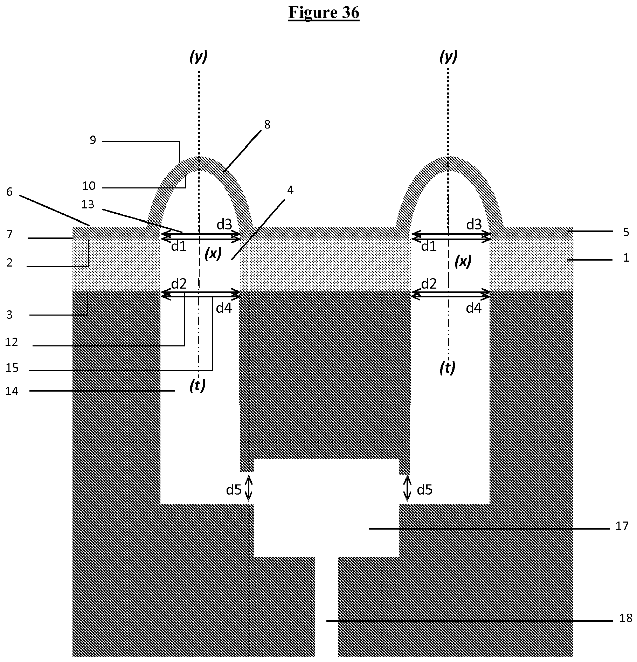

Selon un mode de réalisation particulier, l'invention concerne une puce microfluidique de culture cellulaire, dans laquelle chacun des orifices supérieurs (15) des au moins deux canaux (14) débouche sur une perforation (4) et les au moins deux orifices inférieurs (16) de l'ensemble des au moins deux canaux (14) débouchent sur un même moyen intermédiaire constitué par un réservoir (17) apte à récupérer des liquides et permettant un acheminement à l'extérieur de la puce via un canal de sortie (18) débouchant à l'extérieur du socle (109) dudit module inférieur (107) **(Figure 36)**.

Selon un mode de réalisation particulier, l'invention concerne une puce microfluidique de culture cellulaire dans laquelle ledit module inférieur (107) a une hauteur de 100 μm à 2 cm.

Selon un mode de réalisation particulier, l'invention concerne une puce microfluidique de culture cellulaire dans laquelle ledit socle (109) du module inférieur (107) a une hauteur de 100 μm à 2 cm.

Selon un mode de réalisation particulier, l'invention concerne une puce microfluidique de culture cellulaire dans laquelle ladite unité inférieure (108) a une hauteur de 100 μm à 2 cm.

Selon un mode de réalisation particulier, l'invention concerne une puce microfluidique de culture cellulaire dans laquelle le module inférieur (107) est composé de plastique bioimprimé, de polycarbonate, de plastique de culture tissulaire ou de SU8.

Selon un mode de réalisation particulier, l'invention concerne une puce microfluidique de culture cellulaire dans laquelle le module inférieur (107) est composé de plastique bioimprimé, de polycarbonate, de plastique de culture tissulaire, de verre ou de résine SU-8, et ledit le module inférieur (107) est transparent.

Selon un mode de réalisation particulier, l'invention concerne une puce microfluidique de culture cellulaire dans laquelle le socle du module inférieur (109) est composé de plastique bioimprimé, de polycarbonate, de plastique de culture tissulaire ou de SU8.

Selon un mode de réalisation particulier, l'invention concerne une puce microfluidique de culture cellulaire dans laquelle le socle du module inférieur (109) est composé de plastique bioimprimé, de polycarbonate, de plastique de culture tissulaire, de verre ou de résine SU-8, et ledit socle du module inférieur (109) est transparent.

Selon un mode de réalisation particulier, l'invention concerne une puce microfluidique de culture cellulaire dans laquelle l'unité inférieure (108) est composée de plastique bioimprimé, de polycarbonate, de plastique de culture tissulaire ou de SU8.

Selon un mode de réalisation particulier, l'invention concerne une puce microfluidique de culture cellulaire dans laquelle l'unité inférieure (108) est composée de plastique bioimprimé, de polycarbonate, de plastique de culture tissulaire, de verre ou de résine SU-8, et ladite unité inférieure (108) est transparente.

## MODULE SUPERIEUR

Selon un mode de réalisation particulier, l'invention concerne une puce microfluidique de culture cellulaire qui contient un module supérieur (101) comprenant :

- une unité supérieure (102) solide et creuse, laquelle est constituée d'une surface pleine définissant un volume ouvert (chambre) (203), et les bords de la surface pleine étant aptes à être en contact avec ladite unité centrale (105) dudit module central (104) et délimitant la surface de l'ouverture dudit volume ouvert ;
- et un socle (103),

ladite unité supérieure (102) étant intégrée dans ledit socle (103), et formant un tout avec ledit socle (103).

La chambre (203) est intégrée dans le socle (103) du module supérieur (101), et se trouve ainsi entourée sur toutes ses surfaces par ledit socle (103), à l'exception de la surface du volume ouvert de ladite chambre (203).

Selon un mode de réalisation particulier, l'invention concerne une puce microfluidique de culture cellulaire dans laquelle ladite unité supérieure (102) dudit module supérieur (101) et ladite unité centrale (105) dudit module central (104) sont assemblées de sorte que ladite surface de l'ouverture de ladite unité supérieure (102) est positionnée au dessus de ladite face supérieure (6) de la membrane poreuse nanostructurée en 3D (5) de ladite unité centrale (105), ladite surface de l'ouverture et ladite face supérieure (6) ayant une forme et une surface identiques entre elles.

Dans un mode de réalisation particulier, l'unité supérieure comporte une ouverture sur l'extérieur (fenêtre), au niveau de la surface pleine constituant ladite chambre permettant d'insérer une pièce en verre transparente compatible avec l'observation au microscope, de préférence une lame de microscope, afin d'observer la culture de cellules sur la face externe de la membrane poreuse nanostructurée en 3D et la face externe de la au moins une protubérance.

L'ouverture est située de préférence à un endroit de la surface pleine de ladite chambre, telle qu'elle permet l'observation à la verticale de la culture de cellules sur la face externe de la protubérance.

Ladite pièce en verre transparente est positionnée sur ladite ouverture de la surface pleine constituant ladite chambre, et maintenue de façon étanche à ladite unité supérieure pour maintenir un espace clos entre l'unité supérieure et l'unité centrale.

De préférence, ladite pièce en verre transparente est maintenue de façon étanche à ladite unité supérieure à l'aide d'une colle étanche.

Selon un mode de réalisation particulier, l'invention concerne une puce microfluidique de culture cellulaire dans laquelle ladite unité supérieure (102) dudit module supérieur (101) et ladite unité centrale (105) dudit module central (104) sont assemblées par des éléments de fixation (201,204) situés sur chacun des socles (103,106) du module supérieur (101) et du module central (104), de façon à assembler de manière étanche ladite surface de l'ouverture de ladite unité supérieure (102) et ladite face supérieure (6) de la membrane poreuse nanostructurée en 3D (5) de ladite unité centrale (105).

Selon un mode de réalisation particulier, l'invention concerne une puce microfluidique de culture cellulaire dans laquelle ladite unité supérieure (102) solide et creuse dans laquelle ladite surface pleine comporte une face rectangulaire pleine et quatre faces pleines adjacentes à ladite face rectangulaire pleine et adjacentes entre elles, formant ladite chambre (203), les bords libres desdites quatre faces pleines délimitant ladite surface de l'ouverture de ladite chambre (206).

Selon un mode de réalisation particulier, l'invention concerne une puce microfluidique de culture cellulaire dans laquelle le module supérieur (101) est composé de plastique bioimprimé, de polycarbonate, de plastique de culture tissulaire ou de SU8.

Selon un mode de réalisation particulier, l'invention concerne une puce microfluidique de culture cellulaire dans laquelle le module supérieur (101) est composé de plastique bioimprimé, de polycarbonate, de plastique de culture tissulaire, de verre ou de résine SU-8, et ledit module supérieur (101) est transparent.

Selon un mode de réalisation particulier, l'invention concerne une puce microfluidique de culture cellulaire dans laquelle le socle du module supérieur (103) est composé de plastique bioimprimé, de polycarbonate, de plastique de culture tissulaire ou de SU8.

Selon un mode de réalisation particulier, l'invention concerne une puce microfluidique de culture cellulaire dans laquelle le socle du module supérieur (103) est composé de plastique bioimprimé, de polycarbonate, de plastique de culture tissulaire, de verre ou de résine SU-8, et ledit socle du module supérieur (103) est transparent.

Selon un mode de réalisation particulier, l'invention concerne une puce microfluidique de culture cellulaire dans laquelle l'unité supérieure (102) est composée de plastique bioimprimé, de polycarbonate, de plastique de culture tissulaire ou de SU8.

Selon un mode de réalisation particulier, l'invention concerne une puce microfluidique de culture cellulaire dans laquelle l'unité supérieure (102) est composée de plastique bioimprimé, de polycarbonate, de plastique de culture tissulaire, de verre ou de résine SU-8, et ladite unité supérieure (102) est transparente.

Selon un mode de réalisation particulier, l'invention concerne une microfluidique de culture cellulaire dans laquelle ladite surface de l'ouverture de ladite chambre (203) de ladite unité supérieure (102) est positionnée au dessus de ladite face supérieure (6) de la membrane poreuse nanostructurée en 3D (5) de ladite unité centrale (105) pour former un espace clos délimité par ladite surface pleine de ladite unité supérieure (102) et ladite face supérieure (6) de la membrane poreuse nanostructurée en 3D (5).

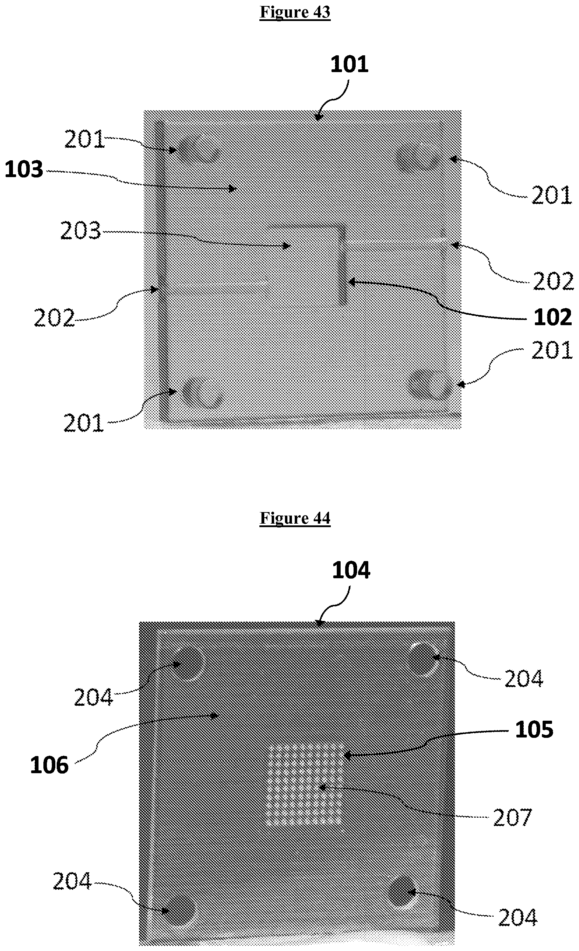

Selon un mode de réalisation particulier, l'invention concerne une puce microfluidique de culture cellulaire dans laquelle ladite surface de l'ouverture de ladite chambre (203) de ladite unité supérieure (102) est positionnée au-dessus de ladite face supérieure (6) de la membrane poreuse nanostructurée en 3D (5) de ladite unité centrale (105), et dans laquelle ladite au moins une face pleine de ladite unité supérieure (102) présente deux orifices débouchant respectivement sur un canal entrée/sortie (202),

pour former un espace clos délimité par ladite surface pleine de ladite unité supérieure (102), et ladite la face supérieure (6) de la membrane poreuse nanostructurée en 3D (5) de ladite unité centrale (105) pouvant communiquer avec l'extérieur de ladite puce microfluidique uniquement par lesdits deux orifices via lesdits canaux entrée/sortie (202) qui débouchent à l'extérieur du socle (109) **(Figures 38 et 43)**.

L'unité supérieure comporte deux orifices débouchant respectivement sur un canal entrée/sortie. Les deux canaux entrée/sortie s'étendent à travers le socle du module supérieur, ladite unité et ledit socle formant une pièce d'un seul tenant, de telle sorte que lesdits deux canaux entrée/sortie débouchent à l'extérieur dudit socle supérieur.

Lorsque le module supérieur est assemblé au module central, l'assemblage de la chambre sur la membrane poreuse nanostructurée en 3D permet de définir un espace clos pouvant communiquer avec l'extérieur de ladite puce microfluidique uniquement par lesdits deux orifices via lesdits canaux entrée/sortie. Ces canaux permettent également l'introduction du milieu de culture dans la chambre, et éventuellement l'introduction des cellules qui sont cultivées sur la face supérieure de ladite membrane ou la face externe de la au moins une protubérance.

Ces canaux entrée/sortie peuvent être couplés à des capteurs permettant un suivi en continu des conditions et paramètres de culture (capteurs de CO2, O2, pression, température, glucose, pH). Dans un mode de réalisation alternatif, ces capteurs peuvent être directement intégrés dans la chambre (203).

Selon un mode de réalisation particulier, l'invention concerne une puce microfluidique de culture cellulaire dans laquelle ledit module supérieur (101) a une hauteur de 50 μm à 2 cm, en particulier de 1 cm.

Selon un mode de réalisation particulier, l'invention concerne une puce microfluidique de culture cellulaire dans laquelle ledit socle (103) dudit module supérieur (101) a une hauteur de 50 μm à 2 cm, en particulier de 1 cm.

Selon un mode de réalisation particulier, l'invention concerne une puce microfluidique de culture cellulaire dans laquelle ladite unité supérieure (102) dudit module supérieur (101) a une hauteur de 50 μm à 2 cm, en particulier de 1 cm.

## ASSEMBLAGE DES TROIS MODULES

Selon un mode de réalisation particulier, l'invention concerne une puce microfluidique de culture cellulaire, comportant un module supérieur (101), un module central (104) et un module inférieur (107), et dans laquelle les susdits modules sont assemblés tel que

ladite surface de l'ouverture de ladite unité supérieure (102) dudit module supérieur (101) est positionnée au dessus de ladite face supérieure (2) de la membrane poreuse nanostructurée en 3D (5) de ladite unité centrale (105) dudit module central (104), et l'orifice supérieur (15) du au moins un canal (14), de ladite face supérieure de ladite unité inférieure (108) dudit module inférieur (107), s'ouvre sur au moins une desdites perforations (4) dudit support (1) de ladite unité centrale (105),

ladite surface de l'ouverture de ladite unité supérieure (102) et ladite face supérieure (2) de la membrane poreuse nanostructurée en 3D (5) de ladite unité centrale (105) ayant une forme et une surface identiques entre elles,

ladite face supérieure de ladite unité inférieure (108) et ladite face inférieure dudit support (3) de ladite unité centrale (105) ayant une forme et une surface identiques entre elles,

et ledit module supérieur (101), ledit module central (104) et ledit module inférieur (107) étant assemblés par des éléments de fixation (201, 204) situés sur chacun des socles respectifs (103, 106, 109) **(Figures 42, 46 et 47).**

Les éléments de fixation situés sur les socles de chacun des modules permettent soit de verrouiller les modules deux à deux, pour assembler le module supérieur avec le module central et assembler le module central avec le module inférieur, soit de verrouiller deux modules sur le troisième module, pour assembler le module central et le module inférieur sur le module supérieur.

Ces éléments de fixation peuvent être des ergots ou éléments mâle-femelle, de préférence des éléments mâle-femelle.

Selon un mode de réalisation particulier, le socle du module supérieur comporte des plots (201) comme éléments de fixation mâle, et les socles des modules central et inférieur comportent des perforations (204) comme éléments de fixation femelle, aptes à se verrouiller sur lesdits plots du module supérieur.

Selon un mode de réalisation particulier, l'invention concerne une puce microfluidique de culture cellulaire, dans laquelle ladite face interne (10) de ladite protubérance (8) est en **totalité en regard** dudit orifice supérieur (15) dudit canal (14) :

lorsque ladite face interne (10) de ladite au moins une protubérance (8) est **en totalité en regard de ladite au moins une perforation (4)** dudit support (1) :

- tel que lorsque la valeur du diamètre supérieur d1 de ladite au moins une perforation (4) est **supérieure ou égale** à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus;** ou

- tel que la valeur du diamètre supérieur d1 de ladite au moins une perforation (4) est **supérieure à** la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure ou égale à [(valeur de d1 - valeur de d3)/2].

et lorsque ladite perforation (4) dudit support (1) est **en totalité en regard** dudit orifice supérieur (15) dudit canal (14) :

tel que l'axe (y), passant par le centre de ladite base circulaire (13) de ladite protubérance (8) et perpendiculaire audit support (1), et l'axe (t), passant par le centre dudit orifice supérieur (15) dudit canal (14) et perpendiculaire audit support (1), sont **confondus, ou distincts** l'un par rapport à l'autre d'une distance inférieure ou égale à [(valeur de d4 - valeur de d3)/2] lorsque la valeur de d4 est supérieure à la valeur de d2.

Selon un mode de réalisation particulier, l'invention concerne une puce microfluidique de culture cellulaire, dans laquelle ladite face interne (10) de ladite protubérance (8) est en partie en regard dudit orifice supérieur (15) dudit canal (14) :

lorsque ladite face interne (10) de ladite au moins une protubérance (8) est en totalité en regard de ladite au moins une perforation (4) dudit support (1) :

- tel que lorsque la valeur du diamètre supérieur d1 de ladite au moins une perforation (4) est supérieure ou égale à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus; ou

- tel que la valeur du diamètre supérieur d1 de ladite au moins une perforation (4) est supérieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à [(valeur de d1 - valeur de d3)/2].

et lorsque

- l'axe (y), passant par le centre de ladite base circulaire (13) de ladite protubérance (8) et perpendiculaire audit support (1), et l'axe (t), passant par le centre dudit orifice supérieur (15) dudit canal (14) et perpendiculaire audit support (1), sont confondus ou distincts l'un de l'autre d'une distance inférieure ou égale à [ (valeur de d4 /2) + (4/10 de valeur de d3) ].

Selon un mode de réalisation particulier, l'invention concerne une puce microfluidique de culture cellulaire, dans laquelle ladite face interne (10) de ladite protubérance (8) est en partie en regard dudit orifice supérieur (15) dudit canal (14) :

lorsque ladite face interne (10) de ladite au moins une protubérance (8) est en totalité en regard de ladite au moins une perforation (4) dudit support (1) :

- tel que lorsque la valeur du diamètre supérieur d1 de ladite au moins une perforation (4) est supérieure ou égale à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus; ou

- tel que la valeur du diamètre supérieur d1 de ladite au moins une perforation (4) est supérieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à [(valeur de d1 - valeur de d3)/2].

et lorsque

- l'axe (y), passant par le centre de ladite base circulaire (13) de ladite protubérance (8) et perpendiculaire audit support (1), et l'axe (t), passant par le centre dudit orifice supérieur (15) dudit canal (14) et perpendiculaire audit support (1), sont confondus ou distincts l'un de l'autre d'une distance inférieure ou égale à [ [ (valeur de d4 + valeur de d4) /2] - 10 $\mu$m].

Selon un mode de réalisation particulier, l'invention concerne une puce microfluidique de culture cellulaire, dans laquelle ladite face interne (10) de ladite protubérance (8) est en **partie en regard** dudit orifice supérieur (15) dudit canal (14) :

lorsque ladite face interne (10) de ladite au moins une protubérance (8) est **en totalité en regard de ladite au moins une perforation** (4) dudit support (1) :

- tel que lorsque la valeur du diamètre supérieur d1 de ladite au moins une perforation (4) est **supérieure ou égale** à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus;** ou
- tel que la valeur du diamètre supérieur d1 de ladite au moins une perforation (4) est **supérieure à** la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure ou égale à [(valeur de d1 - valeur de d3)/2].

et lorsque

- l'axe (y), passant par le centre de ladite base circulaire (13) de ladite protubérance (8) et perpendiculaire audit support (1), et l'axe (t), passant par le centre dudit orifice supérieur (15) dudit canal (14) et perpendiculaire audit support (1), sont **confondus ou distincts** l'un de l'autre d'une distance inférieure ou égale à [valeur de d4 /2].

Selon un mode de réalisation particulier, l'invention concerne une puce microfluidique de culture cellulaire, dans laquelle ladite face interne (10) de ladite protubérance (8) est en partie en regard dudit orifice supérieur (15) dudit canal (14) :

lorsque ladite face interne (10) de ladite au moins une protubérance (8) est en partie en regard de ladite au moins une perforation (4) dudit support (1) :

- tel que la valeur du diamètre supérieur d1 de ladite au moins une perforation (4) est inférieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus, ou distincts l'un de l'autre d'une valeur inférieure ou égale à [ (valeur de d1 /2) + (4/10 de valeur de d3) ].

et ledit axe (x) et l'axe (t), passant par le centre dudit orifice supérieur (15) dudit canal (14) et perpendiculaire audit support (1), sont confondus lorsque la valeur du diamètre d2 est égal ou supérieur à la valeur du diamètre d4,

ou sont distincts l'un par rapport à l'autre d'une distance d'une valeur inférieure ou égale à [(valeur de d4 - valeur de d1)/2], lorsque la valeur du diamètre d4 est supérieur à la valeur du diamètre d2.

Selon un mode de réalisation particulier, l'invention concerne une puce microfluidique de culture cellulaire, dans laquelle ladite face interne (10) de ladite protubérance (8) est en partie en regard dudit orifice supérieur (15) dudit canal (14) :

lorsque ladite face interne (10) de ladite au moins une protubérance (8) est en partie en regard de ladite au moins une perforation (4) dudit support (1) :

- tel que la valeur du diamètre supérieur d1 de ladite au moins une perforation (4) est inférieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus, ou distincts l'un de l'autre d'une valeur inférieure ou égale à [ [ (valeur de d3 + valeur de d1) /2] - 10 μm].

et ledit axe (x) et l'axe (t), passant par le centre dudit orifice supérieur (15) dudit canal (14) et perpendiculaire audit support (1), sont confondus lorsque la valeur du diamètre d2 est égal ou supérieur à la valeur du diamètre d4,

ou sont distincts l'un par rapport à l'autre d'une distance d'une valeur inférieure ou égale à [(valeur de d4 - valeur de d1)/2], lorsque la valeur du diamètre d4 est supérieur à la valeur du diamètre d2.

Selon un mode de réalisation particulier, l'invention concerne une puce microfluidique de culture cellulaire, dans laquelle ladite face interne (10) de ladite protubérance (8) est en **partie en regard** dudit orifice supérieur (15) dudit canal (14) :

lorsque ladite face interne (10) de ladite au moins une protubérance (8) est **en partie en regard de ladite au moins une perforation (4)** dudit support (1) :

- tel que la valeur du diamètre supérieur d1 de ladite au moins une perforation (4) est **inférieure** à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus, ou distincts l'un de l'autre d'une valeur inférieure ou égale à [(valeur de d3 - valeur de d1)12],**

**et** ledit axe (x) et l'axe (t), passant par le centre dudit orifice supérieur (15) dudit canal (14) et perpendiculaire audit support (1), sont **confondus lorsque** la valeur du diamètre d2 est égal ou supérieur à la valeur du diamètre d4,

**ou** sont distincts l'un par rapport à l'autre d'une distance d'une valeur **inférieure ou égale à [(valeur de d4 - valeur de d1)12], lorsque** la valeur du diamètre d4 est supérieur à la valeur du diamètre d2.

Selon un mode de réalisation particulier, l'invention concerne une puce microfluidique de culture cellulaire, dans laquelle ladite face interne (10) de ladite protubérance (8) est en **partie en regard** dudit orifice supérieur (15) dudit canal (14) :

lorsque ladite face interne (10) de ladite au moins une protubérance (8) est **en partie en regard de ladite au moins une perforation (4)** dudit support (1) :

- tel que la valeur du diamètre supérieur d1 de ladite perforation (4) est **égale à** la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure [valeur de d1/2],

**et** ledit axe (x) et l'axe (t), passant par le centre dudit orifice supérieur (15) dudit canal (14) et perpendiculaire audit support (1), sont **confondus lorsque** la valeur du diamètre d2 est égal ou supérieur à la valeur du diamètre d4,

**ou** sont distincts l'un par rapport à l'autre d'une distance d'une valeur **inférieure ou égale à [(valeur de d4 - valeur de dl)/2], lorsque** la valeur du diamètre d4 est supérieur à la valeur du diamètre d2.

Selon un mode de réalisation particulier, l'invention concerne une puce microfluidique de culture cellulaire, dans laquelle ladite face interne (10) de ladite protubérance (8) est en **partie en regard** dudit orifice supérieur (15) dudit canal (14) :

lorsque ladite face interne (10) de ladite au moins une protubérance (8) est **en partie en regard de ladite au moins une perforation (4)** dudit support (1) :

- tel que la valeur du diamètre supérieur d1 de ladite perforation (4) est **supérieure à** la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure ou égale à [(valeur de d1 - valeur de d3) /2],

**et** ledit axe (x) et l'axe (t), passant par le centre dudit orifice supérieur (15) dudit canal (14) et perpendiculaire audit support (1), sont **confondus,**
**ou** ledit axe (x) et ledit axe (y) sont **confondus.**

Selon un mode de réalisation particulier, l'invention concerne une puce microfluidique de culture cellulaire, dans laquelle ladite face interne (10) de ladite protubérance (8) est en partie en regard dudit orifice supérieur (15) dudit canal (14) :

lorsque ladite face interne (10) de ladite au moins une protubérance (8) est en partie en regard de ladite au moins une perforation (4) dudit support (1) :

- tel que la valeur du diamètre supérieur d1 de ladite perforation (4) est supérieure à la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à sont confondus, ou distincts l'un de l'autre d'une valeur inférieure ou égale à [ (valeur de d1 /2) + (4/10 de valeur de d3) ]

et ledit axe (x) et l'axe (t), passant par le centre dudit orifice supérieur (15) dudit canal (14) et perpendiculaire audit support (1), sont distincts l'un par rapport à l'autre d'une distance tel que au moins 1/10 du diamètre d3 soit en totalité en regard du diamètre d4.

Selon un mode de réalisation particulier, l'invention concerne une puce microfluidique de culture cellulaire, dans laquelle ladite face interne (10) de ladite protubérance (8) est en partie en regard dudit orifice supérieur (15) dudit canal (14) :
lorsque ladite face interne (10) de ladite au moins une protubérance (8) est en partie en regard de ladite au moins une perforation (4) dudit support (1) :

- tel que la valeur du diamètre supérieur d1 de ladite perforation (4) est supérieure à la valeur du diamètre d3 de ladite protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à sont confondus, ou distincts l'un de l'autre d'une valeur inférieure ou égale à [ [ (valeur de d3 + valeur de d1) /2] - 10 $\mu$m]
et ledit axe (x) et l'axe (t), passant par le centre dudit orifice supérieur (15) dudit canal (14) et perpendiculaire audit support (1), sont distincts l'un par rapport à l'autre d'une distance tel que au moins 10 $\mu$m du diamètre d3 soit en totalité en regard du diamètre d4.

Dans un mode de réalisation de l'invention, l'unité centrale contient :

- un support constitué d'une membrane non résorbable (1), comportant une face supérieure (2) et une face inférieure (3), perforé par au moins une perforation (4)

- une membrane poreuse nanostructurée en 3D (5) comportant une face supérieure (6) et une face inférieure (7), et comportant au moins une protubérance (8), ladite au moins une protubérance comportant une face externe (9) et une face interne (10) et formant une structure en relief du côté de la face supérieure (6) de la membrane nanostructurée en 3D (5), ladite protubérance étant notamment en forme de dôme creux présentant une base circulaire (13),

ladite face supérieure (6) de la membrane nanostructurée en 3D étant positionnée de façon solidaire sur ladite face inférieure (2) dudit support et ladite au moins une protubérance (8) étant du côté de la face supérieure (2) dudit support (1).

Ainsi, selon un mode particulier, l'invention concerne une puce microfluidique de culture cellulaire dans laquelle ladite unité supérieure (102) dudit module supérieur (101) et ladite unité centrale (105) dudit module central (104) sont assemblées de sorte que ladite surface de l'ouverture de ladite unité supérieure (102) est positionnée au-dessus de ladite face supérieure (2) du support constitué d'une membrane non résorbable (1) de ladite unité centrale (105),

pour former un espace clos délimité par ladite surface pleine de ladite unité supérieure (102), ladite face supérieure (2) du support constitué d'une membrane non résorbable (1) et la face externe (9) des protubérances,
ladite surface de l'ouverture et ladite face supérieure (2) ayant une forme et une surface identiques entre elles,
ladite unité supérieure (102) dudit module supérieur (101) et ladite unité centrale (105) dudit module central (104) étant assemblées par des éléments de fixation (201,204) situés sur chacun des socles (103,106) du module supérieur (101) et du module central (104), de façon à assembler de manière étanche ladite surface de l'ouverture de ladite unité supérieure (102) et ladite face supérieure (2) du support constitué d'une membrane non résorbable (1) de ladite unité centrale (105),
et avantageusement ladite au moins une face pleine de ladite unité supérieure (102) présente deux orifices débouchant respectivement sur un canal entrée/sortie (202), permettant audit espace clos de communiquer avec l'extérieur de ladite puce microfluidique via lesdits canaux entrée/sortie (202) qui débouchent à l'extérieur du socle (109).

Selon un mode de réalisation particulier, l'invention concerne une puce microfluidique de culture cellulaire dans laquelle ladite face interne (10) de ladite au moins une protubérance (8) est en totalité ou en partie en regard de ladite au moins une perforation (4) dudit support (1).

La membrane nanostructurée en 3D comportant au moins une protubérance et le support perforé par au moins une perforation sont positionnés l'un par rapport de façon à ce que la face interne de la dite protubérance soit en totalité ou en partie en regard de la section circulaire de ladite perforation au niveau de la face supérieure du support.

Selon un mode de réalisation particulier, l'invention concerne une puce microfluidique de culture cellulaire dans laquelle ladite face interne (10) de ladite au moins une protubérance (8) est **en totalité en regard de ladite au moins une perforation** (4) dudit support (1) :

- lorsque la valeur du diamètre supérieur d1 de ladite au moins une perforation (4) est **supérieure ou égale** à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **confondus;** ou

- lorsque la valeur du diamètre supérieur d1 de ladite au moins une perforation (4) est **supérieure à** la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont **distincts l'un par rapport à l'autre d'une distance** d'une valeur inférieure ou égale à [(valeur de d1 - valeur de d3)/2].

Selon un mode particulier, l'invention concerne une puce microfluidique de culture cellulaire, comportant un module supérieur (101), un module central (104) et un module inférieur (107), et dans laquelle les susdits modules sont assemblés tel que

ladite surface de l'ouverture de ladite unité supérieure (102) dudit module supérieur (101) est positionnée au-dessus de ladite face supérieure (2) du support constitué d'une membrane non résorbable (1) et de la face externe (9) de ladite au moins un protubérance (8) de ladite unité centrale (105) dudit module central (104),

et l'orifice supérieur (15) du au moins un canal (14), de ladite face supérieure de ladite unité inférieure (108) dudit module inférieur (107), s'ouvre sur au moins une desdites perforations (4) dudit support (1) de ladite unité centrale (105),

ladite surface de l'ouverture de ladite unité supérieure (102) et ladite face supérieure (2) du support constitué d'une membrane non résorbable (1) de ladite unité centrale (105) ayant une forme et une surface identiques entre elles,

ladite face supérieure de ladite unité inférieure (108) et ladite face inférieure dudit support (3) de ladite unité centrale (105) ayant une forme et une surface identiques entre elles,

et ledit module supérieur (101), ledit module central (104) et ledit module inférieur (107) étant assemblés par des éléments de fixation (201, 204) situés sur chacun des socles respectifs (103, 106, 109)

dans laquelle ladite face interne (10) de ladite protubérance (8) est en totalité en regard dudit orifice supérieur (15) dudit canal (14) :

lorsque ladite face interne (10) de ladite au moins une protubérance (8) est en totalité en regard de ladite au moins une perforation (4) dudit support (1) :

- tel que lorsque la valeur du diamètre supérieur d1 de ladite au moins une perforation (4) est supérieure ou égale à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus; ou
- tel que la valeur du diamètre supérieur d1 de ladite au moins une perforation (4) est supérieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à [(valeur de d1 - valeur de d3)/2] ;

et lorsque ladite perforation (4) dudit support (1) est en totalité en regard dudit orifice supérieur (15) dudit canal (14) :
tel que l'axe (y), passant par le centre de ladite base circulaire (13) de ladite protubérance (8) et perpendiculaire audit support (1), et l'axe (t), passant par le centre dudit orifice supérieur (15) dudit canal (14) et perpendiculaire audit support (1), sont confondus, ou distincts l'un par rapport à l'autre d'une distance inférieure ou égale à [(valeur de d4 - valeur de d3)/2] lorsque la valeur de d4 est supérieure à la valeur de d2.

L'invention concerne également un procédé de fabrication d'une puce microfluidique de culture cellulaire selon l'invention, dans lequel la fabrication de ladite unité centrale (105) dudit module central (104) comprend :

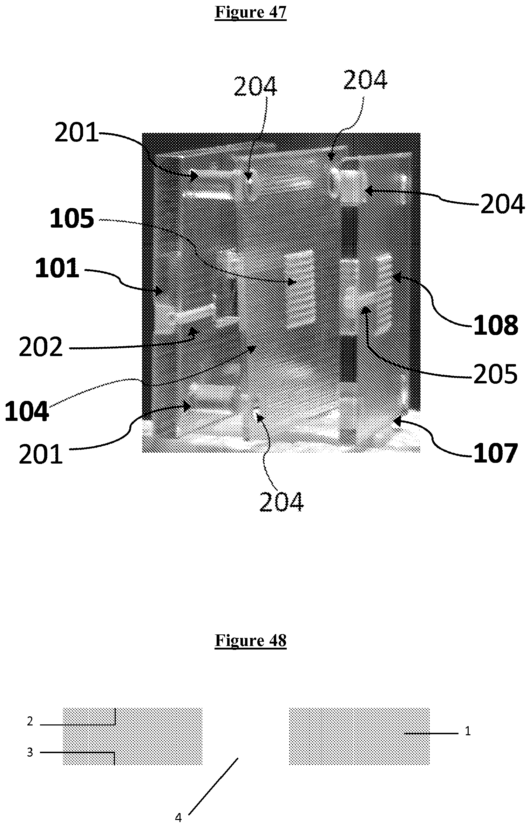

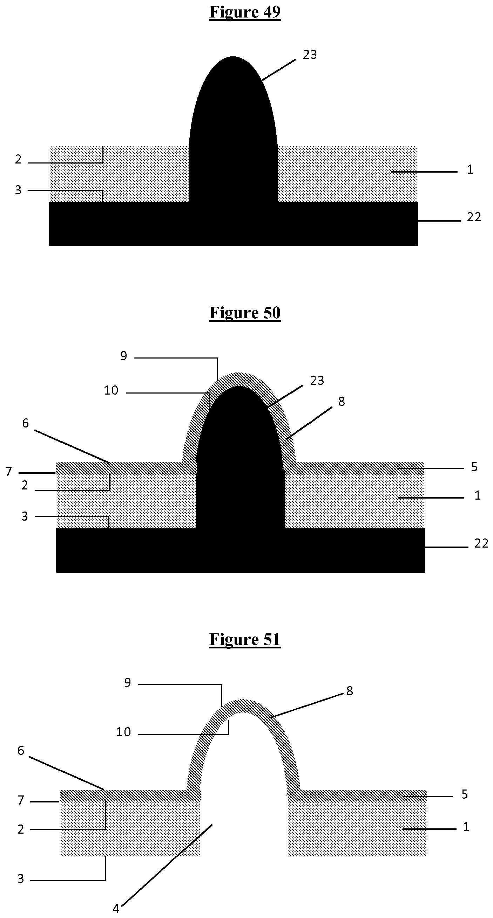

- une étape d'extrusion d'une solution de polymère résorbable (22) à travers la au moins une perforation (4) dudit support (1) pour former au moins une nanostructure 3D (23) du côté de ladite face supérieure (2) dudit support (1) **(Figures 48 et 49),** suivi de

- une étape de polymérisation de ladite solution de polymère résorbable pour rigidifier ladite au moins une nanostructure 3D (23), ladite au moins une nanostructure 3D (23) formant un moule en polymère résorbable (22) du côté de ladite face supérieure dudit support après polymérisation, suivi par

- une étape d'application d'au moins une couche continue d'au moins un polyélectrolyte recouvrant la face supérieure (2) dudit support (1) et la surface dudit moule en polymère résorbable (22), pour constituer la membrane poreuse nanostructurée en 3D (5) **(Figure 50),** par

- une étape de dissolution dudit moule en polymère résorbable pour obtenir ladite face inférieure (7) de ladite membrane poreuse nanostructurée en 3D (5) positionnée de façon solidaire sur ladite face supérieure (2) dudit support (1) et ladite face interne (10) de la au moins une protubérance (8) positionnée en totalité en regard de ladite au moins une perforation (4) **(Figure 51).**

La première étape de fabrication du module central est une étape d'extrusion d'une solution polymérique résorbable à travers une ou des perforations dudit support constitué d'un matériau non résorbable, c'est-à-dire un matériau ne pouvant être éliminé ni par un procédé physique ni par un procédé chimique dans un solvant aqueux.
Le matériau polymérique résorbable utilisé pour la solution est initialement sous forme de liquide visqueux pour permettre l'extrusion à travers les perforations du support.
Le terme « visqueux » est utilisé ici pour qualifier un fluide qui a une résistance à la déformation sous une contrainte de cisaillement, qui est au moins la même que celle de l'eau pure et préférablement sensiblement plus grande que celle de l'eau pure. En d'autres termes, un liquide visqueux utilisé pour l'extrusion circule plus lentement que l'eau pure, propriété due à sa plus grande viscosité que l'eau pure. La propriété de viscosité du liquide utilisé pour l'extrusion est importante, car un tel liquide visqueux permet une meilleure conservation de la forme de la protubérance, pendant le processus d'extrusion, qu'une solution qui a la viscosité de l'eau pure. La plage préférée de la viscosité du liquide pour l'extrusion est comprise de 1 (eau pure) à 100 Pa.s, et de préférence, la viscosité du liquide est comprise de 0,1 Pa.s à 1 Pa.s.
Le terme « extrusion » est ici utilisé pour définir une étape dans laquelle un matériau sous forme de liquide visqueux est passé en force à travers une matrice pour donner une forme prédéterminée par ladite matrice, audit matériau visqueux. Dans le cadre de la présente invention, la matrice est constituée par le support comportant au moins une perforation et ledit matériau à l'état de liquide visqueux est constitué par le polymère résorbable. L'extrusion de ce polymère résorbable à l'état visqueux, de la face inférieur dudit support vers la face extérieure dudit support, au travers de la au moins perforation, permet de donner une forme de nanostructure en 3D audit polymère résorbable du coté de la face supérieure dudit support.

Selon un mode de réalisation particulier, l'invention concerne un procédé de fabrication d'une puce microfluidique de culture cellulaire, dans lequel l'étape d'extrusion de ladite solution en polymère résorbable (22) à travers la au moins une perforation (4) dudit support (1), permet de former des nanostructures 3D (23) du coté de ladite face supérieure (2) dudit support (1) **en forme de dôme.**

La nanostructure 3D obtenue après l'extrusion à travers les perforations et la polymérisation de ladite solution polymérique résorbable, a pour effet technique de servir de moule pour la formation des protubérances de la membrane poreuse nanostructurée en 3D.

L'extrusion est arrêtée lorsque la solution polymérique résorbable a formé une nanostructure 3D de longueur désirée pour servir de moule à la protubérance souhaitée.

La solution polymérique résorbable est alors laissée sans contrainte pour permettre sa polymérisation ou gélification, et ainsi donner des nanostructures 3D solides qui dépassent du coté de la face supérieur dudit support à travers les perforations.

La polymérisation est réalisée directement sur le support constitué d'une membrane non résorbable et comportant au moins une perforation.

Après polymérisation, les nanostructures 3D obtenues permettent de constituer un moule pour l'obtention des protubérances de la membrane poreuse nanostructurée en 3D.

La surface formée par la face supérieure du support et les nanostructures en 3D formées par extrusion et dépassant du coté de la face supérieure dudit support à travers les perforations, est ensuite recouverte par une membrane poreuse constituée d'un film en polyélectrolytes.

Les polyélectrolytes ont la propriété de pouvoir prendre la forme de tout type de structure 3D constitué de n'importe quel type de matériau (même résorbable), et de conserver la forme 3D ainsi donnée, lors du retrait de la structure 3D ayant servi de moule.

Ce film en polyélectrolytes est un film multicouches, réalisé en utilisant la technique couche-par-couche.

Le but de cette technique est d'adsorber des couches successives de polyélectrolytes sur la surface formée par la face supérieure du support et les nanostructures en 3D dépassant à travers les perforations dudit support, pour créer une pluralité de fines couches de polyélectrolytes.

De manière préférentielle, les polyélectrolytes utilisés sont le PAH et le PSS. Les solutions de PAH et de PSS sont préparées à une concentration de 1 mg/ml avec 0.5 mol/l de NaCl.

Le film multicouches de polyélectrolytes est construit en commençant indifféremment par l'un des polyélectrolytes et en terminant indifféremment par l'un des polyélectrolytes, mais en tenant compte de l'alternance des couches entre des polyélectrolytes de charge opposée.

Entre les changements de solution de polyélectrolytes, la surface est rincée rigoureusement avec de l'eau MilliQ (18 MΩ.cm).

Ce procédé est répété jusqu'à obtenir le nombre désiré de couches de polyélectrolytes.

Ce procédé permet d'obtenir un film multicouches en polyélectrolytes final qui constitue un film continu sur la totalité de la surface formée par la face supérieure du support et les nanostructures en 3D dépassant à travers les perforations dudit support.

Ainsi, ce film multicouches en polyélectrolytes final présente des protubérances obtenues par moulage lors de l'application des couches successives de polyélectrolytes sur les nanostructures 3D issues de l'extrusion au travers des perforations du support.

Une fois que la surface formée par la face supérieure du support et les nanostructures en 3D, est recouverte par le film continu multicouches en polyélectrolytes, le moule formé par les nanostructures 3D en matériau polymérique résorbable est dissout.

Il est à noter que l'utilisation d'un support constitué d'un matériau non résorbable, c'est-à-dire un matériau ne pouvant être éliminé ni par un procédé physique ni par un procédé chimique dans un solvant aqueux, permet de conserver ledit support intact à l'issu de cette étape de dissolution.

La dissolution du matériel polymérique résorbable donne un film continu multicouches en polyélectrolytes fermement lié au support, et qui présentent des protubérances conservant la forme des nanostructures 3D sur lesquelles elles ont été moulées. Ce film continu constitue ainsi la membrane poreuse nanostructurée en 3D.

La structure finale composée de la membrane nanostructurée poreuse en 3D fixée sur le support, est facilement manipulable et forme le module central de ladite puce micro fluidique de culture cellulaire.

Ce module central peut être désinfecté en lavant trois fois consécutivement avec de l'éthanol à 70%, puis en lavant trois à quatre fois consécutivement avec du milieu de culture cellulaire pour enlever toute trace résiduelle d'éthanol.

Il est également possible de stériliser le module central en utilisant du gaz d'oxyde d'éthylène.

Selon un mode de réalisation particulier, l'invention concerne un procédé de fabrication d'une puce microfluidique de culture cellulaire, dans lequel l'étape d'extrusion de ladite solution en polymère résorbable (22) à travers la au moins une perforation (4) dudit support (1) est réalisée à **l'aide d'un système de pompage.**

Cette extrusion de la solution polymérique résorbable est réalisée à l'aide d'un système de pompage qui peut être contrôlé manuellement ou mécaniquement (par un moteur).

Le système de pompe peut être constitué par exemple par une seringue ou un piston ou pour induire un flux continu de ladite solution de polymère résorbable (22) à travers ladite au moins une perforation (4).

Dans un autre mode de réalisation, ladite solution de polymère résorbable (22) peut être extrudée à travers ladite au moins une perforation (4) à l'aide de deux plaques plates parallèles qui peuvent être déplacées l'une vers l'autre, soit manuellement, soit mécaniquement, à l'aide d'un moteur par exemple. L'une des plaques plates est positionnée sur la face externe du module supérieur (204) et l'autre plaque plate est positionnée sur la face externe du module inférieur (101), les deux plaques plates étant ainsi positionnées de manière parallèle l'une par rapport à l'autre. Le mouvement desdites plaques plates et parallèles, l'une vers l'autre permet ainsi d'appliquer une force à ladite solution de polymère résorbable (22). Dans un tel cas, la force appliquée à la solution de polymère résorbable (22) oblige ladite solution de polymère résorbable (22) à circuler à travers ladite au moins une perforation (4).

L'extrusion peut être réalisée dans un liquide ou dans l'air.

Selon un mode de réalisation particulier, l'invention concerne un procédé de fabrication d'une puce microfluidique de culture cellulaire, dans lequel l'étape d'extrusion de ladite solution en polymère résorbable (22) à travers la au moins une perforation (4) dudit support (1), est réalisé **dans un liquide.**

Dans le cas de l'extrusion dans un liquide, le liquide est choisi de manière à ne pas provoquer la résorption du polymère utilisé pour former la protubérance.

Dans le cas d'une nanostructure 3D extrudée dans un liquide, lorsque **le liquide est au dessus de la face supérieure du support,** cela entraine une pression statique de fluide qui constitue une force dirigée en sens opposé au développement en longueur de la nanostructure 3D, et agit ainsi à l'encontre du développement en longueur de la nanostructure 3D. Cette magnitude de la pression statique de fluide est calculée en multipliant la densité ($\rho$) du liquide par la profondeur ($t$) du liquide au dessus de la face supérieure du support et par la force de gravité ($g$), soit sous forme de formule $\rho tg$. Cette magnitude de la pression statique de fluide agit de manière à réduire la hauteur de la nanostructure 3D. Par exemple, pour une profondeur de 1 mm d'une solution de sel dilué (densité = 1), solution utilisée dans le cas de mimétisme des fluides extracellulaires du corps, la magnitude de la pression statique de fluide qui agit contre la formation en longueur de la nanostructure 3D est de 9.8 milliPascals.

Comme le module de Young de la solution de polyélectrolyte utilisée en tant que polymère résorbable pour l'extrusion, est en général de l'ordre de 100 à 400 MegaPascals, il y a alors une réduction négligeable de la longueur de la nanostructure 3D formée durant l'extrusion (par exemple, dans le pire des cas, pour un module de Young de 100 MegaPascals, la réduction de la longueur sera de l'ordre de $10^{-5}$%).

Dans le cas d'une nanostructure 3D extrudée dans un liquide, **si le liquide est en dessous de la face inférieure du support,** il n'y a alors pas de force de résistance statique de fluide qui agit à l'encontre de la formation en longueur de la nanostructure 3D. Dans ce cas, la protubérance atteint la même longueur qu'une nanostructure 3D extrudée dans l'air.

Selon un mode de réalisation particulier, l'invention concerne un procédé de fabrication d'une puce microfluidique de culture cellulaire, dans lequel l'étape d'extrusion de ladite solution en polymère résorbable (22) à travers la au moins une perforation (4) dudit support (1), est réalisé **dans l'air.**

Dans le cas d'une nanostructure 3D extrudée dans l'air, il n'y a pas de pression agissant à l'encontre de la formation en longueur de la nanostructure 3D.

L'invention concerne également un procédé de fabrication alternatif d'une puce microfluidique de culture cellulaire selon l'invention, dans lequel la fabrication de ladite unité centrale (105) dudit module central (104) comprend :

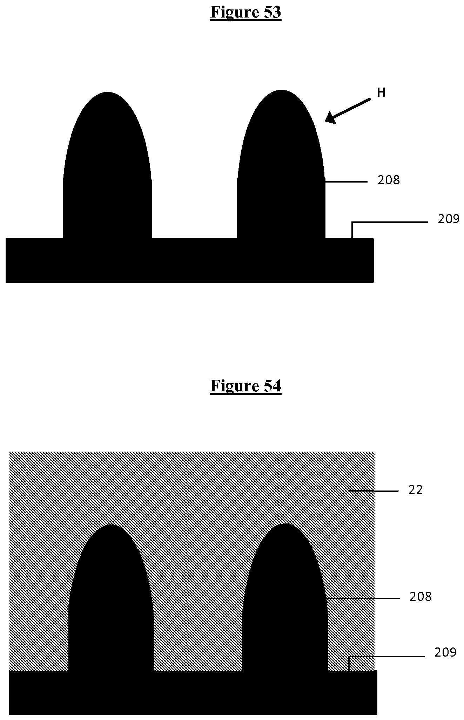

- une étape de coulage d'une solution de polymère résorbable (22) sur la face supérieure (209) d'un moule (H), ledit moule étant en particulier en plastique, comprenant au moins une nanostructure 3D moulée (208) sur ladite face supérieure (209), (figures 53 et 54) suivi de

- une étape de polymérisation de ladite solution de polymère résorbable (22) pour rigidifier ladite solution de polymère résorbable et former une matrice en polymère résorbable (210), suivi de

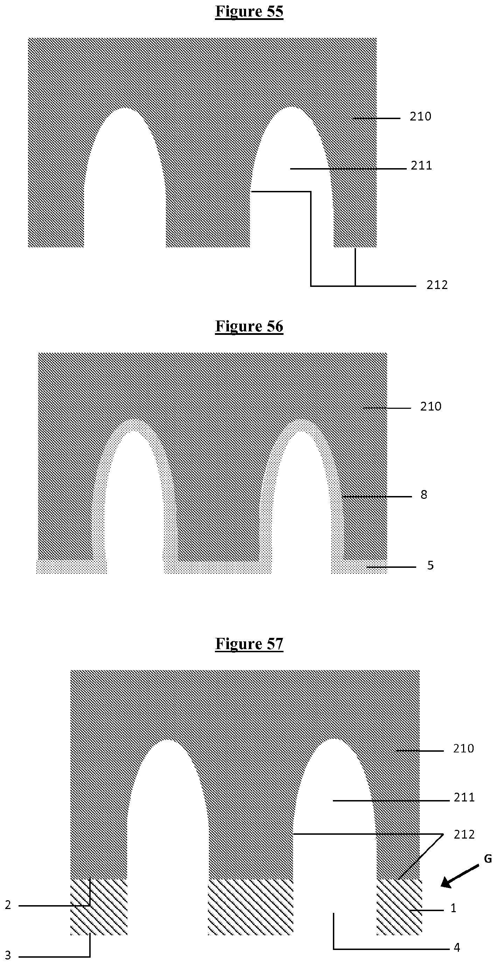

- une étape de retrait dudit moule pour former une matrice en polymère résorbable (210) comportant au moins un moule négatif de ladite au moins une nanostructure 3D (211), (figure 55), suivi de

- une étape d'application d'au moins une couche continue d'au moins un polyélectrolyte sur la face inférieure (212) de ladite matrice en polymère résorbable (210) comportant au moins un moule négatif de la dite au moins une nanostructure 3D (211), pour constituer une membrane poreuse nanostructurée en 3D (5) comprenant au moins une protubérance (8) (Figure 56), suivi de

- une étape de dissolution de ladite matrice en polymère résorbable.

Selon un mode particulier, l'invention concerne également un procédé de fabrication alternatif d'une puce microfluidique de culture cellulaire selon l'invention, dans lequel la fabrication de ladite unité centrale (105) dudit module central (104) comprend :

- une étape de coulage d'une solution de polymère résorbable (22) sur la face supérieure (209) d'un moule (H), ledit moule étant en particulier en plastique, comprenant au moins une nanostructure 3D moulée (208) sur ladite face supérieure (209), (figures 53 et 54) suivi de

- une étape de polymérisation de ladite solution de polymère résorbable (22) pour rigidifier ladite solution de polymère résorbable et former une matrice en polymère résorbable (210), suivi de

- une étape de retrait dudit moule (H) pour former une matrice en polymère résorbable (210) comportant au moins un moule négatif de ladite au moins une nanostructure 3D (211), (figure 55), suivi de

- une étape d'assemblage de ladite matrice en polymère résorbable (210) comportant au moins un moule négatif de ladite au moins une nanostructure 3D (211) avec un support constitué d'une membrane non résorbable (1) comportant au moins une perforation (4), de manière à ce que ledit au moins un moule négatif de ladite au moins une nanostructure 3D (211) soit dans l'alignement de ladite au moins une perforation (4) dudit support (1), (figure 57) suivi de

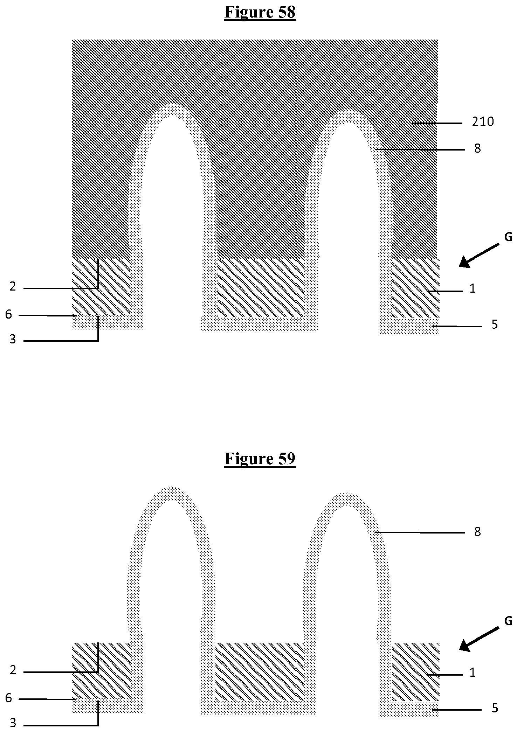

- une étape d'application d'au moins une couche continue d'au moins un polyélectrolyte sur la surface continue formée par la face inférieure (3) dudit support (1) et la face inférieure (212) de ladite matrice en polymère résorbable (210) comportant au moins un moule négatif de la dite au moins une nanostructure 3D (211) au niveau des perforations (4) dudit support (1), pour constituer une membrane poreuse nanostructurée en 3D (5) comprenant au moins une protubérance (8), (figure 58) suivi de

- une étape de dissolution de ladite matrice en polymère résorbable (210) (figure 59).

L'invention concerne également un procédé de fabrication alternatif d'une puce microfluidique de culture cellulaire selon l'invention, dans lequel la fabrication du module central comprenant l'unité centrale, comprend :

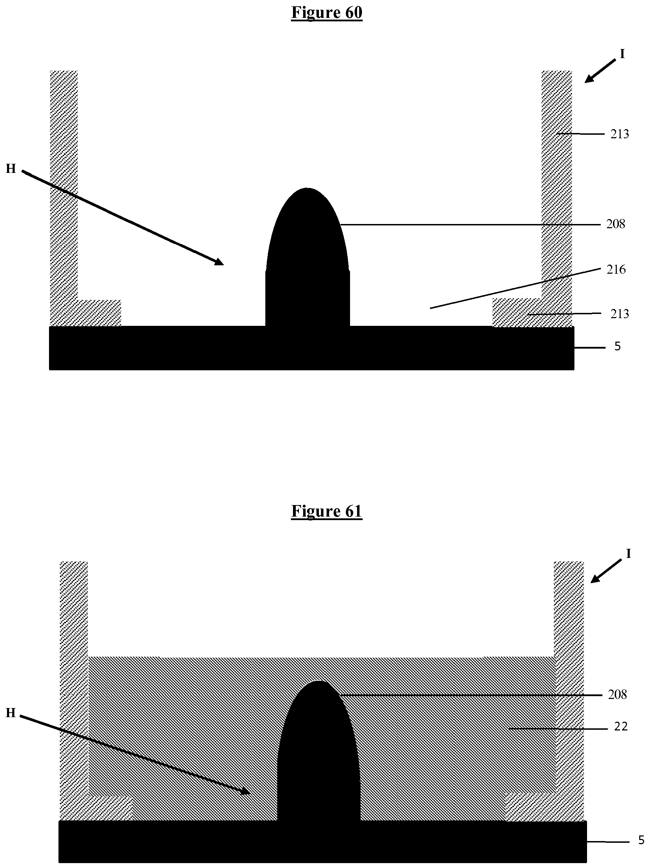

(i) une étape d'assemblage d'une pièce de soutien (I, i), constituée d'un cadre latéral (213), d'une face supérieure ouverte (214) et d'une face inférieure pleine (215) comportant une découpe (216) au format de l'unité centrale, et d'un moule (H, H1, H2, h1, h2), aux formes et dimensions de ladite pièce de soutien (I, i), comprenant au moins une nanostructure 3D moulée (208) sur la face supérieure (209), ledit moule étant en particulier en plastique (figure 60),

(ii) une étape de coulage d'une solution de polymère résorbable (22) sur ladite face supérieure (209) dudit moule (H, H1, H2, h1, h2), (figure 61) suivi de

(iii) une étape de polymérisation de ladite solution de polymère résorbable (22) pour rigidifier ladite solution de polymère résorbable et former une matrice en polymère résorbable (210) comportant au moins un moule négatif de ladite au moins une nanostructure 3D (211), suivi de

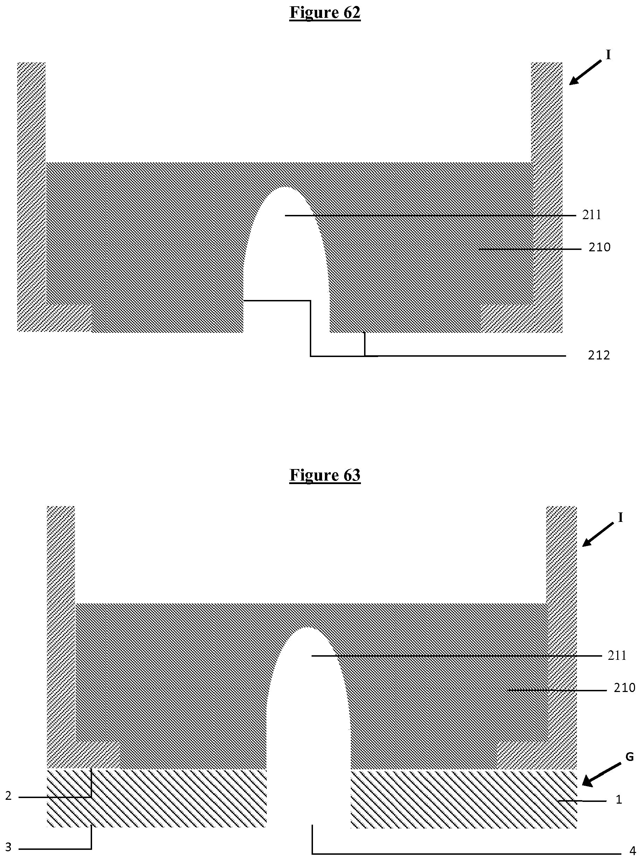

(iv) une étape de retrait dudit moule (H, H1, H2, h1, h2), pour obtenir ladite matrice en polymère résorbable (210) comportant au moins un moule négatif de ladite au moins une nanostructure 3D (211) formée dans ladite découpe (216) de la face inférieure pleine (215) de ladite pièce de soutien (I, i), (figure 62) suivi de

(v) une étape d'assemblage de ladite pièce de soutien (I, i), avec une pièce perforée (G, G1, G2, g1, g2), comprenant un support constitué d'une membrane non résorbable (1) perforé par au moins une perforation (4) intégré dans un socle (106), (figure 63)

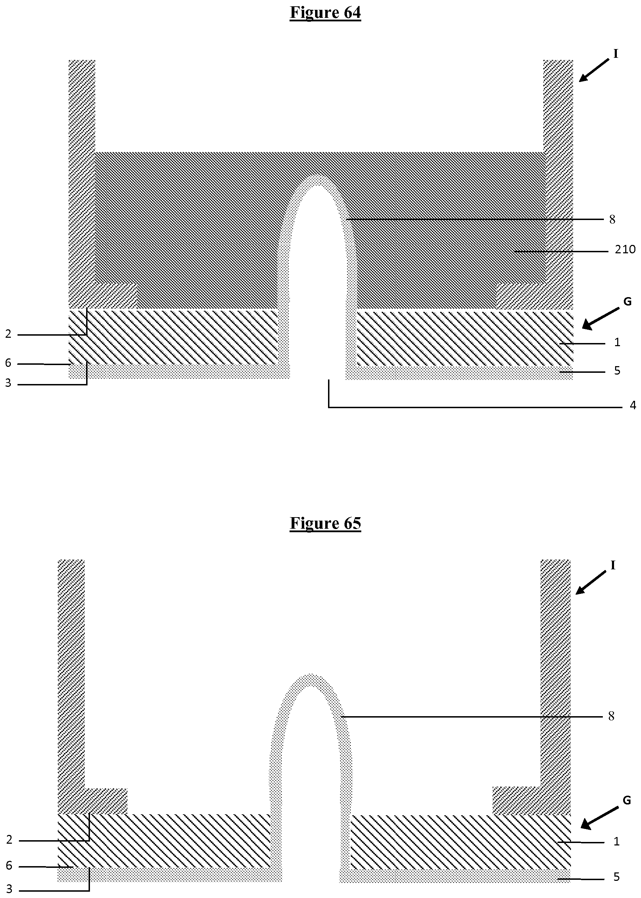

ladite pièce perforée (G, G1, G2, g1, g2) étant aux formes et dimensions de ladite pièce de soutien (I, i), et le nombre de perforations (4) de ladite pièce perforée (G, G1, G2, g1, g2) étant identique au nombre de nanostructures 3D moulées (211) dans ledit moule (H, H1, H2, h1, h2) utilisé à l'étape (i),

de manière à ce que ladite au moins une perforation (4) dudit support (1) soit dans l'alignement avec ledit au moins un moule négatif de ladite au moins une nanostructure 3D (211),

suivi de
(vi) une étape d'application d'au moins une couche continue d'au moins un polyélectrolyte sur la surface continue constituée de la face inférieure (3) dudit support (1) et de la face inférieure (212) de ladite matrice en polymère résorbable (210) comportant au moins un moule négatif de la dite au moins une nanostructure 3D (211), au niveau de ladite au moins une perforation (4) dudit support (1),

pour constituer une membrane poreuse nanostructurée en 3D (5) comportant au moins une protubérance (8) (figure 64), suivi de

(vii) une étape de dissolution de ladite matrice en polymère résorbable (210) comportant au moins un moule négatif de ladite au moins une nanostructure 3D (211), (figure 65),

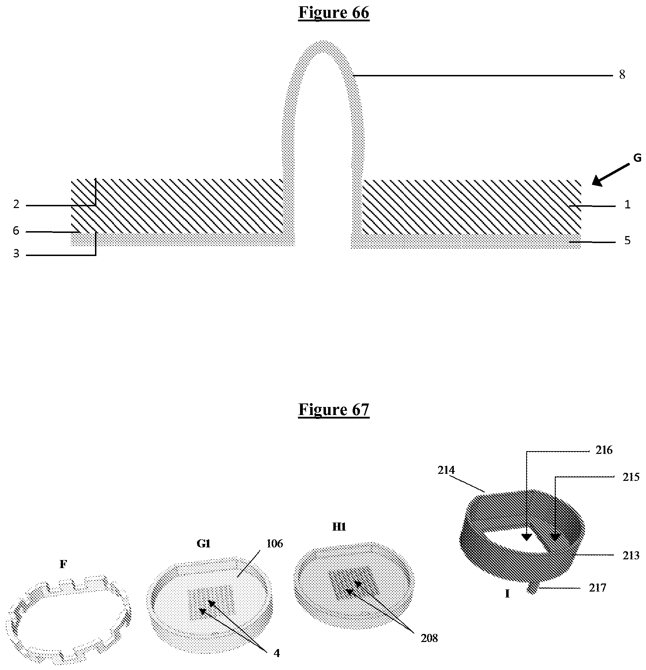

(viii) une étape de retrait de la pièce de soutien (I, i) pour obtenir ladite pièce perforée (G, G1, G2, g1, g2) comprenant sur sa face inférieure (3) une membrane poreuse nanostructurée en 3D (5), et du côté de sa face supérieure ladite au moins une protubérance (figure 66).

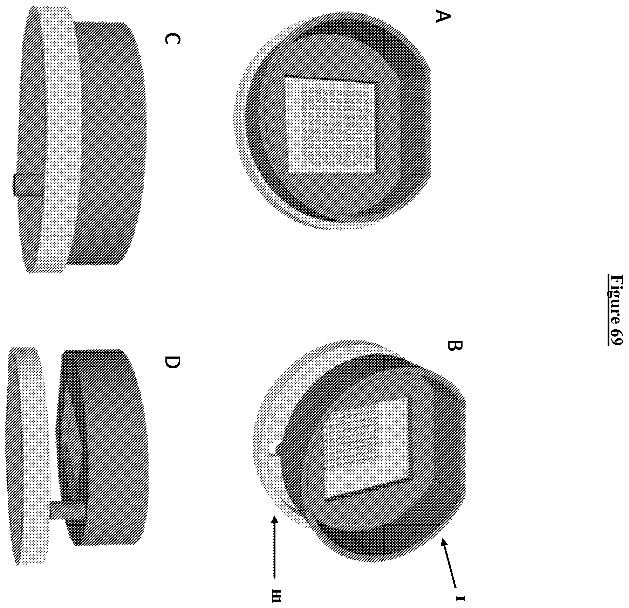

Comme montré à la figure 69, l'étape (i) du procédé décrit précédemment correspond à l'assemblage de la pièce de soutien I avec le moule H, en particulier un moule H1 comprenant 100 nanostructures 3D moulées (208), dans un alignement reproductible et spécifique qui est guidé par la section plate des pièces et la goupille d'alignement (217) de la pièce de soutien I qui s'insère dans le trou d'alignement (218) du moule H1.

Pour l'assemblage de la pièce de soutien I et du moule H, la face pleine (215) de ladite pièce de soutien I est placée sur la face supérieure (209) du moule H, de façon à ce que les nanostructures 3D moulées (208) dudit moule H viennent se positionner au niveau de la découpe (216) de ladite face pleine de la pièce de soutien I.

La chambre ainsi formée par la connexion du moule H avec la pièce de soutien I est ensuite remplie avec le polymère résorbable (22) selon l'étape (ii).

Après polymérisation du polymère résorbable (étape iii), une matrice en polymère résorbable (210) comportant au moins un moule négatif de ladite au moins une nanostructure 3D (211) est formée dans la découpe (216) sur la face pleine (215) de la pièce de soutien I.

Le moule H est ensuite retirée de la pièce de soutien I selon l'étape (iv). La base de la pièce de soutien I est alors formée au niveau de la découpe (216) par une matrice en polymère résorbable (210) comportant au moins un moule négatif d'au moins une nanostructure 3D (211).

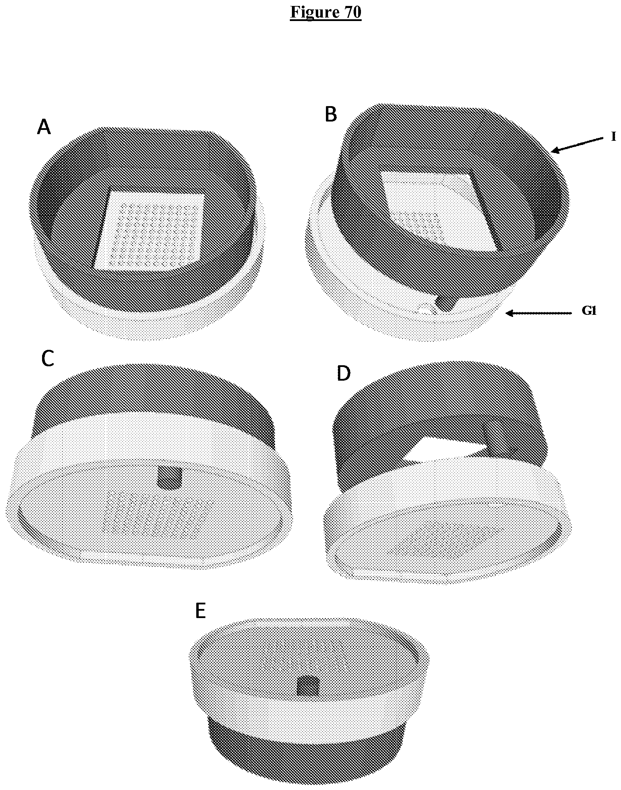

La pièce de soutien I est ensuite assemblée avec la pièce perforée G, en particulier une pièce perforée G1 comprenant 100 perforations (4), selon l'étape (v), dans un alignement reproductible et spécifique qui est guidé par la section plate des pièces et la goupille d'alignement (217) de la pièce de soutien I qui s'insère dans le trou d'alignement (218) de la pièce perforée G (figure 70).

La pièce perforée G est constituée d'un support (1) comprenant au moins une perforation (4), ledit support (1) étant inséré dans un socle (106).

Le choix de la pièce perforée (G1, G2, g1, g2) dépend du choix du moule (H1, H2, h1, h2) dans l'étape (i), la forme et le nombre de nanostructures 3D du moule (H1, H2, h1, h2) devant être identique à la forme et au nombre de perforations de la pièce perforée (G1, G2, g1, g2) (Figures 67, 68, 72, 73).

La face supérieure (2) du support (1) de la pièce perforée G se positionne au contact de la face inférieure (212) de ladite matrice en polymère résorbable (210).

Les perforations (4) de la pièce perforée G sont alors alignées avec les moules négatifs des nanostructures 3D (211) de ladite matrice (210) grâce à la goupille d'alignement (217) de la pièce de soutien I qui s'enclenche dans le trou d'alignement (218) de la pièce perforée G.

Les pièces I et G1 assemblées sont ensuite retournées comme montré à la figure 70-E, de façon à avoir la pièce G1 au-dessus de la pièce de soutien I.

Les parois des perforations de la pièce G1 permettent la formation d'une chambre, de sorte à faciliter la formation des protubérances (8) par l'application d'au moins une couche continue d'au moins un polyélectrolyte selon l'étape (vi), sur la surface continue formée par la face inférieure (3) du support (1) et la face inférieure (212) de ladite matrice en polymère résorbable (210) comprenant au moins un moule négatif de ladite au moins une nanostructure 3D (211), au niveau des perforations (4) dudit support (1).

Une fois la au moins une protubérance (8) formée, le polymère résorbable formant ladite matrice (210) de moules négatifs (211) est dissout selon l'étape (vii).

La pièce de soutien I est alors retirée de la pièce perforée G1.

La pièce perforée G1 comprend alors du côté de la face inférieure (3) du support (1) une membrane poreuse nanostructurée en 3D (5), et du côté de la face supérieure (2) du support (1), ladite au moins une protubérance dans le prolongement de ladite au moins une perforation du support de la pièce perforée G1, (figure 66).

Dans ce mode de réalisation, la face supérieure (6) de la membrane poreuse nanostructurée en 3D (5) est solidaire (au contact) de la face inférieure (3) du support constitué d'une membrane non résorbable (1).

Cette pièce perforée G1 alors obtenue comprenant un support (1) avec des perforations (4) et la membrane nanostructurée en 3D (5) comportant des protubérances (8), constitue le module central de la puce microfluidique et est utilisée pour la culture de cellules de chaque côté de ladite au moins une protubérance.

## Exemples de pièces I, H et G de forme circulaire

Selon un mode de réalisation particulier, ladite pièce de soutien (I), ledit moule (H1, H2) et ladite pièce perforée (G1, G2) sont de forme circulaire, permettant en particulier de s'adapter à des boites de culture cellulaire d'un diamètre de 35 mm.

La figure 67 présente un mode de réalisation particulier du procédé dans lequel ledit moule (H1) et ladite pièce perforée (G1) comportent respectivement 100 nanostructures 3D moulées et 100 perforations.

Un tel procédé permet l'obtention d'une unité centrale avec 100 protubérances.

La figure 68 présente un mode de réalisation particulier du procédé dans lequel ledit moule (H2) et ladite pièce perforée (G2) comportent respectivement 9 nanostructures 3D moulées et 9 perforations.

Un tel procédé permet l'obtention d'une unité centrale avec 9 protubérances.

Ces exemples de nombres de perforations et de nanostructures 3D moulées, ne sont pas exhaustifs.

Le choix du moule et de la pièce perforée dépend du nombre de protubérances désiré pour la membrane nanostructurée en 3D de l'unité centrale.

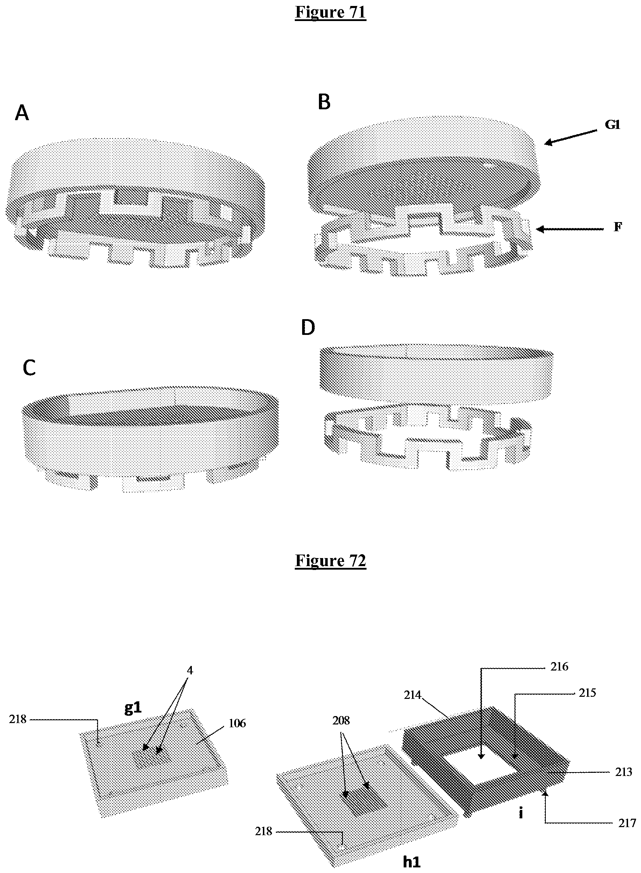

Dans ce mode particulier de réalisation, le module central constitué par la pièce perforée G avec les protubérances obtenue à l'issue du procédé détaillé ci-dessus, peut être placé sur une chambre de culture cellulaire, tel qu'une boite de culture cellulaire de diamètre 35 mm contenant du milieu de culture, par l'intermédiaire d'une pièce F, comme montré à la figure 71.

Selon un mode de réalisation de l'invention, le module central obtenu par le procédé décrit ci-dessus, est placé sur un module inférieur tel que décrit dans la présente invention, comprenant au moins un canal pour collecter les sécrétions de la au moins une protubérance.

Le module inférieur est de forme et de dimensions identiques audit module central.

Le module inférieur est assemblé au module central dans un alignement reproductible et spécifique qui est guidé par la section plate des pièces et la goupille d'alignement de la pièce du module inférieur qui s'insère dans le trou d'alignement du module central.

Le module inférieur comprend un nombre de canaux identique au nombres de perforations et donc de protubérances du module central, de manière à ce que l'assemblage dudit module central avec ledit module inférieur permette l'alignement des canaux avec les perforations et donc les protubérances, pour collecter les sécrétions des cellules via un sytème microfluidique.

Dans ce autre mode de réalisation particulier de la puce microfluidique selon l'invention, le module inférieur est remplacé par la pièce F. Cette pièce F, utilisé comme support du module central sur la boite de culture telle que représentée aux figures 67, 68 et 71 comporte des ouvertures carrées permettant la circulation du milieu de culture pour apporter les nutriments aux cellules en croissance sur la face interne de ladite au moins une protubérance.

### Exemples de pièces I, H et G de forme carrée

Selon un mode de réalisation particulier, ladite pièce de soutien (i), ledit moule (h1, h2) et ladite pièce perforée (g1, g2) sont de forme carrée.

La figure 72 présente un mode de réalisation particulier du procédé dans lequel ledit moule (h1) et ladite pièce perforée (g1) comportent respectivement 100 nanostructures 3D moulées et 100 perforations.

Un tel procédé permet l'obtention d'une unité centrale avec 100 protubérances.

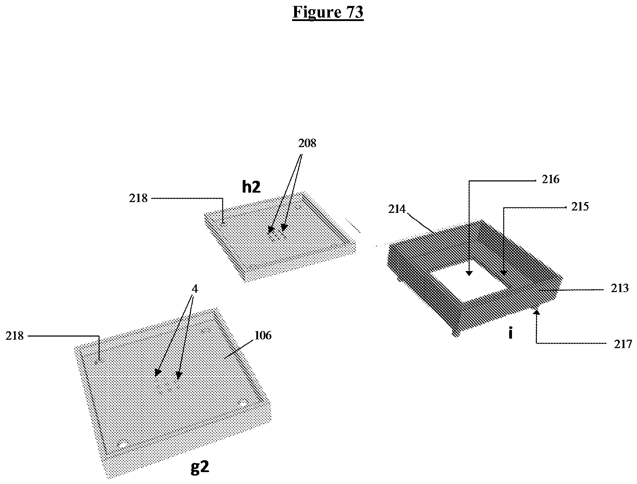

La figure 73 présente un mode de réalisation particulier du procédé dans lequel ledit moule (h2) et ladite pièce perforée (g2) comportent respectivement 9 nanostructures 3D moulées et 9 perforations.

Un tel procédé permet l'obtention d'une unité centrale avec 9 protubérances.

Ces exemples de nombres de perforations et de nanostructures 3D moulées, ne sont pas exhaustifs.

Le choix du moule et de la pièce perforée dépend du nombre de protubérances désiré pour la membrane nanostructurée en 3D de l'unité centrale.

La solution polymérique résorbable est préférablement réalisée avec du chitosan, de l'agarose ou de l'alginate.

Selon un mode de réalisation particulier, l'invention concerne un procédé de fabrication d'une puce microfluidique de culture cellulaire, dans lequel ladite solution en polymère résorbable (22) est du **chitosan.**

Lorsque le chitosan est utilisé, le moule résorbable peut être préparé en dissolvant 2% de chitosan dans 2% d'acide acétique durant une nuit, puis en diluant à 1.5% de chitosan avec de l'éthanol. La solution de chitosan est ensuite polymérisée dans un bain chaud à 5M de NaOH : éthanol à un ratio 1 :1.

Selon un mode de réalisation particulier, l'invention concerne un procédé de fabrication d'une puce microfluidique de culture cellulaire, dans lequel l'étape de polymérisation de ladite solution en polymère résorbable (22), ladite solution en polymère résorbable (22) étant du **chitosan,** est réalisée par une incubation avec une solution à 2% **d'acide acétique.**

Lorsque le chitosan est utilisé comme matériel polymérique résorbable, la dissolution est réalisée par une incubation sur la nuit avec une solution à 2% d'acide acétique, selon un protocole bien connu de l'Homme du métier.

Selon un mode de réalisation particulier, l'invention concerne un procédé de fabrication d'une puce microfluidique de culture cellulaire, dans lequel ladite solution en polymère résorbable (22) est **de l'agarose.**

Lorsque l'agarose est utilisé, le moule résorbable peut être préparé en chauffant et en dissolvant 40 $\mu$g/ml d'agarose dans du PBS (tampon phosphate salin). L'agarose est polymérisé en plaçant la solution obtenue à température au dessous de son point de gélification.

Selon un mode de réalisation particulier, l'invention concerne un procédé de fabrication d'une puce microfluidique de culture cellulaire, dans lequel l'étape de polymérisation de ladite solution en polymère résorbable (22), ladite solution en polymère résorbable (22) étant **de l'agarose,** est réalisée par une incubation à une température supérieure à la température de gélification de l'agarose.

Lorsque l'agarose est utilisé comme matériel polymérique résorbable, la dissolution est réalisée par un chauffage lent à partir de la température ambiante à une température de 70°C, durant 120 minutes, puis en laissant la température de l'agarose revenir à température ambiante sur une nuit.

Ce chauffage peut être réalisé dans un bain-marie. Il est important que la température augmente lentement pour minimiser les courants de convection thermiques qui pourraient abimer la membrane poreuse nanostructurée en 3D.

Des variantes de ce protocole de chauffage, bien connues de l'Homme du métier, incluent l'addition de DMSO dans l'eau du bain-marie pour modifier les propriétés de gélification de l'agarose.

Selon un mode de réalisation particulier, l'invention concerne un procédé de fabrication d'une puce microfluidique de culture cellulaire, dans lequel ladite solution en polymère résorbable (22) est **de l'alginate.**

Selon un mode de réalisation particulier, l'invention concerne un procédé de fabrication d'une puce microfluidique de culture cellulaire, dans lequel l'étape de polymérisation de ladite solution en polymère résorbable, ladite solution en polymère résorbable (22) étant **de l'alginate,** est réalisée par une incubation sur la nuit avec une solution dépourvue de $Ca^{2+}$ et additionnée d'un agent chélateur d'ions $Ca^{2+}$, tel que l'EDTA ou l'EGTA.

Le film multicouches de polyélectrolytes comporte comme paramètres variables :

-   le nombre de couches,

-   l'épaisseur de chacune des couches,

-   la charge du ou des polyélectrolytes utilisés.

En variant le nombre de couches, la rugosité, l'épaisseur et la rigidité du film multicouches final peuvent être modifiées.

De préférence, le film est composé de 15 couches de 2 nm d'épaisseur, de polyélectrolytes.

En variant le nombre de couches ou le type de charge des polyélectrolytes utilisés, l'hydrophobicité du film multicouches final peut également être modifiée.

L'extrusion de la nanostructure 3D peut être sujette aux défauts suivants dus au système de pompage utilisé pour l'extrusion :

-   défaut de translation, lorsqu'il y a translation de la protubérance par rapport à partir de l'emplacement prévu directement aligné avec la perforation du support,

-   défaut d'extrusion, lorsqu'il y a des défauts dans la forme de la protubérance, comme par exemple un épaississement de la base ou d'autres défauts qui seront connus de l'homme du métier.

Une protubérance ainsi formée à partir de nanostructures 3D avec un défaut de translation ou un défaut d'extrusion peut continuer d'exercer sa fonction technique prévue initialement au sein du dispositif, cependant, comme la protubérance ainsi formée possède une forme moins optimale, la performance de celle-ci au sein du dispositif est également moins optimale. Toutefois, le dispositif peut continuer d'exercer sa fonction prévue, mais avec une performance réduite.

La protubérance peut présenter différents changements tels que :

-   une inclinaison par rapport à un axe (y), passant par le centre de ladite ouverture et qui est perpendiculaire audit support,

-   une variation de sa hauteur,

-   une translation par rapport à la perforation, due à la translation de la membrane poreuse nanostructurée en 3D sur le support.

Ces changements sont dus au procédé de préparation du module central, et en particulier à la phase d'extrusion de la solution polymérique à travers les perforations dudit support.

Certains changements sont également entraînés directement lors de l'utilisation de la protubérance dans le dispositif.

## I- Exemple d'utilisation de la puce pour une co-culture

### 1. Conditions de maintien de cultures lignées de cellules épithéliales de prostate et de cellules stromales

Le milieu de culture utilisé pour l'ensemble des expériences est un milieu Kératinocyte sans sérum (KSFM, Kératinocyte Sérum Free Medium) (Life Technologies, Carlsbad, CA, Ref. 17005-075) supplémenté de 5 ng/mL de facteur de croissance épidermal (EGF) et de 50 $\mu$g/mL d'extrait pituitaire bovin.

Les lignées de cellules épithéliales de prostate et de cellules stromales sont maintenues dans ce milieu et sont cultivées dans une atmosphère à 37°C et 5% $CO_2$.

Le repiquage des cellules dans un milieu frais est effectué tous les trois jours pour les cellules épithéliales et tous les deux jours pour les cellules stromales. Pour cela, les cellules sont lavées avec une solution de tampon phosphate salin de Dulbecco (D-PBS) sans calcium et sans magnésium (Life Technologies, Réf. 14190), puis incubées avec 1 mL de Trypsine-EDTA à 0,25 mg/mL, à 37°C, (Lonza, Basel, CH, Réf. CC-5012) pendant environ 7 minutes. Pour l'ensemble des expériences, le milieu de culture des cellules a été supplémenté chaque jour avec du milieu de culture frais.

### 2. Préparation des cellules avant l'introduction dans l'unité centrale

Une dissociation chimique des cellules est réalisée par une incubation de 5 minutes à 37°C avec 1 ml de la trypsine-EDTA à 0,25 mg/ml (Life Technologies, Réf. 25300-054) dans du milieu PBS sans calcium et sans magnésium.

Indépendamment, une puce microfluidique selon l'invention est stérilisée en faisant circuler une solution à 70% d'éthanol (volume/volume) à travers les canaux, puis en séchant l'ensemble du système microfluidique dans un four à une température entre 35°C et 45°C pendant au moins 30 minutes, puis en l'exposant à un rayonnement U.V. et à l'ozone pendant 40 min.

### 3. Préparation de la membrane poreuse nanostructurée en 3D de l'unité centrale

La membrane poreuse nanostructurée en 3D est constituée de couches successives de polyélectrolytes alternant une couche de polyélectrolyte chargé positivement et une couche de polyélectrolyte chargé négativement. Selon le procédé de fabrication, cette même membrane constitue les protubérances.

La face externe et la face interne des protubérances, constituées de la membrane poreuse en polyélectrolytes, sont recouvertes d'une préparation de matrice extracellulaire (ECM) composée de Matrigel® et/ou de collagène, de fibronectine ou d'acide hyaluronique.

La matrice Matrigel® utilisé ici est un produit commercial fabriqué par la société Corning®.

Il s'agit d'une préparation de membrane basale reconstituée qui est extraite de sarcome Engelbreth-Holm-Swarm (EHS) de souris, tumeur riche en protéines de matrice extracellulaire. Une fois isolée, cette matière est composée d'environ 60% de laminine, de 30% de collagène IV, et de 8% d'entactine. L'entactine est une molécule de pontage qui interagit avec la laminine et le collagène IV, et contribue à l'organisation structurelle de ces molécules de la matrice extracellulaire.

La matrice Matrigel® de Corning® contient également des protéoglycanes héparane sulfate (perlecan), du facteur de croissance transformant $\beta$ (TGF-$\beta$), du facteur de croissance épidermique, du facteur de croissance de type insuline, du facteur de croissance de fibroblastes, un activateur tissulaire du plasminogène et d'autres facteurs de croissance qui sont naturellement présent dans la tumeur EHS. Il contient également des métalloprotéinases de matrice résiduelles dérivées de cellules tumorales.

Le Matrigel® peut être utilisé seul pour fonctionnaliser la membrane poreuse, à une concentration de 6 mg/ml, ou en mélange avec du collagène de type I à une concentration comprise entre 0,75 et 2,5 mg/ml.

### 4. Introduction des deux types cellulaires et co-culture cellulaire dans l'unité centrale

4.1.Introduction des cellules épithéliales

Dans un premier temps, les cellules épithéliales sont introduites pour former une couche jointive de cellules, c'est-à-dire une culture de cellules au stade de la confluence.

Selon un mode de réalisation particulier, les cellules épithéliales sont introduites sur les faces internes des protubérances de l'unité centrale.

Trois heures sont requises pour obtenir l'adhésion des cellules et 24h pour la formation d'une couche de cellules jointives, c'est-à-dire à un stade de confluence des cellules. Ces cellules adhérentes et prolifératives sécrètent leur propre matrice extracellulaire et établissent ainsi une couche basale jouant le rôle de barrière.

La face interne des protubérances de l'unité centrale est ainsi recouverte d'une monocouche dense de cellules épithéliales, qui sert de support physiologiquement pour la croissance et la différenciation des cellules humaines, isolées à partir de l'urine du patient.

L'introduction des cellules sur la face interne des protubérances peut se faire selon 3 modes :

- retourner le module central afin d'avoir les sections inférieures des perforations vers le haut et pipeter manuellement une suspension cellulaire.
- retourner le module central afin d'avoir les sections inférieures des perforations vers le haut et utiliser un robot manipulateur de fluides pour introduire une suspension cellulaire.
- assembler le module supérieur, le module central et le module inférieur et remplir l'unité centrale du côté de la face interne des protubérances à l'aide des canaux microfluidiques de l'unité inférieure. Dans le cas de ce pré-assemblage des trois modules, les cellules sont donc introduites via les canaux du module inférieur. Ce mode d'introduction des cellules après un pré-assemblage des trois modules est préféré aux deux autres modes, car il prévient toute contamination bactérienne du fait que le système préalablement stérilisé est maintenu clos.

Selon un mode de réalisation particulier, les cellules épithéliales sont introduites à une concentration de $3.10^6$ cellules/mL dans l'unité centrale soit directement via les perforations (1er et 2ème mode) avec une seringue, soit via les canaux de l'unité inférieure (3ème mode) en utilisant un système fluidique automatisé et contrôlé en pression et débit (Fluigent) ou un pousse-seringue.

L'utilisation d'une pompe à seringue avec un débit réglable est préférée afin de fournir une introduction douce et contrôlée des cellules.

Un flux stable et continu est délivré par l'utilisation de pompes à pression (Fluigent, France). Des récipients pressurisés contenant du milieu de culture sont maintenus dans une chambre à température et $CO_2$ contrôlés. Le débit est ajusté à environ 5-10 mL/h (10 mbar) et l'adhésion et la prolifération des cellules est observée dans le temps. Tous les échantillons sont gardés dans un incubateur humidifié à 37°C et 5% $CO_2$.

Dans un mode de réalisation particulier, l'unité centrale comporte des protubérances d'une hauteur de 350 $\mu$m avec une base circulaire de 150 $\mu$m de diamètre. L'aire de la surface interne de la protubérance est alors

de 329 700 $\mu m^2$, sur laquelle environ 50 cellules épithéliales sont dénombrées au stade de confluence (couche de cellules jointives), soit environ une cellule pour 66 $\mu m^2$.

4.2.Introduction des cellules sur la face externe des protubérances

Dans un second temps, une fois la couche de cellules épithéliales jointives (ou confluentes) formée sur la face interne des protubérances, les cellules stromales sont dispensées sur la membrane poreuse au niveau des faces externes et internes des protubérances.

L'introduction des cellules sur la face interne des protubérances peut se faire selon deux modes :

- retourner le module central afin d'avoir les sommets des protubérances vers le haut et pipeter manuellement une suspension cellulaire.

- assembler le module supérieur, le module central et le module inférieur et remplir l'unité centrale du côté de la face interne des protubérances à l'aide des canaux microfluidiques de l'unité inférieure. Dans le cas de ce pré-assemblage des trois modules, les cellules sont donc introduites via les canaux entrée/sortie de l'unité supérieure. Ce mode d'introduction des cellules après un pré-assemblage des trois modules, est préféré aux autres modes, car il prévient toute contamination bactérienne du fait que le système préalablement stérilisé est maintenu clos.

Selon un mode de réalisation particulier, les cellules stromales sont introduites via les canaux entrée/sortie du module supérieur à une concentration de $3.10^6$ cellules/mL dans l'unité centrale soit directement à l'aide d'une seringue (1er mode), soit via les canaux du module supérieur (2$^{ème}$ mode) en utilisant un système fluidique automatisé et contrôlé en pression et débit (Fluigent) ou un pousse-seringue.

Les cellules stromales adhèrent très rapidement (moins d'une heure).

Il n'est pas nécessaire que les cellules stromales forment une couche de cellules confluentes (ou jointives), leur simple adhésion sur la face externe dans cet exemple suffit.

De manière générale, le ratio entre les cellules épithéliales et les cellules stromales est de 1 :2.

Ainsi, selon un mode de réalisation particulier, pour une co-culture sur une surface de 0.7 cm$^2$, la membrane poreuse au niveau des faces externes et internes des protubérances est fonctionnalisée avec 90 $\mu$l d'une solution de Matrigel® diluée à 6 mg/ml, puis ensemencée pour obtenir au final 7000 cellules épithéliales/cm$^2$ et 14 000 cellules stromales (fibroblastes)/cm$^2$.

Le milieu de culture, introduit via les canaux entrée/sortie du module supérieur et via les canaux du module inférieur pour alimenter les cultures cellulaires, est identique de part et d'autre des protubérances et est constitué par du milieu de culture KSFM supplémenté de 5 ng/mL de facteur de croissance épidermal (EGF) et de 50 $\mu$g/mL d'extrait pituitaire bovin.

4.3.Exemples de cellules épithéliales et de cellules stromales

Ces cellules épithéliales peuvent être des lignées cellulaires commerciales non tumorigènes (prostate ou vessie ou rein) ou des cultures primaires commerciales.

Ces cellules stromales peuvent être :

- soit des fibroblastes (cultures primaires commerciales ou lignées),
- soit des cellules mésenchymateuses (cultures commerciales ou lignées),
- soit autres cellules stromales (endothéliales, ...).

Les deux types de cellules utilisés pour former ces monocouches cellulaires, sont dites « neutres » ou « saines », elles sont non tumorigènes et jouent uniquement un rôle de couche basale. Ces cellules « neutres » forment au stade de la confluence une couche très jointive de cellules sur la face interne et externe des protubérances, établissant des jonctions serrées qu'il est possible de caractériser par immunofluorescence et imagerie (cf. marquage de E-cadhérine partie 5).

4.4.Interchangeabilité des cultures sur les faces internes et externes des protubérances

Selon un mode de réalisation particulier, les cellules épithéliales sont introduites sur la face interne des protubérances et les cellules stromales sont introduites sur la face externe des protubérances.

Cependant, la co-culture peut être établie de façon interchangeable, c'est-à-dire que les cellules stromales peuvent également être introduites sur la face interne des protubérances, et les cellules épithéliales sur la face externe des protubérances. Dans les deux cas, la couche de polyélectrolyte se trouvant entre les deux types cellulaires, permet de constituer une barrière poreuse grâce à son maillage de polyélectrolytes chargés positivement et négativement.

**5. Visualisation des cellules dans l'unité centrale (Preuve de concept de la co-culture sur les protubérances)**

Afin de valider la méthode de co-culture sur les protubérances de l'unité centrale, un immunomarquage est réalisé.

Cet immunomarquage est donc effectué sur des cellules mortes (fixées par du PFA) et cette visualisation a pour seul but de contrôler que la co-culture est bien en place et que la méthodologie d'introduction des cellules est correcte.

Les cellules sont visualisées dans le module central par immunomarquage.

- La phalloïdine est utilisée pour identifier les filaments d'actine corticale, qui suivent les contours de la membrane plasmique et, par conséquent fournissent un moyen pour délimiter l'étendue de la cellule et de sa membrane.
- L'E-cadhérine est utilisée pour détecter les jonctions cellule-cellule.

  L'immunocoloration est réalisée en introduisant l'E-cadhérine avec une pompe à seringue via les canaux à température ambiante.

  Après la formation d'une couche confluente de cellules épithéliales, environ 24h après leur introduction, elles sont fixées pendant 20 minutes avec 4% de Perfluoroalkoxy (PFA) (volume à volume) dans une solution composée de 10% de sucrose dans un tampon cytosquelette (solution A).

  Les cellules sont ensuite lavées avec de la solution A et perméabilisées pendant 3 minutes avec une solution A additionnée de 0,1% de Triton TX-100. Un lavage avec une solution de TBS est réalisé pendant 10 minutes, suivi d'un second lavage avec une solution de PBS pendant 30 minutes. L'autofluorescence du PFA est inactivée par le $NH_4Cl$ contenu dans la solution de TBS. Les sites non spécifiques sont bloqués par une incubation avec une solution de PBS à 10% de sérum de chèvre et à 3% de BSA. Les cellules sont ensuite incubées avec un anticorps primaire pendant une heure. L'anticorps primaire utilisé est un anticorps anti-E-cadhérine (Abcam, Ref. ab1416) dilué au 1/50 dans une solution de PBS à 0.1% de Tween-20 et 1% de BSA. Les cultures sont ensuite lavées pendant 30 minutes avec une solution de PBS, puis incubées avec un anticorps secondaire anti-souris couplé au cytochrome Cy3 (Jackson, Ref. 115- 162-062), dilué au 1/1000 et de la Phalloidin FITC (Sigma, Ref. P5282) diluée au 1:1000 dans une solution de PBS à 0.1% de Tween-20 et 1% de BSA, pendant 20 minutes.

- Après un lavage de 30 minutes avec une solution de PBS, les noyaux sont contre-colorés avec du colorant Hoechst (Life Technologies, Réf. H- 1399), dilué au 1:7000, pendant 5 minutes. Les cellules sont ensuite lavées pendant 10 minutes et le milieu fluorescent Dako est introduit manuellement.
- Les points focaux d'adhésion ont été détectés par marquage en utilisant de la Vinculine. Pour le contremarquage à la Vinculine, les cellules sont pré-perméabilisées pendant 40 secondes avec du Triton X-100 et fixées avec une solution de PBS à 4% de PFA (v/v), pendant 20 minutes, puis lavées une fois avec une solution de PBS.

  Pour éviter toute adsorption non spécifique d'anticorps, les cellules sont incubées avec une solution à 0.1% de BSA et à 10% de sérum de chèvre pendant une heure.
  Les cellules sont ensuite incubées pendant une heure avec un anticorps primaire dirigé contre la Vinculine (Sigma, Ref. V9131) dilué à 1:700 dans une solution de PBS à 0.05% de Tween 20 et 5% de sérum de chèvre, puis lavées 4 fois consécutives pendant 45 minutes avec une solution de PBS.
  Les cellules sont ensuite incubées avec un anticorps anti-souris couplé au cytochrome Cy5, dilué 1/500 dans une solution de PBS à 0,05% de Tween 20 et à 5% de sérum de chèvre (Jackson).

Le module central est ensuite lavé 4 fois pendant 15 minutes avec une solution de PBS. Les noyaux et l'actine sont colorés comme décrit ci-dessus.

La co-culture est observée par microscopie à fluorescence ou peut être observée par d'autres modes de microscopie tel que la microscopie à contraste de phase, l'imagerie sans lentilles, la microscopie confocale, la microscopie à feuille de lumière.

Les images sont capturées durant la culture cellulaire.

Pour fournir une vue d'ensemble de la largeur totale du dispositif, des images de cellules sont enregistrées à l'aide d'un capteur sans lentille. Des analyses SEM sont également réalisées.

Dans un mode de réalisation particulier, les images en fluorescence du module central contenant la co-culture de cellules, sont obtenues à l'aide d'un microscope Zeiss AxioImager Z1 avec un objectif 20x équipé du module droit Apotome pour les acquisitions en profondeur de champ z-stack, avec la prise d'image tous les 3 mm dans l'axe z, pour un tube de 150 mm de diamètre. Les images sont enregistrées à l'aide d'une caméra monochrome digital AxioCam MRm montée sur le microscope.

**6. Visualisation des cellules dans l'unité centrale en temps réel**

Les cultures cellulaires dans l'unité centrale peuvent être suivies en temps réel par une observation au microscope en contraste de phase qui permet de visualiser les cellules non marquées et vivantes, du fait de la transparence des matériaux constituant les modules.

**II- Exemple d'utilisation de la puce pour le diagnostic**

**1. Introduction des cellules provenant du patient**

Selon un mode de réalisation particulier, les cellules épithéliales sont introduites sur la face interne des protubérances et les cellules stromales sont introduites sur la face externe des protubérances.

Une fois une monocouche de cellules obtenue sur chacune des faces, soit après 24h, la puce microfluidique, ainsi pourvue de cellules, peut être utilisée pour le diagnostic d'un patient.

Pour cela, des cellules sont isolées à partir d'un échantillon d'urine de patient d'au moins 50 mL, en particulier de 50 à 100 ml. L'isolement est réalisé par centrifugation de l'échantillon d'urine à une faible vitesse, en particulier 800 g pendant 5 min, permettant la sédimentation des cellules contenues dans l'échantillon d'urine. Cette étape de centrifugation est bien connue de l'Homme du métier.

Le culot de cellules sédimentées est ensuite resuspendu dans du milieu de culture et la suspension de cellules est directement introduite dans la puce microfluidique selon l'invention, ce qui signifie que les cellules n'exigent pas de pré-culture avant leur introduction dans la puce.

La concentration des cellules obtenue à partir de l'échantillon d'urine est ou varie de quelques centaines de cellules à plusieurs milliers.

Les cellules isolées de l'urine du patient peuvent être introduites du côté de la face de la protubérance qui supporte la culture des cellules épithéliales, ou du côté de la face de la protubérance qui supporte la culture des cellules stromales. Autrement dit, ces cultures étant interchangeables de part et d'autre de la protubérance, les cellules isolées de l'urine du patient peuvent être introduites aussi bien sur la face interne que sur la face externe des protubérances.

Selon un mode de réalisation particulier, les cellules isolées de l'urine du patient sont introduites du côté de la face de la protubérance qui supporte la culture des cellules épithéliales. Ainsi, elles sont introduites soit via les canaux de l'unité inférieure, lorsque la monocouche de cellules épithéliales est formée du côté de la face interne des protubérances, soit via les canaux entrée/sortie du module supérieur lorsque la monocouche de cellules épithéliales est formée du côté de la face externe des protubérances.

Les cellules isolées à partir de l'urine du patient sont des cellules uroépithéliales (ou urothéliales) exfoliées, incluant à la fois les cellules épithéliales de vessie, de prostate et de rein.

Dans un mode de réalisation particulier, la face interne des protubérances est recouverte d'une couche pré-formée de cellules épithéliales préalablement cultivées, la face externe des protubérances est recouverte d'une couche pré-formée de fibroblastes (cellules stromales), et les cellules isolées sont dispensées via les canaux du module inférieur.

Ces cellules isolées s'insèrent dans cette couche pré-formée de cellules épithéliales saines du coté de la face interne des protubérances, et qui est supportée par une couche de fibroblastes sains.

**2. Observation de la prolifération des cellules provenant du patient**

La prolifération des cellules isolées est alors suivie, afin d'observer la progression de la prolifération des cellules isolées dans le dispositif et examiner si cette prolifération aboutit au remplacement des cellules basales saines et affecte le profil sécrétoire globale du tissu.

**3. Récupération des sécrétions**

Une fois l'introduction des cellules isolées à partir de l'urine du patient réalisée, les cellules épithéliales de patients sont stimulées par ajout de 0,1 ng/mL de DHT (Dihydrotestostérone) sur la face externe ou interne de la protubérance Cette stimulation des cellules par DHT dure entre 24h et 48h.

La membrane constituant les faces externes et internes des protubérances étant poreuse, cette stimulation peut être effectuée indifféremment d'un côté ou de l'autre des protubérances.

Les cellules épithéliales peuvent également être stimulées par l'ajout de mibolerone (hormone non métabolisée).

La stimulation des cellules épithéliales est ainsi, réalisée après l'adhésion des deux types cellulaires de part et d'autre des protubérances, et après leur croissance jusqu'au stade de confluence.

Les sécrétions peuvent être récupérées lorsque les cellules isolées du patient adhèrent et s'insèrent dans cette couche pré-formée de cellules épithéliales saines du coté de la face interne des protubérances, et qui est supportée par une couche de fibroblastes sains du coté de la face externe des protubérances. L'adhésion des cellules isolées du patient dure environ 3 h et leur intégration dure environ 6h.

L'accumulation d'un volume suffisant de sécrétions intervient progressivement.

La récupération finale des sécrétions pour l'analyse du sécrétome se fait après avoir laissé passer au moins 12h.

Plus particulièrement, les sécrétions sont récupérées au terme des 24 à 48h de stimulation au DHT.

Elles sont ensuite analysées par un dispositif permettant l'analyse de composés en solution. Selon un mode de réalisation particulier, le sécrétome est analysé par spectrométrie de masse.

Les sécrétions peuvent être analysées en ligne par des capteurs incorporés dans ladite puce.

Il est à noter que les différents modules composant ladite puce ne sont pas affectés lorsque les sécrétions sont récupérées ou lorsque les sécrétions sont analysées en continu par des capteurs en ligne.

La recherche de marqueurs spécifiques par des méthodes immunologiques peut également être réalisée dans les sécrétions récupérées.

Par exemple, la détection du PSA (antigène spécifique de la prostate), biomarqueur de référence du cancer de la prostate, peut être effectuée.

Pour une protubérance d'une hauteur de 350 μm et d'une base circulaire d'un diamètre de 150 μm, le volume de sécrétions récupéré au bout de 24h est d'environ 2 nL.

La détection et la quantification du PSA est réalisé par test Elisa.

Pour cela, environ 50 μl de milieu à l'intérieur de plusieurs protubérances sont collectés puis déposés dans une plaque 96 puits, placée à 37°C durant 45 min. Cinq lavages successifs à l'eau distillée sont nécessaires afin de bien éliminer les protéines non fixées à l'anticorps primaire anti-PSA.

100 μL d'anticorps secondaire anti-PSA libre couplés à la HRP (Horseradish peroxidase) sont ensuite ajoutés dans chaque puits avant 45 min d'incubation à 37°C de la plaque ELISA. Enfin, 100 μL de substrat (TMB) sont ajoutés donnant lieu à une réaction colorimétrique enzyme substrat.

Après 15 min à 37°C, la réaction est arrêtée par l'ajout de 100 μL d'acide sulfurique et l'absorbance est détectée à l'aide d'un lecteur de plaque ELISA à 450 nm.

### III- Exemple d'utilisation de la puce pour le criblage de molécules

Dans un mode de réalisation particulier, la puce microfluidique de culture cellulaire selon l'invention, est utilisée pour le criblage de molécules.

### IV- Exemple d'utilisation de la puce pour déterminer l'effet d'un traitement de cancers urologiques sur un patient

Dans un mode de réalisation particulier, la puce microfluidique de culture cellulaire selon l'invention, est utilisée pour déterminer l'effet d'un traitement pour un cancer urologique sur un patient atteint d'un cancer urologique.

Dans ce mode de réalisation, l'analyse du sécrétome des cellules isolées de l'urine du patient, insérées dans la culture de cellules épithéliales sur la protubérance, est effectuée avant et après le traitement du patient, et/ou pendant le traitement.

La comparaison du sécrétome obtenu avant le traitement avec celui obtenu après le traitement, et/ou celui obtenu pendant le traitement, permet de déterminer l'effet du traitement sur le cancer urologique dont est atteint le patient.

### FIGURES

1 support constitué d'une membrane non résorbable (unité centrale)

2 face supérieure du support constitué d'une membrane non résorbable (unité centrale)

3 face inférieure du support constitué d'une membrane non résorbable (unité centrale)

4 perforation du support constitué d'une membrane non résorbable (unité centrale)

5 membrane poreuse nanostructurée en 3D (unité centrale)

6 face supérieure de membrane poreuse nanostructurée en 3D (unité centrale)

7 face inférieure de membrane poreuse nanostructurée en 3D (unité centrale)

8 protubérance (unité centrale)

9 face externe de protubérance (unité centrale)

10 face interne de protubérance (unité centrale)

11 section de la perforation au niveau de la face supérieure du support (unité centrale)

12 section de la perforation au niveau de la face inférieure du support (unité centrale)

13 base circulaire de la protubérance (unité centrale)

14 canal (unité inférieure)

15 orifice supérieur du canal (unité inférieure)

16 orifice inférieur du canal (unité inférieure)

17 réservoir (unité inférieure)

18 canal du réservoir (unité inférieure)

19 orifices de l'unité supérieure débouchant sur les canaux entrée/sortie (unité supérieure)

20 orifice supérieur du canal (module inférieur)

21 orifice inférieur du canal (module inférieur)

22 polymère résorbable

23 nanostructure en 3D

24 cellule épithéliale

101 module supérieur

102 unité supérieure

103 socle du module supérieur

104 module central

105 unité centrale

106 socle du module central

107 module inférieur

108 unité inférieure

109 socle du module inférieur

201 éléments de fixation

202 canaux entrée/sortie (module supérieur)

203 chambre (unité supérieure)

204 éléments de fixation

205 ensemble d'orifices inférieurs des canaux (module inférieur)

206 ensemble d'orifices supérieurs des canaux (module inférieur)

207 ensemble de protubérances (module central)

208 nanostructure 3D moulée

209 face supérieure du moule

210 matrice polymère résorbable

211 moule négatif d'une nanostructure 3D

212 face inférieure de la matrice

F pièce de support du module central

213 cadre latéral de la pièce de soutien

214 face supérieure ouverte de la pièce de soutien

215 face inférieure pleine de la pièce de soutien

216 découpe de la face pleine de la pièce de soutien

217 goupille d'alignement

218 trou d'alignement

H, H1, H2, h1, h2 moule

I, i pièce de soutien

G, G1, G2, g1, g2 pièce perforée

**Figure 1 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, et dans laquelle la protubérance est en totalité en regard de la perforation.

**Figure 2 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est inférieure à la valeur du diamètre d2 de la section inférieure de ladite perforation, et est égale à la valeur du diamètre d3 de la base circulaire de ladite protubérance, et dans laquelle la protubérance est en totalité en regard de la perforation.

**Figure 3 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et à la valeur du diamètre d3 de la base circulaire de ladite protubérance, et dans laquelle la protubérance est en partie en regard de la perforation.

**Figure 4 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est inférieure à la valeur du diamètre d3 de la base circulaire de ladite protubérance, et dans laquelle la protubérance est en partie en regard de la perforation.

**Figure 5 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est inférieure à la valeur du diamètre d2 de la section inférieure de ladite perforation, et est inférieure à la valeur du diamètre d3 de la base circulaire de ladite protubérance, et dans laquelle la protubérance est en partie en regard de la perforation.

**Figure 6 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est inférieure à la valeur du diamètre d3 de la base circulaire de ladite protubérance, et dans laquelle la protubérance est en partie en regard de la perforation.

**Figure 7 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, et dans laquelle la protubérance est en totalité en regard de la perforation.

**Figure 8 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, et dans laquelle la protubérance est en totalité en regard de la perforation.

**Figure 9 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est inférieure à la valeur du diamètre d2 de la section inférieure de ladite perforation, et est supérieure à la valeur du diamètre d3 de la base circulaire de ladite protubérance, et dans laquelle la protubérance est en totalité en regard de la perforation.

**Figure 10 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est inférieure à la valeur du diamètre d2 de la section inférieure de ladite perforation, et est supérieure à la valeur du diamètre d3 de la base circulaire de ladite protubérance, et dans laquelle la protubérance est en partie en regard de la perforation.

**Figure 11 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est inférieure à la valeur du diamètre d2 de la section inférieure de ladite perforation, et est supérieure à la valeur du diamètre d3 de la base circulaire de ladite protubérance, et dans laquelle la protubérance est en totalité en regard de la perforation.

**Figure 12 :** Vue schématique en coupe transversale d'une unité centrale comportant une protubérance dans la membrane nanostructurée en 3D, et une perforation dans le support, et dans laquelle la protubérance est inclinée selon un axe (z) par rapport à l'axe vertical (y).

**Figure 13 :** Vue schématique en coupe transversale d'une unité centrale comportant une protubérance dans la membrane nanostructurée en 3D, et une perforation dans le support, et dans laquelle la face interne de la protubérance est recouverte d'un ensemble d'un premier type cellulaire au stade de la confluence, et la face externe de la protubérance est recouverte d'un ensemble d'un deuxième type cellulaire au stade de la confluence.

**Figure 14 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en totalité en regard du canal.

**Figure 15 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est supérieure à la valeur du diamètre d5 de l'orifice inférieur, et dans laquelle la protubérance est en totalité en regard du canal.

**Figure 16 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en totalité en regard du canal.

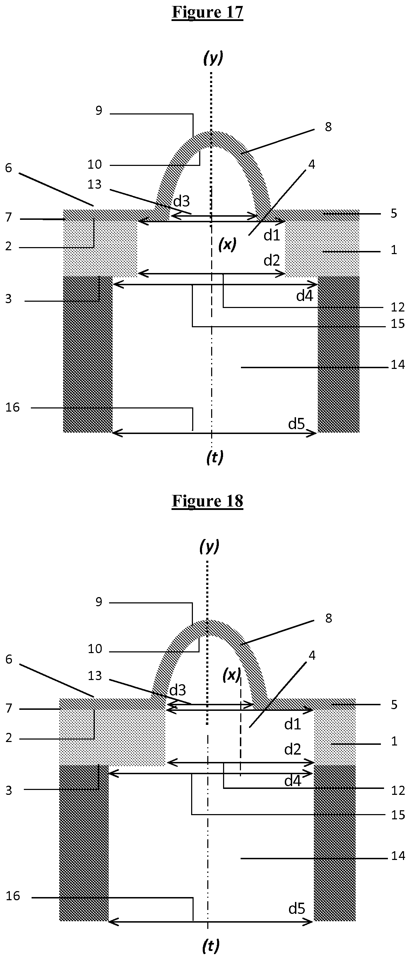

**Figure 17 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en totalité en regard du canal.

**Figure 18 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en totalité en regard du canal.

**Figure 19 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

**Figure 20 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

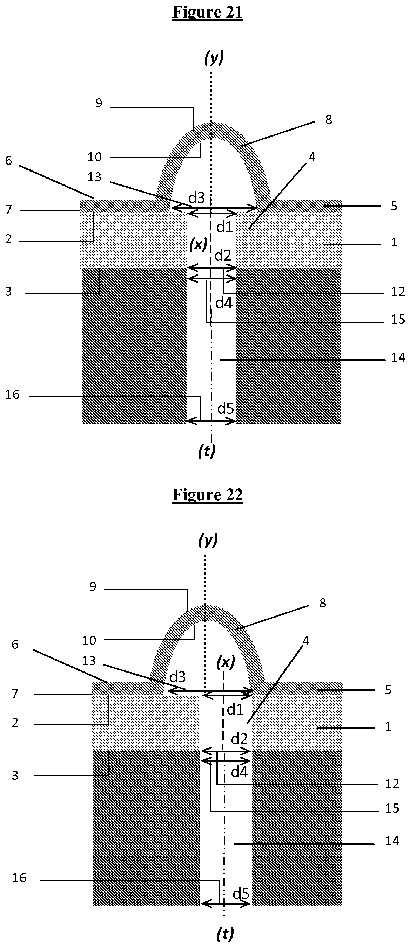

**Figure 21** : Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est inférieure à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

**Figure 22 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est inférieure à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

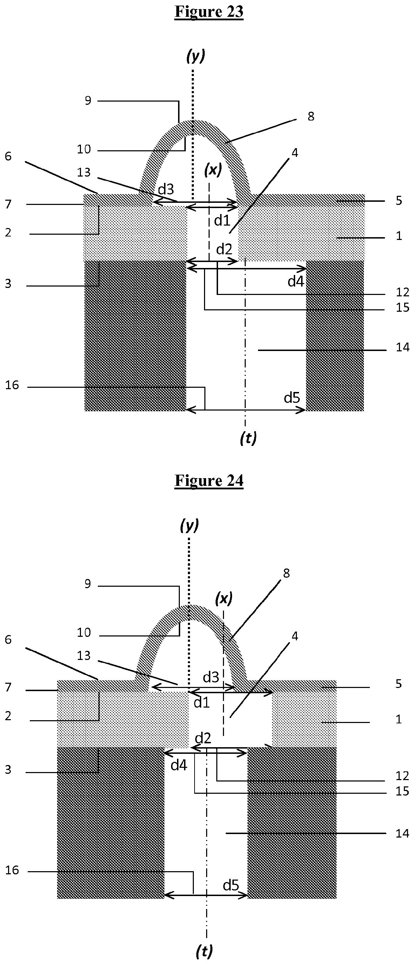

**Figure 23** : Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est inférieure à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

**Figure 24 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

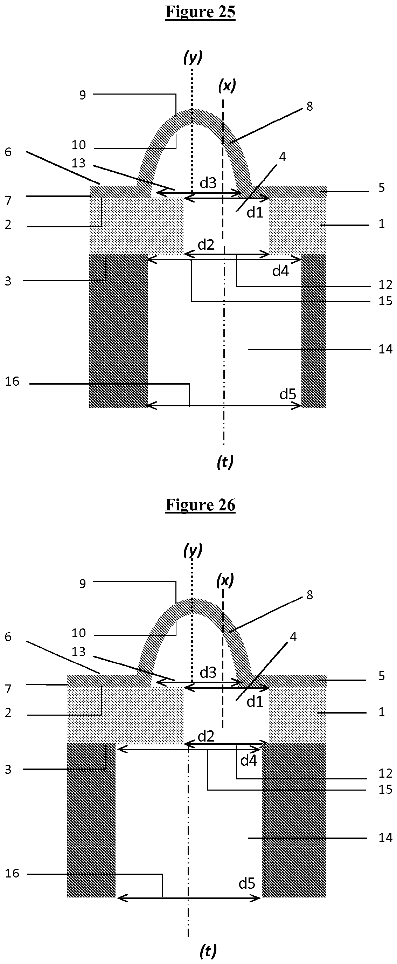

**Figure 25 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

**Figure 26 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est égale à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

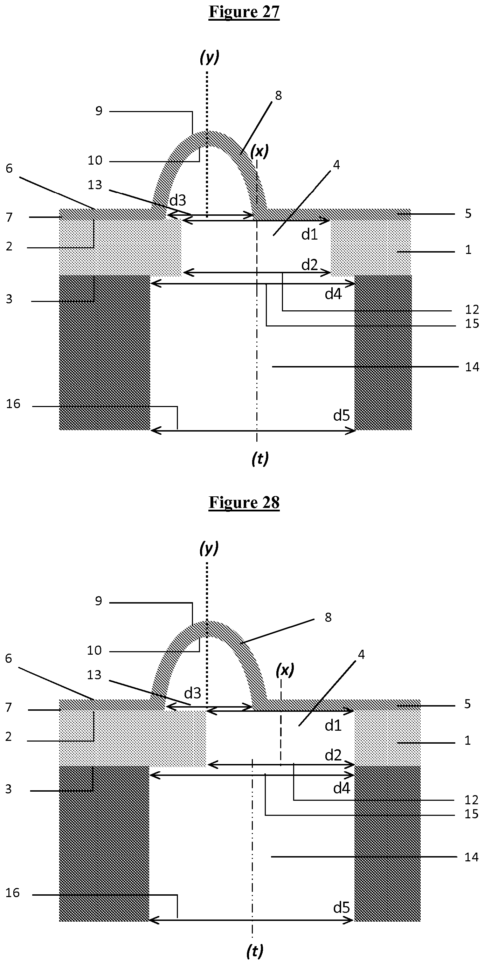

**Figure 27 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur d3 du diamètre de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

**Figure 28 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, ladite unité centrale étant positionnée sur une unité inférieure comportant un canal, dont la valeur du diamètre d4 de l'orifice supérieur est supérieure à la valeur du diamètre d2 de la section inférieure de ladite perforation, et est égale à la valeur du diamètre d5 de l'orifice inférieur, et dans laquelle la protubérance est en partie en regard du canal.

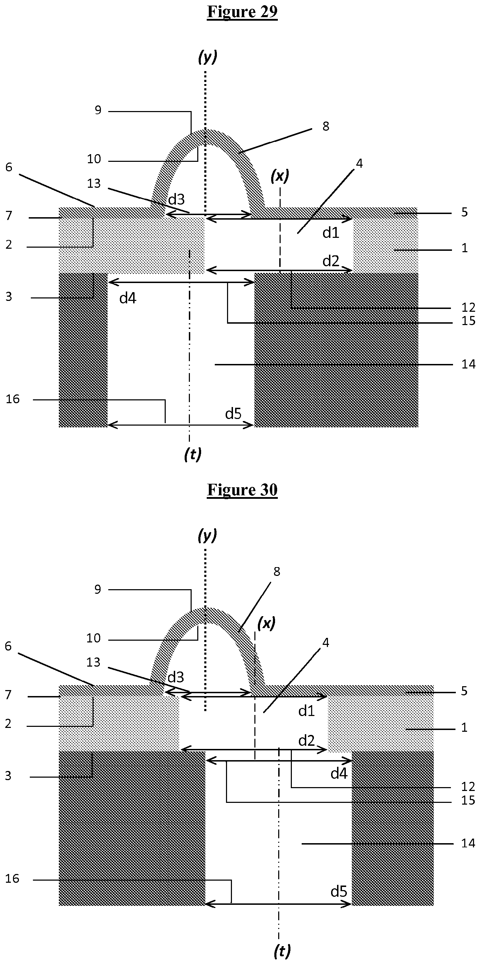

**Figure 29 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

**Figure 30 :** Vue schématique en coupe transversale d'une unité centrale comportant une protubérance dans la membrane nanostructurée en 3D, et une perforation dans le support, et dans laquelle la valeur du diamètre d1 de la section supérieure de ladite perforation est égale à la valeur du diamètre d2 de la section inférieure de ladite perforation, et est supérieure à la valeur du diamètre d3 de la base circulaire de ladite protubérance, ladite unité centrale étant positionnée sur une unité inférieure comportant un canal, dont la valeur du diamètre d4 de l'orifice supérieur est égale à la valeur du diamètre d2 de la section inférieure de ladite perforation, et est égale à la valeur du diamètre d5 de l'orifice inférieur, et dans laquelle la protubérance est en partie en regard du canal.

**Figure 31 :** Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations et est égale à la valeur du diamètre d5 de l'orifice inférieur, et les deux orifices inférieurs des deux canaux débouchant respectivement sur un réservoir, débouchant à l'extérieur du module inférieur via un canal de sortie (protubérance en totalité en regard du canal).

**Figure 32 :** Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations et est égale à la valeur du diamètre d5 de l'orifice inférieur, et les deux orifices inférieurs des deux canaux débouchant à l'extérieur du module inférieur à deux emplacements distincts (protubérance en totalité en regard du canal).

**Figure 33 :** Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations, et est égale à la valeur du diamètre d5 de l'orifice inférieur, et les deux canaux sont connectés entre eux de sorte que les deux orifices inférieurs des deux canaux débouchent à l'extérieur du module inférieur au même emplacement (protubérance en totalité en regard du canal).

**Figure 34 :** Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations, et est égale à la valeur du diamètre d5 de l'orifice inférieur, et les deux orifices inférieurs des deux canaux débouchant respectivement sur un réservoir, chacun des réservoirs débouchant à l'extérieur du module inférieur au même emplacement, via des canaux de sortie connectés entre eux (protubérance en totalité en regard du canal).

**Figure 35 :** Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations, et est supérieure à la valeur du diamètre d5 de l'orifice inférieur, et les deux canaux sont connectés entre eux de sorte que les deux orifices inférieurs des deux canaux débouchent au même emplacement sur un réservoir, lequel débouche à l'extérieur du module inférieur via un canal de sortie (protubérance en totalité en regard du canal).

**Figure 36 :** Vue schématique en coupe transversale d'une unité centrale comportant deux protubérances dans la membrane nanostructurée en 3D, et deux perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant deux canaux, dont la valeur du diamètre d4 de l'orifice supérieur de chacun des canaux est égale à la valeur du diamètre d2 de la section inférieure de chacune des perforations et est supérieure à la valeur du diamètre d5 de l'orifice inférieur, et les deux orifices inférieurs des deux canaux débouchent respectivement à deux emplacements distincts sur un même réservoir, lequel débouche à l'extérieur du module inférieur via un canal de sortie (protubérance en totalité en regard du canal).

**Figure 37 :** Vue schématique en coupe transversale d'une unité centrale comportant quatre protubérances dans la membrane nanostructurée en 3D, et quatre perforations dans le support, et dans laquelle la valeur du diamètre d1 de la section supérieure de chacune des perforations est égale à la valeur du diamètre d2 de la section inférieure de chacune des perforations, et est égale à la valeur du diamètre d3 de la base circulaire de chacune des protubérances, ladite unité centrale étant positionnée sur une unité inférieure comportant quatre canaux, dont la valeur du diamètre d4 de l'orifice supérieur de chacun des canaux est égale à la valeur du diamètre d2 de la section inférieure de chacune des perforations, et les deux orifices inférieurs d'un premier ensemble de deux canaux débouchent respectivement à deux emplacements distincts sur un premier réservoir, et les deux orifices inférieurs d'un deuxième ensemble de deux canaux débouchent respectivement à deux emplacements distincts sur un deuxième réservoir, le premier et le second réservoir débouchant respectivement à l'extérieur du module inférieur à des emplacements distincts (protubérance en totalité en regard du canal).

**Figure 38 :** Vue schématique en perspective du module supérieur.

**Figure 39 :** Vue schématique en perspective du module central.

**Figure 40 :** Vue schématique en perspective prise au dessus de la face supérieure de la membrane poreuse nanostructurée en 3D, d'une unité centrale comportant un ensemble de protubérances.

**Figure 41 :** Vue schématique en perspective du module inférieur.

**Figure 42 :** Vue schématique en perspective de la puce micro fluidique comportant l'assemblage du module supérieur, du module central et du module inférieur.

**Figure 43 :** Photo du module supérieur (vue du côté de l'ouverture de la chambre)

**Figure 44 :** Photo du module central. Vue du dessus de la face supérieure de la membrane poreuse nanostructurée en 3D comportant un ensemble de protubérances.

**Figure 45 :** Photo du module inférieur. Vue du dessus de la face supérieure de l'unité inférieure comprenant l'ensemble des orifices supérieurs des canaux.

**Figure 46 :** Photo du module supérieur et du module inférieur.

**Figure 47 :** Photo du module supérieure, du module central et du module inférieure désassemblés.

**Figure 48 :** Vue schématique en coupe transversale du support constitué d'une membrane non résorbable, de l'unité centrale, comportant une perforation.

**Figure 49 :** Vue schématique en coupe transversale du support constitué d'une membrane non résorbable, de l'unité centrale, comportant une perforation, au travers duquel un polymère résorbable a été extrudé pour former une nanostructure en 3D du côté de la face supérieure dudit support.

**Figure 50 :** Vue schématique en coupe transversale du support constitué d'une membrane non résorbable, de l'unité centrale, comportant une perforation, au travers duquel un polymère résorbable a été extrudé pour former une nanostructure en 3D du côté de la face supérieure dudit support sur laquelle une couche de polyélectrolyte a été appliquée pour obtenir la membrane poreuse nanostructurée en 3D comportant une protubérance moulée sur ladite nanostructure en 3D.

**Figure 51 :** Vue schématique en coupe transversale du support constitué d'une membrane non résorbable et comportant une perforation, de l'unité centrale, sur lequel est positionnée de façon solidaire la membrane nanostructurée en 3D comportant une protubérance creuse.

**Figure 52 :** Photo au microscope confocal de la face interne d'une protubérance soutenant une culture de cellules épithéliales au stade de la confluence.

**Figure 53 :** Vue schématique en coupe transversale d'un moule comprenant au moins une nanostructure 3D moulée du côté de sa face supérieure

**Figure 54 :** Vue schématique en coupe transversale d'un moule recouvert de polymère résorbable du côté la face supérieure dudit moule

**Figure 55 :** Vue schématique en coupe transversale d'une matrice en polymère résorbable comprenant au moins un moule négatif d'au moins une nanostructure 3D moulée

**Figure 56 :** Vue schématique en coupe transversale d'une matrice en polymère résorbable comprenant au moins un moule négatif d'au moins une nanostructure 3D moulée, la face inférieure de ladite matrice étant recouverte d'une couche polyélectrolyte.

**Figure 57 :** Vue schématique en coupe transversale d'une matrice en polymère résorbable comprenant au moins un moule négatif d'au moins une nanostructure 3D moulée assemblée avec une pièce perforée comprenant un support constitué d'une membrane non résorbable perforé par au moins une perforation, ladite matrice étant assemblée du côté de sa face inférieure avec la face supérieure dudit support, de façon à ce que le moule négatif de la nanostructure 3D soit dans l'alignement de ladite perforation du support.

**Figure 58 :** Vue schématique en coupe transversale d'une matrice en polymère résorbable comprenant au moins un moule négatif d'au moins une nanostructure 3D moulée assemblée avec une pièce perforée comprenant un support constitué d'une membrane non résorbable perforé par au moins une perforation, ladite matrice étant assemblée du côté de sa face inférieure avec la face supérieure dudit support, de façon à ce que le moule négatif de la nanostructure 3D soit dans l'alignement de ladite perforation du support,

dans laquelle la surface continue constituée par la face inférieure dudit support et la face inférieure de ladite matrice en polymère résorbable comportant au moins un moule négatif au niveau de ladite au moins une perforation dudit support, est recouvert d'une couche de polyélectrolyte pour former une membrane nanostructurée en 3D comportant au moins une protubérance.

**Figure 59 :** Vue schématique en coupe transversale du module central correspondant à la pièce perforée comprenant du côté de la face inférieure du support, une membrane poreuse nanostructurée en 3D et du côté de la face supérieure du support, au moins une protubérance dans le prolongement de la au moins une perforation du support de la pièce perforée.

**Figure 60 :** Vue schématique en coupe transversale d'un moule comprenant au moins une nanostructure 3D moulée du côté de sa face supérieure, assemblé du côté de sa face supérieure avec une pièce de soutien comportant une découpe dans sa face inférieure.

**Figure 61 :** Vue schématique en coupe transversale d'un moule comprenant au moins une nanostructure 3D moulée du côté de sa face supérieure, assemblé du côté de sa face supérieure avec une pièce de soutien comportant une découpe dans sa face inférieure, où ledit moule est recouvert de polymère résorbable du côté sa face supérieure.

**Figure 62 :** Vue schématique en coupe transversale d'une matrice en polymère résorbable comprenant au moins un moule négatif d'au moins une nanostructure 3D moulée, ladite matrice étant formée au niveau de la découpe de la pièce de soutien

**Figure 63 :** Vue schématique en coupe transversale d'une pièce de soutien contenant au niveau de la découpe de sa face inférieure pleine, un matrice en polymère résorbable comprenant au moins un moule négatif d'au moins une nanostructure 3D moulée, ladite pièce de soutien étant assemblée à une pièce perforée comprenant un support avec au moins une perforation, de façon à ce que ledit moule négatif d'au moins une nanostructure 3D moulée soit dans l'alignement de ladite perforation.

**Figure 64 :** Vue schématique en coupe transversale d'une pièce de soutien contenant au niveau de la découpe de sa face inférieure pleine, un matrice en polymère résorbable comprenant au moins un moule négatif d'au moins une nanostructure 3D moulée, ladite pièce de soutien étant assemblée à une pièce perforée comprenant un support avec au moins une perforation, de façon à ce que ledit moule négatif d'au moins une nanostructure 3D moulée soit dans l'alignement de ladite perforation,

la surface continue constituée par la face inférieure dudit support et la face inférieure de ladite matrice en polymère résorbable comportant au moins un moule négatif au niveau de ladite au moins une perforation dudit support, étant recouvert d'une couche de polyélectrolyte pour former une membrane nanostructurée en 3D comportant au moins une protubérance.

**Figure 65 :** Vue schématique en coupe transversale du support de la pièce perforée comprenant du côté de sa face inférieure, une membrane poreuse nanostructurée en 3D et du côté de sa face supérieure, au moins une protubérance en polyélectrolyte dans le prolongement de la au moins une perforation du support de la pièce perforée, et la pièce de soutien I.

**Figure 66 :** Vue schématique en coupe transversale du module central correspondant à la pièce perforée comprenant du côté de la face inférieure du support, une membrane poreuse nanostructurée en 3D et du côté de la face supérieure du support, au moins une protubérance dans le prolongement de la au moins une perforation du support de la pièce perforée.

**Figure 67 :** Vue schématique en perspective d'une pièce de soutien I de forme circulaire, d'un moule H de forme circulaire et comprenant 100 nanostructures 3D moulées (H1), d'une pièce perforée G de forme circulaire et comprenant 100 perforations (G1) et d'une pièce F, support du module central.

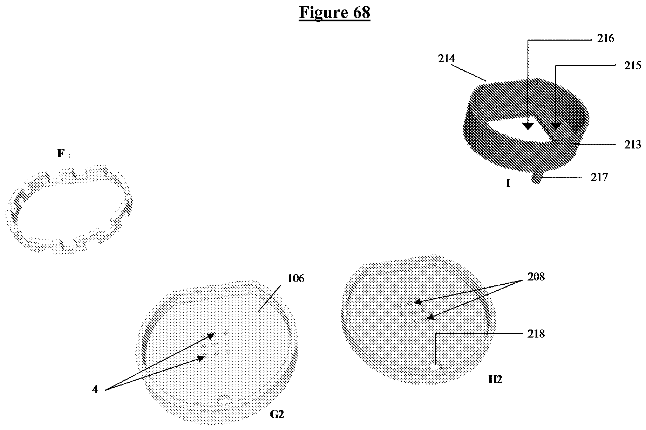

**Figure 68 :** Vue schématique en perspective d'une pièce de soutien I de forme circulaire, d'un moule H de forme circulaire et comprenant 9 nanostructures 3D moulées (H2), d'une pièce perforée G de forme circulaire et comprenant 9 perforations (G2) et d'une pièce F, support du module central.

**Figure 69 :** Vues schématiques en perspective de l'assemblage d'une pièce de soutien I de forme circulaire sur un moule H de forme circulaire et comprenant 100 nanostructures 3D moulées (H1), via la goupille d'alignement de I et le trou d'alignement de H1. **A :** Vue du dessus lorsque les deux éléments sont assemblés. **B :** Vue du dessus lorsque les éléments sont désassemblés. **C :** Vue de profil lorsque les deux éléments sont assemblés. **D :** Vue de profil lorsque les deux éléments sont désassemblés.

**Figure 70 :** Vues schématiques en perspective de l'assemblage d'une pièce de soutien I de forme circulaire sur d'une pièce perforée G de forme circulaire et comprenant 100 perforations (G1) via la goupille d'alignement de I et le trou d'alignement de G1. **A :** Vue du dessus lorsque les deux éléments sont assemblés. **B :** Vue du dessus lorsque les éléments sont désassemblés. **C :** Vue du dessous lorsque les deux éléments sont assemblés. **D :** Vue du dessous lorsque les deux éléments sont désassemblés. **E :** Vue de profil lorsque les deux éléments assemblés sont retournés de manière à ce que G1 soit orienté vers le haut et I soit orienté vers le bas.

**Figure 71 :** Vues schématiques en perspective de l'assemblage d'une pièce perforée G de forme circulaire et comprenant 100 perforations (G1) sur une pièce F, support du module central. **A :** Vue du dessous des deux éléments assemblés. **B :** Vue du dessous lorsque que les deux éléments sont désassemblés. **C :** Vue de profil lorsque les deux éléments assemblés. **D :** Vue de profil lorsque les deux éléments sont assemblés.

**Figure 72 :** Vue schématique en perspective d'une pièce de soutien i de forme carrée, d'un moule H de forme carrée et comprenant 100 nanostructures 3D moulées (h1), d'une pièce perforée G de forme carrée comprenant 100 perforations (g1).

**Figure 73 :** Vue schématique en perspective d'une pièce de soutien i de forme carrée, d'un moule H de forme carrée et comprenant 9 nanostructures 3D moulées (h2), d'une pièce perforée G de forme carrée et comprenant 9 perforations (g2).

**Revendications**

1. Puce microfluidique de culture cellulaire qui contient un module central (104) comprenant :

   - une unité centrale (105), laquelle contient

     - un support constitué d'une membrane non résorbable (1), comportant une face supérieure (2) et une face inférieure (3), perforé par au moins une perforation (4), ledit support (1) étant composé notamment de plastique bioimprimé, de polycarbonate, de plastique de culture tissulaire, de verre ou de SU8,
     - une membrane poreuse nanostructurée en 3D (5), comportant une face supérieure (6) et une face inférieure (7), et comportant au moins une protubérance (8), ladite au moins une protubérance étant creuse et comportant une face externe (9) et une face interne (10) et formant une structure en relief du coté de la face supérieure (6) de la membrane nanostructurée en 3D (5), ladite protubérance étant notamment en forme de dôme creux présentant une base circulaire (13),

ladite face inférieure (7) de ladite membrane poreuse nanostructurée en 3D (5) étant positionnée de façon solidaire sur ladite face supérieure (2) dudit support (1),

ou ladite face supérieure (6) de la membrane nanostructurée en 3D (5) étant positionnée de façon solidaire sur ladite face inférieure (3) dudit support (1),

et ladite membrane poreuse nanostructurée en 3D (5) et la au moins une protubérance (8) étant composées de matériaux appropriés pour la culture de deux types de cellules distincts ;

- un socle (106),

ladite unité centrale (105) étant intégrée dans ledit socle (106), et formant un tout avec ledit socle (106).

2. Puce microfluidique de culture cellulaire selon la revendication 1, ladite face inférieure (7) de ladite membrane poreuse nanostructurée en 3D (5) étant positionnée de façon solidaire sur ladite face supérieure (2) dudit support (1), dans laquelle ladite au moins une perforation (4) du support (1) présente

une section (11) de forme circulaire au niveau de la face supérieure du support (1) (section supérieure de la perforation) présentant un diamètre d1 (diamètre supérieur d1 de la perforation) et un axe (x) passant par le centre de ladite section supérieure (11) de la perforation (4) et perpendiculaire audit support (1),

et une section (12) de forme circulaire au niveau de la face inférieure (3) du support (1) (section inférieure de la perforation) présentant un diamètre d2 (diamètre inférieur d2 de la perforation) et un axe (w) passant par le centre de ladite section inférieure de la perforation et perpendiculaire audit support (1),

dans laquelle lesdits axes (x) et (w) sont confondus,

le diamètre d1 étant d'une valeur supérieure ou égale à 10 μm à une valeur inférieure ou égale à 500 μm, et le diamètre d2 étant d'une valeur supérieure ou égale à 10 μm à une valeur inférieure ou égale à 500 μm,

tel que la valeur du diamètre d2 est supérieure ou égale à la valeur du diamètre d1,

et dans laquelle ladite base circulaire (13) de ladite protubérance (8) présente un diamètre d3 (diamètre d3 de la base circulaire de la protubérance), ledit diamètre d3 de la protubérance (8) étant d'une valeur de 10 μm à une valeur de 500 μm, et étant d'une valeur inférieure, égale ou supérieure à d1, et ladite protubérance a une hauteur de 1 à 600 μm, plus particulièrement de 1 à 300 μm, plus particulièrement de 50 à 300 μm, plus particulièrement de 50 μm.

3. Puce microfluidique de culture cellulaire selon les revendications 1 et 2, dans laquelle ladite face interne (10) de ladite au moins une protubérance (8) est en totalité ou en partie en regard de ladite au moins une perforation (4) dudit support (1), et notamment en totalité en regard de ladite au moins une perforation (4) dudit support (1) :

- lorsque la valeur du diamètre supérieur d1 de ladite au moins une perforation (4) est supérieure ou égale à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus; ou
- lorsque la valeur du diamètre supérieur d1 de ladite au moins une perforation (4) est supérieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à [(valeur de d1 - valeur de d3)/2].

4. Puce microfluidique de culture cellulaire selon les revendications 1, 2 et 3, dans laquelle la face externe (9) de ladite protubérance (8) supporte au moins une cellule adhérente, ladite au moins une cellule adhérente étant en particulier une cellule stromale, et dans laquelle la face interne (10) de ladite protubérance (8) supporte au moins une cellule adhérente, ladite au moins une cellule adhérente étant en particulier une cellule épithéliale, et plus particulièrement, ladite face externe (9) de ladite protubérance (8) supporte un premier ensemble de cellules adhérentes au stade de la confluence, ledit premier ensemble étant un ensemble de cellules stromales, et ladite face interne (10) de ladite protubérance (8) supporte un deuxième ensemble de cellules adhérentes au stade de la confluence, ledit deuxième ensemble étant un ensemble de cellules épithéliales.

5. Puce microfluidique de culture cellulaire selon les revendications 1 à 4, comportant au moins deux protubérances (8) et dans laquelle la distance entre deux protubérances contigües est d'une valeur 1, ladite valeur 1 étant de 10 à 100 $\mu$m, plus particulièrement de 50 à 100 $\mu$m,

et un nombre maximal de protubérances (8) pour une surface de l'unité centrale de 1 cm$^2$ égal à la formule

$$\frac{\pi\,(d3/2)^2}{(l + d3) - \pi\,(d3/2)^2}$$

où 1 est la distance entre deux protubérances (8) contigües, ladite valeur 1 étant de 10 à 100 $\mu$m, plus particulièrement de 50 à 100 $\mu$m, et où d3 est le diamètre de la base circulaire (13) de ladite protubérance (8).

6. Puce microfluidique de culture cellulaire selon l'une des revendications 1 à 5, dans laquelle la membrane poreuse nanostructurée en 3D (5) est constituée d'au moins une couche d'un polyélectolyte choisi parmi le poly(sodium 4-styrènesulphonate) (PSS), le poly(éthylèneimine), le poly(chlorure de diallyldiméthylammonium), le poly(chlorure de acrylamide-co-diallyldimethylammonium), le chlorure de diallyldiméthyllammonium, le poly(hydrochlorure d'allylamine) (PAH), l'acide polyanetholesulfonique, l'acide polyacrylique, l'acide polystyrène-alt-maléique, le sulfate de polyvinyle, l'acide polyvinylsulfonique, l'acide poly(2-acrylamido-2-méthyl-1-propanesulfonique), le poly(acide 2-acrylamido-2-méthyl-1-propanesulfonique -co-acrylonitrile), l'acide poly 4-styrènesulfonique, le poly(acide 4-styrènesulfonique-co-acide maléique), le sel de sodium de 4-styrènesulfonique hydraté,

et dans laquelle lorsque ladite membrane poreuse nanostructurée en 3D (5) est constituée d'au moins deux couches successives, une couche constituée d'un un polyélectrolyte chargé positivement est en alternance d'une couche constituée d'un polyélectrolyte chargé négativement, ladite membrane poreuse nanostructurée en 3D (5) étant en particulier constituée 1 à 150 couches successives de polyélectrolyte, et plus particulièrement ladite membrane poreuse nanostructurée en 3D (5) étant constituée de 15 couches successives de poly(sodium 4-styrènesulphonate) (PSS) et/ou de poly(allylamine hydrochloride) (PAH), en alternance l'une de l'autre.

7. Puce microfluidique de culture cellulaire selon les revendications 1 à 6, qui contient un module inférieur (107) solide, comportant

- une unité inférieure (108)

laquelle contient une face supérieure, une face inférieure et au moins une face latérale, et comprenant au moins un canal (14) de forme tubulaire comportant un orifice supérieur (15) de diamètre d4 présentant un axe (t) passant par le centre dudit orifice supérieur (15) et perpendiculaire audit support (1), et un orifice inférieur de diamètre d5 (16),
- un socle (109),
ladite unité inférieure (108) étant intégrée dans ledit socle (109), et formant un tout avec ledit socle (109), ladite unité inférieure (108) et ledit socle (109) sont composés notamment de plastique bioimprimé, de polycarbonate, de plastique de culture tissulaire ou de SU8,
ladite valeur du diamètre d4 dudit orifice supérieur (15) dudit canal (14) étant supérieure ou égale à la valeur du diamètre d5 dudit orifice inférieur (16) dudit canal (14) et ladite valeur du diamètre d4 étant supérieure ou égale à la valeur du diamètre inférieur d2 de ladite au moins une perforation (4).
ladite face supérieure de ladite unité inférieure (108) comportant ledit orifice supérieur (15) du au moins un canal, et ledit orifice inférieur (16) débouchant, lui-même ou par des moyens intermédiaires (17), à l'extérieur dudit socle (109).

8. Puce microfluidique de culture cellulaire selon les revendications 1 à 7, dans laquelle ledit module inférieur (107) et ledit module central (104) sont assemblés tel que l'orifice supérieur (15) du au moins un canal (14), de ladite face supérieure de ladite unité inférieure (108), s'ouvre sur au moins une desdites perforations (4) dudit support (1) de ladite unité centrale (105), et l'orifice inférieur (16) du au moins un canal (14), débouche lui-même à l'extérieur de la puce, ou via un moyen intermédiaire constitué par un réservoir (17) apte à récupérer des liquides et permettant un acheminement à l'extérieur de la puce via un canal de sortie (18) débouchant à l'extérieur du socle (109) dudit module inférieur (107),

ladite face supérieure de ladite unité inférieure (108) et ladite face inférieure dudit support de ladite unité centrale (105) ayant une forme et une surface identique entre elles, et ladite unité inférieure (108) et ladite unité centrale (105) étant assemblées par des éléments de fixation (204) situés respectivement sur chacun des socles du module inférieur (109) et du module central (106), de façon à assembler de manière étanche ladite face supérieure de ladite unité inférieure (108) et ladite face inférieure dudit support (3) de ladite unité centrale (105).

9. Puce microfluidique de culture cellulaire selon les revendications 1 à 8, dans laquelle ledit orifice supérieur (15) dudit au moins un canal (14) débouche sur une seule perforation (4) dudit support (1), et dans laquelle ladite perforation (4) dudit support (1) est en totalité en regard dudit orifice supérieur (15) dudit au moins un canal (14) :

- lorsque la valeur du diamètre inférieure d2 de ladite perforation (4) est égale à la valeur du diamètre d4 dudit orifice supérieur (15) dudit canal (14), et que l'axe (w), passant par le centre de ladite section inférieure (12) de la perforation (4) et perpendiculaire audit support (1), et l'axe (t), passant par le centre dudit orifice supérieur (15) dudit canal (14) et perpendiculaire audit support (1), sont confondus, ou
- lorsque la valeur du diamètre inférieure d2 de ladite perforation (4) est inférieure à la valeur du diamètre d4 dudit orifice supérieur (15) dudit canal (14), et que l'axe (w), passant par le centre de ladite section inférieure (12) de la perforation (4) et perpendiculaire audit support (1), et l'axe (t), passant par le centre dudit orifice supérieur (15) dudit canal (14) et perpendiculaire audit support (1), sont confondus, ou distincts l'un par rapport à l'autre d'une distance d'une valeur inférieure ou égale à [ (valeur de d4 - valeur de d2) /2].

10. Puce microfluidique de culture cellulaire selon les revendications 1 à 9, dans laquelle ledit module inférieur (107) comporte au moins deux canaux (14), et dans laquelle chacun des orifices supérieurs (15) des au moins deux canaux (14) débouche sur une perforation (4)

- et les au moins deux orifices inférieurs (16) des au moins deux canaux (14) débouchent à l'extérieur de la puce respectivement à au moins deux emplacements distincts,
- ou les au moins deux orifices inférieurs (16) de l'ensemble des au moins deux canaux (14) débouchent sur un même moyen intermédiaire constitué par un réservoir (17) apte à récupérer des liquides et permettant un acheminement à l'extérieur de la puce via un canal de sortie (18) débouchant à l'extérieur du socle (109) dudit module inférieur (107).

11. Puce microfluidique de culture cellulaire selon les revendications 1 à 6, qui contient un module supérieur (101) comprenant :

- une unité supérieure (102) solide et creuse, laquelle est constituée d'une surface pleine définissant un volume ouvert (chambre) (203), et les bords de la surface pleine étant aptes à être en contact avec ladite unité centrale (105) dudit module central (104) et délimitant la surface de l'ouverture dudit volume ouvert ;
- et un socle (103),
ladite unité supérieure (102) étant intégrée dans ledit socle (103), et formant un tout avec ledit socle (103).
et dans laquelle ladite unité supérieure (102) et ledit socle (103) sont notamment composés de plastique bioimprimé, de polycarbonate, de plastique de culture tissulaire ou de SU8.

12. Puce microfluidique de culture cellulaire selon les revendications 1 à 6 et selon la revendication 11 dans laquelle ladite unité supérieure (102) dudit module supérieur (101) et ladite unité centrale (105) dudit module central (104) sont assemblées de sorte que ladite surface de l'ouverture de ladite unité supérieure (102) est positionnée au dessus de ladite face supérieure (6) de la membrane poreuse nanostructurée en 3D (5) de ladite unité centrale (105),

pour former un espace clos délimité par ladite surface pleine de ladite unité supérieure (102) et ladite face supérieure (6) de la membrane poreuse nanostructurée en 3D (5), ladite surface de l'ouverture et ladite face supérieure (6) ayant une forme et une surface identiques entre elles, ladite unité supérieure (102) dudit module supérieur (101) et ladite unité centrale (105) dudit module central (104) étant assemblées par des éléments de fixation (201,204) situés sur chacun des socles (103,106) du module supérieur (101) et du module central (104), de façon à assembler de manière étanche ladite surface de l'ouverture de ladite unité supérieure (102) et ladite face supérieure (6) de la membrane poreuse nanostructurée en 3D (5) de ladite unité centrale (105),
et avantageusement ladite au moins une face pleine de ladite unité supérieure (102) présente deux orifices débouchant respectivement sur un canal entrée/sortie (202),
permettant audit espace clos de communiquer avec l'extérieur de ladite puce microfluidique via lesdits canaux entrée/sortie (202) qui débouchent à l'extérieur du socle (109).

13. Puce microfluidique de culture cellulaire selon les revendications 1 à 6 et selon les revendications 11 et 12, comportant un module supérieur (101), un module central (104) et un module inférieur (107), et dans laquelle les susdits modules sont assemblés tel que

ladite surface de l'ouverture de ladite unité supérieure (102) dudit module supérieur (101) est positionnée au dessus de ladite face supérieure (2) de la membrane poreuse nanostructurée en 3D (5) de ladite unité centrale (105) dudit module central (104),

et l'orifice supérieur (15) du au moins un canal (14), de ladite face supérieure de ladite unité inférieure (108) dudit module inférieur (107), s'ouvre sur au moins une desdites perforations (4) dudit support (1) de ladite unité centrale (105),

ladite surface de l'ouverture de ladite unité supérieure (102) et ladite face supérieure (2) de la membrane poreuse nanostructurée en 3D (5) de ladite unité centrale (105) ayant une forme et une surface identiques entre elles,

ladite face supérieure de ladite unité inférieure (108) et ladite face inférieure dudit support (3) de ladite unité centrale (105) ayant une forme et une surface identiques entre elles,

et ledit module supérieur (101), ledit module central (104) et ledit module inférieur (107) étant assemblés par des éléments de fixation (201, 204) situés sur chacun des socles respectifs (103, 106, 109)

notamment dans laquelle ladite face interne (10) de ladite protubérance (8) est en totalité en regard dudit orifice supérieur (15) dudit canal (14) :

lorsque ladite face interne (10) de ladite au moins une protubérance (8) est en totalité en regard de ladite au moins une perforation (4) dudit support (1) :

- tel que lorsque la valeur du diamètre supérieur d1 de ladite au moins une perforation (4) est supérieure ou égale à la valeur du diamètre d3 de ladite au moins une protubérance (4), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont confondus; ou
- tel que la valeur du diamètre supérieur d1 de ladite au moins une perforation (4) est supérieure à la valeur du diamètre d3 de ladite au moins une protubérance (8), et que l'axe (y), passant par le centre de ladite base circulaire (13) et perpendiculaire audit support (1), et l'axe (x), passant par le centre de la section supérieure (11) de ladite perforation (4) et perpendiculaire audit support (1), sont distincts l'un par rapport à l'autre d'une distance d'une valeur inférieure ou égale à [(valeur de d1 - valeur de d3)/2] ;

et lorsque ladite perforation (4) dudit support (1) est en totalité en regard dudit orifice supérieur (15) dudit canal (14) :

tel que l'axe (y), passant par le centre de ladite base circulaire (13) de ladite protubérance (8) et perpendiculaire audit support (1), et l'axe (t), passant par le centre dudit orifice supérieur (15) dudit canal (14) et perpendiculaire audit support (1), sont confondus, ou distincts l'un par rapport à l'autre d'une distance inférieure ou égale à [(valeur de d4 - valeur de d3)/2] lorsque la valeur de d4 est supérieure à la valeur de d2.

14. Procédé de fabrication d'une puce microfluidique de culture cellulaire selon les revendications 1 à 6, dans lequel la fabrication de ladite unité centrale (105) dudit module central (104) comprend :

- une étape d'extrusion d'une solution de polymère résorbable (22), à travers la au moins une perforation (4) dudit support (1) pour former au moins une nanostructure 3D (23) du côté de ladite face supérieure (2) dudit support (1), ladite au moins une nanostructure étant notamment en forme de dôme, suivi de
- une étape de polymérisation de ladite solution de polymère résorbable pour rigidifier ladite au moins une nanostructure 3D (23), ladite au moins une nanostructure 3D (23) formant un moule en polymère résorbable (22) du côté de ladite face supérieure dudit support après polymérisation, suivi par
- une étape d'application d'au moins une couche continue d'au moins un polyélectrolyte recouvrant la face supérieure (2) dudit support (1) et la surface dudit moule en polymère résorbable (22), pour constituer la membrane poreuse nanostructurée en 3D (5), par
- une étape de dissolution dudit moule en polymère résorbable pour obtenir ladite face inférieure (7) de ladite membrane poreuse nanostructurée en 3D (5) positionnée de façon solidaire sur ladite face supérieure (2) dudit support (1) et ladite face interne (10) de la au moins une protubérance (8) positionnée en totalité en regard de ladite au moins une perforation (4),

ledit polymère résorbable (22) étant en particulier choisi parmi le chitosan, l'agarose et l'alginate.

15. Procédé de fabrication d'une puce microfluidique de culture cellulaire selon la revendication 1, ladite face supérieure (6) de la membrane nanostructurée en 3D (5) de la puce microfluidique étant positionnée de façon solidaire sur ladite face inférieure (3) dudit support (1), dans lequel la fabrication du module central comprenant l'unité centrale, comprend :

(i) une étape d'assemblage d'une pièce de soutien (I, i), constituée d'un cadre latéral (213), d'une face supérieure ouverte (214) et d'une face inférieure pleine (215) comportant une découpe (216) au format de l'unité centrale, et d'un moule (H, H1, H2, h1, h2), aux formes et dimensions de ladite pièce de soutien (I, i), comprenant au moins une nanostructure 3D moulée (208) sur la face supérieure (209), ledit moule étant en particulier en plastique,

(ii) une étape de coulage d'une solution de polymère résorbable (22) sur ladite face supérieure (209) dudit moule (H, H1, H2, h1, h2), (figure 61) suivi de

(iii) une étape de polymérisation de ladite solution de polymère résorbable (22) pour rigidifier ladite solution de polymère résorbable et former une matrice en polymère résorbable (210) comportant au moins un moule négatif de ladite au moins une nanostructure 3D (211), suivi de

(iv) une étape de retrait dudit moule (H, H1, H2, h1, h2), pour obtenir ladite matrice en polymère résorbable (210) comportant au moins un moule négatif de ladite au moins une nanostructure 3D (211) formée dans ladite découpe (216) de la face inférieure pleine (215) de ladite pièce de soutien (I, i), suivi de

(v) une étape d'assemblage de ladite pièce de soutien (I, i), avec une pièce perforée (G, G1, G2, g1, g2), comprenant un support constitué d'une membrane non résorbable (1) perforé par au moins une perforation (4) intégré dans un socle (106),

ladite pièce perforée (G, G1, G2, g1, g2) étant aux formes et dimensions de ladite pièce de soutien (I, i), et le nombre de perforations (4) de ladite pièce perforée (G, G1, G2, g1, g2) étant identique au nombre de nanostructures 3D moulées (211) dans ledit moule (H, H1, H2, h1, h2) utilisé à l'étape (i),

de manière à ce que ladite au moins une perforation (4) dudit support (1) soit dans l'alignement avec ledit au moins un moule négatif de ladite au moins une nanostructure 3D (211), suivi de

(vi) une étape d'application d'au moins une couche continue d'au moins un polyélectrolyte sur la surface continue constituée de la face inférieure (3) dudit support (1) et de la face inférieure (212) de ladite matrice en polymère résorbable (210) comportant au moins un moule négatif de la dite au moins une nanostructure 3D (211) au niveau de ladite au moins une perforation (4) dudit support (1),

pour constituer une membrane poreuse nanostructurée en 3D (5) comportant au moins une protubérance (8), suivi de

(vii) une étape de dissolution de ladite matrice en polymère résorbable (210) comportant au moins un moule négatif de ladite au moins une nanostructure 3D (211),

(viii) une étape de retrait de la pièce de soutien (I, i) pour obtenir ladite pièce perforée (G, G1, G2, g1, g2) comprenant sur sa face inférieure (3) une membrane poreuse nanostructurée en 3D (5), et du côté de sa face supérieure ladite au moins une protubérance.

## Patentansprüche

1. Mikrofluidischer Zellkulturchip, der ein mittleres Modul (104) enthält, Folgendes umfassend:

   - eine mittlere Einheit (105), die enthält

     - einen Träger, der aus einer nicht resorbierbaren Membran (1) besteht, eine obere Seite (2) und eine untere Seite (3) aufweisend, durch mindestens eine Perforation (4) perforiert, wobei der Träger (1) insbesondere aus biobedrucktem Kunststoff, Polycarbonat, GewebekulturKunststoff, Glas oder SU8 besteht,
     - eine poröse, 3D-nanostrukturierte Membran (5), die eine obere Seite (6) und eine untere Seite (7) aufweist und mindestens einen Vorsprung (8) umfasst, wobei der mindestens eine Vorsprung hohl ist und eine äußere Seite (9) und eine innere Seite (10) umfasst und eine Reliefstruktur auf der Seite der oberen Seite (6) der 3D -nanostrukturierten Membran (5) bildet, wobei der Vorsprung insbesondere in Form einer hohlen Kuppel vorliegt, die eine kreisförmigen Basis (13) aufweist,
     wobei die untere Seite (7) der porösen, 3D-nanostrukturierten Membran (5) auf feste Weise auf der oberen Seite (2) des Trägers (1) positioniert ist,
     oder die obere Seite (6) der 3D-nanostrukturierten Membran (5) auf feste Weise auf der unteren Seite (3) des Trägers (1) positioniert ist,
     und wobei die poröse 3D-nanostrukturierte Membran (5) und der mindestens eine Vorsprung (8) aus Materialien bestehen, die für die Kultivierung von zwei unterschiedlichen Zelltypen geeignet sind;

   - einen Sockel (106),
   wobei die mittlere Einheit (105) in den Sockel (106) integriert ist und ein Ganzes mit dem Sockel (106) bildet.

2. Mikrofluidischer Zellkulturchip nach Anspruch 1, wobei die untere Seite (7) der porösen, 3D-nanostrukturierten Membran (5) auf feste Weise auf der oberen Seite (2) des Trägers (1) positioniert ist, wobei die mindestens eine Perforation (4) des Trägers (1) aufweist

einen kreisförmigen Abschnitt (11) auf der Ebene der oberen Seite des Trägers (1) (oberer Abschnitt der Perforation), der einen Durchmesser d1 (oberer Durchmesser d1 der Perforation) und eine Achse (x), die durch die Mitte des oberen Abschnitts (11) der Perforation (4) und senkrecht zu dem Träger verläuft (1),
und einen kreisförmigen Abschnitt (12) auf der Ebene der unteren Seite (3) des Trägers (1) (unterer Abschnitt der Perforation), der einen Durchmesser d2 (unterer Durchmesser d2 der Perforation) und einer Achse (w), die durch die Mitte des unteren Durchmessers der Perforation und senkrecht zu dem Träger (1) verläuft, aufweist,
wobei die Achsen (x) und (w) zusammenfallen, wobei der Durchmesser d1 einen Wert von größer oder gleich 10 $\mu$m bis zu einem Wert von kleiner oder gleich 500 $\mu$m aufweist, und der Durchmesser d2 einen Wert von größer oder gleich 10 $\mu$m bis zu einem Wert von kleiner oder gleich 500 $\mu$m aufweist,
so dass der Wert des Durchmessers d2 größer oder gleich dem Wert des Durchmessers d1 ist, und wobei die kreisförmige Basis (13) des Vorsprungs (8) einen Durchmesser d3 (Durchmesser d3 der kreisförmigen Basis des Vorsprungs) aufweist, wobei der Durchmesser d3 des Vorsprungs (8) einen Wert von 10 $\mu$m bis zu einem Wert von 500 $\mu$m aufweist und einen Wert kleiner oder gleich d1 aufweist, und der Vorsprung eine Höhe von 1 bis 600 pm, insbesondere von 1 bis 300 pm, insbesondere von 50 bis 300 pm, insbesondere von 50 $\mu$m aufweist.

3. Mikrofluidischer Zellkulturchip nach den Ansprüchen 1 und 2, bei dem die innere Seite (10) des mindestens einen Vorsprungs (8) ganz oder teilweise der mindestens einen Perforation (4) des Trägers (1) gegenüberliegt,
und vor allem ganz der mindestens einen Perforation (4) des Trägers (1) gegenüberliegt:

- wenn der Wert des oberen Durchmessers d1 der mindestens einen Perforation (4) größer oder gleich dem Wert des Durchmessers d3 des mindestens einen Vorsprungs (4) ist, und die Achse (y), die durch die Mitte der kreisförmigen Basis (13) und senkrecht zu dem Träger (1) verläuft, und die Achse (x), die durch die Mitte des oberen Abschnitts (11) der Perforation (4) und senkrecht zu dem Träger (1) verläuft, zusammenfallen; oder
- wenn der Wert des oberen Durchmessers d1 der mindestens einen Perforation (4) größer ist als der Wert des Durchmessers d3 des mindestens einen Vorsprungs (8), und die Achse (y), die durch die Mitte der kreisförmigen Basis (13) und senkrecht zu der Halterung (1) verläuft, und die Achse (x), die durch die Mitte des oberen Abschnitts (11) der Perforation (4) und senkrecht zu dem Träger (1) verläuft, um einen Abstand mit einem Wert kleiner oder gleich [(Wert von d1 - Wert von d3)/2] voneinander getrennt sind.

4. Mikrofluidischer Zellkulturchip nach den Ansprüchen 1, 2 und 3, wobei die äußere Seite (9) des Vorsprungs (8) mindestens eine adhärente Zelle trägt, wobei die mindestens eine adhärente Zelle insbesondere eine Stromazelle ist, und wobei die innere Seite (10) des Vorsprungs (8) mindestens eine adhärente Zelle trägt, wobei die mindestens eine adhärente Zelle insbesondere eine Epithelzelle ist, und insbesondere die äußere Seite (9) des Vorsprungs (8) einen ersten Satz von adhärenten Zellen im Konfluenzstadium trägt, wobei der erste Satz ein Satz von Stromazellen ist, und die Innenseite (10) des Vorsprungs (8) einen zweiten Satz von adhärenten Zellen im Konfluenzstadium trägt, wobei der zweite Satz ein Satz von Epithelzellen ist.

5. Mikrofluidischer Zellkulturchip nach den Ansprüchen 1 bis 4, der mindestens zwei Vorsprünge (8) umfasst und wobei der Abstand zwischen zwei benachbarten Vorsprüngen einen Wert 1 aufweist, wobei der Wert 1 10 bis 100 $\mu$m, insbesondere 50 bis 100 $\mu$m beträgt,

und eine maximale Anzahl von Vorsprüngen (8) für eine Fläche der mittleren Einheit von 1 cm² gleich der Formel

$$\frac{\pi\,(d3/2)^2}{(l + d3) - \pi\,(d3/2)^2}$$

wobei 1 der Abstand zwischen zwei benachbarten Vorsprüngen (8) ist, wobei der Wert 1 von 10 bis 100 $\mu$m, insbesondere von 50 bis 100 $\mu$m, beträgt,
und wobei d3 der Durchmesser der kreisförmigen Basis (13) des Vorsprungs (8) ist.

6. Mikrofluidischer Zellkulturchip nach einem der Ansprüche 1 bis 5, wobei die 3D-nanostrukturierte poröse Membran (5) aus mindestens einer Schicht eines Polyelektrolyten besteht, der ausgewählt ist aus Poly(natrium-4-styrolsulfonat) (PSS), Poly(ethylenimin), Poly(diallyldimethylammoniumchlorid), Poly(acrylamid-co-diallyldimethylammoniumchlorid),

Diallyldimethylammoniumchlorid, Poly(allylaminhydrochlorid) (PAH), Polyanetholsulfonsäure, Polyacrylsäure, Polystyrol-Maleinsäure, Polyvinylsulfat, Polyvinylsulfonsäure, Poly(2-acrylamido-2-methyl-1-propansulfonsäure), Poly(2-acrylamido-2-methyl-1-propansulfonsäure-co-acrylnitril), Poly-4-styrolsulfonsäure, Poly(4-styrolsulfonsäure-co-maleinsäure), 4-Styrolsulfonsäurenatriumsalzhydrat,

und wobei, wenn die 3D-nanostrukturierte poröse Membran (5) aus mindestens zwei aufeinanderfolgenden Schichten besteht, eine Schicht, die aus einem positiv geladenen Polyelektrolyten besteht, abwechselnd mit einer Schicht, die aus einem negativ geladenen Polyelektrolyten besteht, vorliegt,

wobei die poröse 3D-nanostrukturierte Membran (5) insbesondere aus 1 bis 150 aufeinanderfolgenden Polyelektrolytschichten besteht,

und wobei die poröse 3D-nanostrukturierte Membran (5) insbesondere aus 15 aufeinanderfolgenden Schichten von Poly(natrium-4-styrolsulfonat) (PSS) und/oder Poly(allylaminhydrochlorid) (PAH) besteht, die sich gegenseitig abwechseln.

7. Mikrofluidischer Zellkulturchip nach den Ansprüchen 1 bis 6, der eine feste untere Einheit (107) enthält, umfassend

- eine untere Einheit (108),
die eine obere Seite, eine untere Seite und mindestens eine seitliche Seite enthält und mindestens einen röhrenförmigen Kanal (14) mit einer oberen Öffnung (15) mit einem Durchmesser d4, eine Achse (t) aufweisend, die durch die Mitte der oberen Öffnung (15) und senkrecht zu dem Träger (1) verläuft, und einer unteren Öffnung mit einem Durchmesser d5 (16) umfasst,
- einen Sockel (109),
wobei die untere Einheit (108) in den Sockel (109) integriert ist und mit dem Sockel (109) ein Ganzes bildet, wobei die untere Einheit (108) und der Sockel (109) insbesondere aus biobedrucktem Kunststoff, Polycarbonat, Kunststoff aus Gewebekulturen oder SU8 bestehen,
wobei der Wert des Durchmessers d4 der oberen Öffnung (15) des Kanals (14) größer oder gleich dem Wert des Durchmessers d5 der unteren Öffnung (16) des Kanals (14) ist und der Wert des Durchmessers d4 größer oder gleich dem Wert des unteren Durchmessers d2 der mindestens einen Perforation (4) ist,
wobei die obere Seite der unteren Einheit (108) die obere Öffnung des mindestens einen Kanals umfasst und die untere Öffnung (16) selbst oder über Zwischenmittel (17) zum Äußeren des Sockels (109) mündet.

8. Mikrofluidischer Zellkulturchip nach den Ansprüchen 1 bis 7, wobei das untere Modul (107) und das mittlere Modul (104) so zusammengesetzt sind, dass sich die obere Öffnung (15) des mindestens einen Kanals (14) auf der oberen Seite der unteren Einheit (108) in mindestens eine der Perforationen (4) des Trägers (1) der mittleren Einheit (105) öffnet, und die untere Öffnung (16) des mindestens einen Kanals (14) zur Außenseite des Chips mündet, selbst oder über ein Zwischenmittel, das aus einem Behälter (17) besteht, der geeignet ist, Flüssigkeiten aufzufangen und eine Weiterleitung zur Außenseite des Chips über einen Ausgangskanal (18) ermöglicht, der auf die Außenseite des Sockels (109) der unteren Einheit (107) mündet,

wobei die obere Seite der unteren Einheit (108) und die untere Seite des Sockels der mittleren Einheit (105) eine zueinander identische Form und Oberfläche aufweisen,
und wobei die untere Einheit (108) und die mittlere Einheit (105) durch Befestigungselemente (204), die sich jeweils an jedem der Sockel des unteren Moduls (109) und des mittleren Moduls (106) befinden, zusammengefügt sind, um die obere Seite der unteren Einheit (108) und die untere Seite des Trägers (3) der mittleren Einheit (105) auf dichte Weise zusammenzufügen.

9. Mikrofluidischer Zellkulturchip nach den Ansprüchen 1 bis 8, wobei die obere Öffnung (15) des mindestens einen Kanals (14) in eine einzige Perforation (4) des Trägers (1) mündet, und wobei die Perforation (4) des Trägers (1) vollständig der oberen Öffnung (15) des mindestens einen Kanals (14) gegenüberliegt:

- wenn der Wert des unteren Durchmessers d2 der Perforation (4) gleich dem Wert des Durchmessers d4 der oberen Öffnung (15) des Kanals (14) ist, und die Achse (w), die durch die Mitte des unteren Abschnitts (12) der Perforation (4) und senkrecht zu dem Träger (1) verläuft, und die Achse (t), die durch die Mitte der oberen Öffnung (15) des Kanals (14) und senkrecht zu dem Träger (1) verläuft, zusammenfallen, oder
- wenn der Wert des unteren Durchmessers d2 der Perforation (4) kleiner ist als der Wert des Durchmessers d4 der oberen Öffnung (15) des Kanals (14), und die Achse (w), die durch die Mitte des unteren Abschnitts (12) der Perforation (4) und senkrecht zu dem Träger (1) verläuft, und die Achse (t), die durch die Mitte der oberen Öffnung (15) des Kanals (14) und senkrecht zu dem Träger (1) verläuft, zusammenfallen oder um einen Abstand mit einem Wert kleiner oder gleich [(Wert von d4 - Wert von d2)/2] voneinander getrennt sind.

**10.** Mikrofluidischer Zellkulturchip nach den Ansprüchen 1 bis 9, wobei das untere Modul (107) mindestens zwei Kanäle (14) aufweist,
und wobei jede der oberen Öffnungen (15) der mindestens zwei Kanäle (14) in eine Perforation (4) mündet

     - und die mindestens zwei unteren Öffnungen (16) der mindestens zwei Kanäle (14) jeweils an mindestens zwei verschiedenen Stellen an der Außenseite des Chips münden,
     - oder die mindestens zwei unteren Öffnungen (16) des Satzes der mindestens zwei Kanäle (14) in ein und dasselbe Zwischenmittel münden, das aus einem Reservoir (17) besteht, das geeignet ist, Flüssigkeiten aufzufangen und eine Weiterleitung zum Äußeren des Chips über einen Ausgangskanal (18) ermöglicht, der zum Äußeren des Sockels (109) des unteren Moduls (107) mündet.

**11.** Mikrofluidischer Zellkulturchip nach Anspruch 1 bis 6, der ein oberes Modul (101) enthält, Folgendes umfassend:

     - eine feste und hohle obere Einheit (102), die aus einer massiven Oberfläche besteht, die ein offenes Volumen (Kammer) (203) definiert, und wobei die Ränder der massiven Oberfläche geeignet sind, mit der mittleren Einheit (105) des mittleren Moduls (104) in Kontakt zu sein und die Oberfläche der Öffnung des offenen Volumens zu begrenzen;
     - einen Sockel (103),

wobei die obere Einheit (102) in den Sockel (103) integriert ist und ein Ganzes mit dem Sockel (103) bildet, und wobei die obere Einheit (102) und der Sockel (103) vor allem aus biobedrucktem Kunststoff, Polycarbonat, Gewebekulturkunststoff oder SU8 bestehen.

**12.** Mikrofluidischer Zellkulturchip nach Anspruch 1 bis 6 und nach Anspruch 11, wobei die obere Einheit (102) des oberen Moduls (101) und die mittlere Einheit (105) des mittleren Moduls (104) so zusammengefügt sind, dass die Oberfläche der Öffnung der oberen Einheit (102) oberhalb der oberen Seite (6) der 3D-nanostrukturierten porösen Membran (5) der mittleren Einheit (105) positioniert ist, um einen abgeschlossenen Raum zu bilden, der von der massiven Oberfläche der oberen Einheit (102) und der oberen Seite (6) der porösen 3D-nanostrukturierten Membran (5) begrenzt wird, wobei die Oberfläche der Öffnung und die obere seite (6) eine zueinander identische Form und Oberfläche aufweisen,

wobei die obere Einheit (102) des oberen Moduls (101) und die mittlere Einheit (105) des mittlere Moduls (104) durch Befestigungselemente (201, 204) zusammengefügt werden, die sich auf jedem der Sockel (103, 106) des oberen Moduls (101) und des mittleren Moduls (104) befinden, so dass die Oberfläche der Öffnung der oberen Einheit (102) und die obere Seite (6) der porösen, 3D-nanostrukturierten Membran (5) der mittleren Einheit (105) auf dichte Weise zusammengefügt werden,
und vorteilhafterweise weist die mindestens eine massive Fläche der oberen Einheit (102) zwei Öffnungen auf, die jeweils in einen Eingangs-/Ausgangskanal (202) münden, was es dem abgeschlossenen Raum ermöglicht, über die Eingangs-/Ausgangskanäle (202), die zum Äußeren des Sockels (109) münden, mit dem Äußerens des mikrofluidischen Chips in Verbindung zu stehen.

**13.** Mikrofluidischer Zellkultur-Chip nach den Ansprüchen 1 bis 6 und nach den Ansprüchen 11 und 12, der ein oberes Modul (101), ein mittleres Modul (104) und ein unteres Modul (107) umfasst, und wobei die Module so zusammengefügt sind, dass

die Oberfläche der Öffnung der oberen Einheit (102) des oberen Moduls (101) oberhalb der oberen Seite (2) der porösen, 3D-nanostrukturierten Membran (5) der mittleren Einheit (105) des mittleren Moduls (104) positioniert ist, und sich die obere Öffnung (15) des mindestens einen Kanals (14), der unteren Seite der unteren Einheit (108) des unteren Moduls (107) zu mindestens einer der Perforationen (4) des Trägers (1) der mittleren Einheit (105) öffnet,
wobei die Oberfläche der Öffnung der oberen Einheit (102) und die obere Seite (2) der porösen, 3D-nanostrukturierten Membran (5) der mittleren Einheit (105) eine untereinander identische Form und Oberfläche aufweisen,
wobei die obere Seite der unteren Einheit (108) und die untere Seite des Trägers (3) der mittleren Einheit (105) eine untereinander identische Form und Oberfläche aufweisen,
und wobei das obere Modul (101), das mittlere Modul (104) und das untere Modul (107) durch Befestigungselemente (201, 204) zusammengefügt sind, die sich auf jedem der jeweiligen Sockel (103, 106, 109) befinden
wobei insbesondere in innere Seite (10) des Vorsprungs (8) vollständig der oberen Öffnung (15) des Kanals (14) gegenüberliegt:
wenn die innere Seite (10) des mindestens einen Vorsprungs (8) der mindestens einen Perforation (4) des Trägers (1) vollständig gegenüberliegt:

- so dass, wenn der Wert des Durchmessers d1 der mindestens einen Perforation (4) größer oder gleich dem Wert (4) des Durchmessers d3 des wenigstens einen Vorsprungs ist, und die Achse (y), die durch die Mitte der kreisförmigen Basis (13) und senkrecht zu dem Träger (1) verläuft, zusammenfallen; oder

- so dass der Wert des oberen Durchmessers d1 der wenigstens einen Perforation (4) höher ist als der Wert des Durchmessers d3 des wenigstens einen Vorsprungs (8), und dass die Achse (y), die durch die Mitte der kreisförmigen Basis (13) und senkrecht zu dem Träger (1) verläuft, und die Achse (x), die durch die Mitte des oberen Abschnitts (11) der Perforation (4) und senkrecht zu dem Träger (1) verläuft, sich voneinander durch einen Abschnitt eines Wertes kleiner als oder gleich [(Wert von d1 - Wert von d3)/2] unterscheiden;

und wenn die Perforation (4) des Trägers (1) vollständig der oberen Öffnung (15) des Kanals (14) gegenüberliegt:

so dass die Achse (y), die durch die Mitte der kreisförmigen Basis (13) des Vorsprungs und senkrecht zu dem Träger (1) verläuft, und die Achse (t), die durch die Mitte der oberen Öffnung (15) des Kanals (14) und senkrecht zu dem Träger (1) verläuft, zusammenfallen, oder sich voneinander durch einen Abstand kleiner als oder gleich [(Wert von d4 - Wert von d3)/2] unterscheiden, wenn der Wert von d4 größer ist als der Wert von d2.

**14.** Verfahren zur Herstellung eines mikrofluidischen Zellkulturchips nach den Ansprüchen 1 bis 6, wobei die Herstellung der mittleren Einheit (105) des mittleren Moduls (104) Folgendes umfasst:

- einen Schritt der Extrusion einer resorbierbaren Polymerlösung (22) durch die mindestens eine Perforation (4) des Trägers (1), um mindestens eine 3D-Nanostruktur (23) auf der Seite der oberen Seite (2) des Trägers (1) zu bilden, wobei die mindestens eine Nanostruktur insbesondere kuppelförmig ist, gefolgt von

- einem Schritt der Polymerisierung der resorbierbaren Polymerlösung, um die mindestens eine 3D-Nanostruktur (23) zu versteifen, wobei die mindestens eine 3D-Nanostruktur (23) nach der Polymerisierung eine Form aus resorbierbarem Polymer (22) auf der Seite der oberen Seite des Trägers bildet, gefolgt von

- einem Schritt des Aufbringens mindestens einer kontinuierlichen Schicht aus mindestens einem Polyelektrolyten, der die obere Seite (2) des Trägers (1) und die Oberfläche der resorbierbaren Polymerform (22) bedeckt, um die poröse 3D-nanostrukturierte Membran (5) zu bilden, von

- einem Schritt des Auflösens der Form aus resorbierbarem Polymer, um die untere Seite (7) der porösen, 3D-nanostrukturierten Membran (5), die auf feste Weise auf der oberen Seite (2) des Trägers (1) positioniert ist, und die Innenseite (10) des mindestens einen Vorsprungs (8), der vollständig gegenüber der mindestens einen Perforation (4) positioniert ist, zu erhalten,

wobei das resorbierbare Polymer (22) insbesondere aus Chitosan, Agarose und Alginat ausgewählt ist.

**15.** Verfahren zur Herstellung eines mikrofluidischen Zellkulturchips nach Anspruch 1, wobei die obere Seite (6) der 3D-nanostrukturierten Membran (5) des mikrofluidischen Chips fest auf der unteren Seite des Trägers (1) positioniert ist, wobei die Herstellung des mittleren Moduls, das die mittlere Einheit umfasst, Folgendes umfasst:

(i) einen Schritt des Zusammenfügens eines Stützteils (I, i), das aus einem seitlichen Rahmen (213), einer offenen obere Seite (214) und einer massiven unteren Seite (215) besteht, die einen Ausschnitt (216) im Format der mittleren Einheit umfasst, und einer Form (H, H1, H2, h1, h2) mit den Formen und Abmessungen des Stützteils (I, i), umfassend mindestens eine 3D-Nanostruktur (208), die auf der oberen Seite (209) geformt ist, wobei die Form insbesondere aus Kunststoff besteht,

(ii) einen Schritt des Gießens einer resorbierbaren Polymerlösung (22) auf die obere Seite (209) der Form (H, H1, H2, h1, h2), (Fig. 61), gefolgt von

(iii) einem Schritt der Polymerisierung der resorbierbaren Polymerlösung (22), um die resorbierbare Polymerlösung zu versteifen und eine resorbierbare Polymermatrix (210) zu bilden, die mindestens eine Negativform der mindestens einen 3D-Nanostruktur (211) aufweist, gefolgt von

(iv) einem Schritt des Entfernens der Form (H, H1, H2, h1, h2), um die resorbierbare Polymermatrix (210) zu erhalten, die mindestens eine Negativform der mindestens einen 3D-Nanostruktur (211) umfasst, die in dem Ausschnitt (216) der massiven unteren Seite (215) des Stützteils (I, i) ausgebildet ist, gefolgt von

(v) einem Schritt des Zusammenfügens des Stützteils (I, i) mit einem perforierten Teil (G, G1, G2, g1, g2), das einen Träger umfasst, der aus einer nicht resorbierbaren Membran (1) besteht, die durch mindestens eine Perforation (4) perforiert ist, die in einen Sockel (106) integriert ist, wobei das perforierte Teil (G, G1, G2, g1, g2) die Formen und Abmessungen des Stützteils (I, i) aufweist und die Anzahl der Perforationen (4) des perforierten Teils (G, G1, G2, g1, g2) identisch ist mit der Anzahl der geformten 3D-Nanostrukturen (211) in der in Schritt (i) verwendeten Form (H, H1, H2, h1, h2),

solcherart, dass die mindestens eine Perforation (4) des Trägers (1) mit der mindestens einen Negativform der mindestens einen 3D-Nanostruktur (211) fluchtet,

gefolgt von

(vi) einem Schritt des Aufbringens mindestens einer kontinuierlichen Schicht mindestens eines Polyelektrolyten auf die kontinuierliche Oberfläche, die aus der unteren Seite (3) des Trägers (1) und der unteren Seite (212) der Matrix aus resorbierbarem Polymer (210) besteht, die mindestens eine Negativform der mindestens einen 3D-Nanostruktur (211) auf der Ebene der mindestens einen Perforation (4) des Trägers (1) umfasst,

um eine 3D-nanostrukturierte poröse Membran (5) zu bilden, die mindestens einen Vorsprung (8) umfasst, gefolgt von (vii) einem Schritt des Auflösens der Matrix aus resorbierbarem Polymer (210), die mindestens eine Negativform der mindestens einen 3D-Nanostruktur (211) umfasst,

(viii) einen Schritt des Entfernens des Stützteils (I, i), um das perforierte Teil (G, G1, G2, g1, g2) zu erhalten, das auf seiner unteren Seite (3) eine poröse, 3D-nanostrukturierte Membran (5) und auf der Seite seiner oberen Seite den mindestens einen Vorsprung umfasst.

**Claims**

**1.** Microfluidic cell culture chip that contains a central module (104) comprising:

- a central unit (105), which contains

-- a support consisting of a non-absorbable membrane (1), including an upper face (2) and a lower face (3), perforated by at least one perforation (4), said support (1) being composed in particular of bioprinted plastic, polycarbonate, tissue culture plastic, glass or SU8,

-- a 3D nanostructured porous membrane (5), including an upper face (6) and a lower face (7), and including at least one protrusion (8), said at least one protrusion being hollow and including an outer face (9) and an inner face (10) and forming a structure in relief on the upper face (6) side of the 3D nanostructured membrane (5), said protrusion being in particular in the shape of a hollow dome having a circular base (13),

oo said lower face (7) of said 3D nanostructured porous membrane (5) being positioned in a rigidly connected manner on said upper face (2) of said support (1),

oo or said upper face (6) of the 3D nanostructured membrane (5) being positioned in a rigidly connected manner on said lower face (3) of said support (1),

oo and said 3D nanostructured porous membrane (5) and the at least one protrusion (8) being composed of materials suitable for the culture of two types of distinct cells;

- a base (106),

said central unit (105) being integrated into said base (106), and forming a whole with said base (106).

**2.** Microfluidic cell culture chip according to claim 1, said lower face (7) of said 3D nanostructured porous membrane (5) being positioned in a rigidly connected manner on said upper face (2) of said support (1), wherein said at least one perforation (4) of the support (1) has

○ a cross-section (11) having a circular shape at the upper face of the support (1) (upper cross-section of the perforation) having a diameter d1 (upper diameter d1 of the perforation) and an axis (x) passing through the centre of said upper cross-section (11) of the perforation (4) and perpendicular to said support (1),

○ and a cross-section (12) having a circular shape at the lower face (3) of the support (1) (lower cross-section of the perforation) having a diameter d2 (lower diameter d2 of the perforation) and an axis (w) passing through the centre of said lower cross-section of the perforation and perpendicular to said support (1),

○ wherein said axes (x) and (w) are the same, the diameter d1 having from a value greater than or equal to $10 \mu m$ to a value less than or equal to $500 \mu m$, and the diameter d2 having from a value greater than or equal to $10 \mu m$ to a value less than or equal to $500 \mu m$,

such that the value of the diameter d2 is greater than or equal to the value of the diameter d1, and wherein said circular base (13) of said protrusion (8) as a diameter d3 (diameter d3 of the circular base of the protrusion), said diameter d3 of the protrusion (8) having from a value of $10 \mu m$ to a value of $500 \mu m$, and having a value less than, equal to or greater than d1, and said protrusion has a height of 1 to 600$\mu$m, more particularly of 1 to 300$\mu$m, more particularly of 50 to 300$\mu$m, more particularly of 50$\mu$m.

3.  Microfluidic cell culture chip according to claims 1 and 2, wherein said inner face (10) of said at least one protrusion (8) is totally or partly facing said at least one perforation (4) of said support (1), and in particular totally facing said at least one perforation (4) of said support:

    - when the value of the upper diameter d1 of said at least one perforation (4) is greater than or equal to the value of the diameter d3 of said at least one protrusion (4), and the axis (y), passing through the centre of said circular base (13) and perpendicular to said support (1), and the axis (x), passing through the centre of the upper cross-section (11) of said perforation (4) and perpendicular to said support (1), are the same; or
    - when the value of the upper diameter d1 of said at least one perforation (4) is greater than the value of the diameter d3 of said at least one protrusion (8), and the axis (y), passing through the centre of said circular base (13) and perpendicular to said support (1), and the axis (x), passing through the centre of the upper cross-section (11) of said perforation (4) and perpendicular to said support (1), are distinct from one another by a distance having a value less than or equal to [(value of d1 - value of d3)/2].

4.  Microfluidic cell culture chip according to claims 1, 2 and 3, wherein the outer face (9) of said protrusion (8) supports at least one adherent cell, said at least one adherent cell being in particular a stromal cell, and wherein the inner face (10) of said protrusion (8) supports at least one adherent cell, said at least one adherent cell being in particular an epithelial cell, and more particularly, said outer face (9) of said protrusion (8) supports a first set of adherent cells in the confluence stage, said first set being a set of stromal cells, and said inner face (10) of said protrusion (8) supports a second set of adherent cells in the confluence stage, said second set being a set of epithelial cells.

5.  Microfluidic cell culture chip according to claims 1 to 4, including at least two protrusions (8) and wherein the distance between two contiguous protrusions has a value of l, said value 1 being from 10 to 100um, more particularly from 50 to 100$\mu$m,

    and a maximum number of protrusions (8) for a surface area of the central unit of 1cm$^2$ equal to the formula

$$\frac{\pi\,(d3/2)^2}{(1+d3)-\pi\,(d3/2)^2}$$

    where l is the distance between two contiguous protrusions (8), said value l being from 10 to 100$\mu$m, more particularly from 50 to 100$\mu$m, and where d3 is the diameter of the circular base (13) of said protrusion (8).

6.  Microfluidic cell culture chip according to one of claims 1 to 5, wherein the 3D nanostructured porous membrane (5) consists of at least one layer of polyelectrolyte chosen from poly(sodium 4-styrenesulphonate) (PSS), poly(ethyleneimine), poly(diallyldimethylammonium chloride), poly(acrylamide-co-diallyldimethylammonium chloride), diallyldimethylammonium chloride, poly(allylamine hydrochloride) (PAH), polyanetholesulfonic acid, polyacrylic acid, polystyrene-alt-maleic acid, polyvinyl sulphate, polyvinyl sulfonic acid, poly(2-acrylamido-2-methyl-1-propanesulfonic) acid, poly(2-acrylamido-2-methyl-1-propanesulfonic-co-acrylonitrile acid), poly 4-styrenesulfonic acid, poly(4-styrenesulfonic acid-co-maleic acid), 4-styrenesulfonic sodium salt hydrate,

    and wherein when said 3D nanostructured porous membrane (5) consists of at least two successive layers, one layer consisting of a positively charged polyelectrolyte alternates with a layer consisting of a negatively charged electrolyte, said 3D nanostructured porous membrane (5) in particular consisting of 1 to 150 successive layers of polyelectrolyte, and more particularly said 3D nanostructured porous membrane (5) consisting of 15 successive layers of poly(sodium 4-styrenesulphonate) (PSS) and/or of poly(allylamine hydrochloride) (PAH), alternating with one another.

7.  Microfluidic cell culture chip according to claims 1 to 6, which contains a solid lower module (107), including

    - a lower unit (108)
    which contains an upper face, a lower face and at least one lateral face, and comprising at least one channel (14) having a tubular shape including an upper orifice (15) having a diameter d4 having an axis (t) passing through the centre of said upper orifice (15) and perpendicular to said support (1), and a lower orifice having a diameter d5 (16),
    - a base (109),
    said lower unit (108) being integrated into said base (109), and forming a whole with said base (109), said lower unit (108) and said base (109) are composed in particular of bioprinted plastic, polycarbonate, tissue culture plastic or SU8, said value of the diameter d4 of said upper orifice (15) of said channel (14) being greater than or equal to the value of the diameter d5 of said lower orifice (16) of said channel (14) and said value of the diameter d4 being greater than or equal to the value of the lower diameter d2 of said at least one perforation (4), said upper face of said lower unit (108) including said upper orifice (15) of the at least one channel, and said lower orifice (16) opening, itself or via intermediate means (17), outside of said base (109).

8. Microfluidic cell culture chip according to claims 1 to 7, wherein said lower module (107) and said central module (104) are assembled such that the upper orifice (15) of the at least one channel (14), of said upper face of said lower unit (108), opens onto at least one of said perforations (4) of said support (1) of said central unit (105), and the inner orifice (16) of said at least one channel (14) opens itself outside of the chip, or via an intermediate means consisting of a tank (17) capable of recovering liquids and allowing a transfer to outside of the chip via an outlet channel (18) opening outside of the base (109) of said lower module (107),

   said upper face of said lower unit (108) and said lower face of said support of said central unit (105) having a shape and a surface identical to each other, and said lower unit (108) and said central unit (105) being assembled by fastening elements (204) located respectively on each of the bases of the lower module (109) and of the central module (106), so as to assemble in a sealed manner said upper face of said lower unit (108) and said lower face of said support (3) of said central unit (105).

9. Microfluidic cell culture chip according to claims 1 to 8, wherein said upper orifice (15) of said at least one channel (14) opens onto a single perforation (4) of said support (1), and wherein said perforation (4) of said support (1) is totally facing said upper orifice (15) of said at least one channel (14):

   - when the value of the lower diameter d2 of said perforation (4) is equal to the value of the diameter d4 of said upper orifice (15) of said channel (14), and the axis (w), passing through the centre of said lower cross-section (12) of the perforation (4) and perpendicular to said support (1), and the axis (t), passing through the centre of said upper orifice (15) of said channel (14) and perpendicular to said support (1), are the same,

or
   - when the value of the lower diameter d2 of said perforation (4) is less than the value of the diameter d4 of said upper orifice (15) of said channel (14), and the axis (w), passing through the centre of said lower cross-section (12) of the perforation (4) and perpendicular to said support (1), and the axis (t), passing through the centre of said upper orifice (15) of said channel (14) and perpendicular to said support (1), are the same, or distinct from one another by a distance having a value less than or equal to [(value of d4 - value of d2)/2].

10. Microfluidic cell culture chip according to claims 1 to 9, wherein said lower module (107) includes at least two channels (14), and wherein each of the upper orifices (15) of the at least two channels (14) opens onto a perforation (4)

    - and the at least two lower orifices (16) of the at least two channels (14) open outside of the chip respectively at at least two distinct locations,
    - or the at least two lower orifices (16) of all of the at least two channels (14) open onto the same intermediate means consisting of a tank (17) capable of recovering liquids and allowing a transfer to outside of the chip via an outlet channel (18) opening outside of the base (109) of said lower module (107).

11. Microfluidic cell culture chip according to claims 1 to 6, which contains an upper module (101) comprising:

    - a solid and hollow upper unit (102), which consists of a solid surface defining an open volume (chamber) (203), and the edges of the solid surface being capable of being in contact with said central unit (105) of said central module (104) and defining the surface of the opening of said open volume;
    - and a base (103), said upper unit (102) being integrated into said base (103), and forming a whole with said base (103), and wherein said upper unit (102) and said base (103) are in particular composed of bioprinted plastic, polycarbonate, tissue culture plastic or SU8.

12. Microfluidic cell culture chip according to claims 1 to 6 and according to claim 11, wherein said upper unit (102) of said upper module (101) and said central unit (105) of said central module (104) are assembled so that said surface of the opening of said upper unit (102) is positioned above said upper face (6) of the 3D nanostructured porous membrane (5) of said central unit (105),

to form a closed space defined by said solid surface of said upper unit (102) and said upper face (6) of the 3D nanostructured porous membrane (5),

said surface of the opening and said upper face (6) having a shape and a surface identical to each other,

said upper unit (102) of said upper module (101) and said central unit (105) of said central module (104) being assembled by fastening elements (201, 204) located on each of the bases (103, 106) of the upper module (101) and of the central module (104), so as to assemble in a sealed manner said surface of the opening of said upper unit (102) and said upper face (6) of the 3D nanostructured porous membrane (5) of said central unit (105),

and advantageously said at least one solid face of said upper unit (102) has two orifices opening respectively onto an inlet/outlet channel (202), allowing said closed space to communicate with the outside of said microfluidic chip via said inlet/outlet channels (202) which open outside of the base (109).

13. Microfluidic cell culture chip according to claims 1 to 6 and according to claims 11 and 12, including an upper module (101), a central module (104) and a lower module (107), and wherein the aforementioned modules are assembled such that

said surface of the opening of said upper unit (102) of said upper module (101) is positioned above said upper face (2) of the 3D nanostructured porous membrane (5) of said central unit (105) of said central module (104),

and the upper orifice (15) of the at least one channel (14) of said upper face of said lower unit (108) of said lower module (107) opens onto at least one of said perforations (4) of said support (1) of said central unit (105),

said surface of the opening of said upper unit (102) and said upper face (2) of the 3D nanostructured porous membrane (5) of said central unit (105) having a shape and a surface identical to each other,

said upper face of said lower unit (108) and said lower face of said support (3) of said central unit (105) having a shape and a surface identical to each other,

and said upper module (101), said central module (104) and said lower module (107) being assembled by fastening elements (201, 204) located on each of the respective bases (103, 106, 109)

in particular wherein said inner face (10) of said protrusion (8) is totally facing said upper orifice (15) of said channel (14):

o when said inner face (10) of said at least one protrusion (8) is totally facing said at least one perforation (4) of said support (1):

- such that when the value of the upper diameter d1 of said at least one perforation (4) is greater than or equal to the value of the diameter d3 of said at least one protrusion (4), and the axis (y), passing through the centre of said circular base (13) and perpendicular to said support (1), and the axis (x), passing through the centre of the upper cross-section (11) of said perforation (4) and perpendicular to said support (1), are the same; or
- such that the value of the upper diameter d1 of said at least one perforation (4) is greater than the value of the diameter d3 of said at least one protrusion (8), and the axis (y), passing through the centre of said circular base (13) and perpendicular to said support (1), and the axis (x), passing through the centre of the upper cross-section (11) of said perforation (4) and perpendicular to said support (1), are distinct from one another by a distance having a value less than or equal to [(value of d1 - value of d3)/2];

and when said perforation (4) of said support (1) is totally facing said upper orifice (15) of said channel (14) :

oo such that the axis (y), passing through the centre of said circular base (13) of said protrusion (8) and perpendicular to said support (1), and the axis (t), passing through the centre of said upper orifice (15) of said channel (14) and perpendicular to said support (1), are the same, or distinct from one another by a distance less than or equal to [(value of d4 - value of d3)/2] when the value of d4 is greater than the value of d2.

14. Method for manufacturing a microfluidic cell culture chip according to claims 1 to 6, wherein the manufacturing of said central unit (105) of said central module (104) comprises:

- a step of extrusion of a solution of absorbable polymer (22), through the at least one perforation (4) of said support (1) to form at least one 3D nanostructure (23) on said upper face (2) side of said support (1), said at least one nanostructure being in particular in the shape of a dome, followed by

- a step of polymerisation of said solution of absorbable polymer to rigidify said at least one 3D nanostructure (23), said at least one 3D nanostructure (23) forming a mould made of absorbable polymer (22) on said upper face side of said support after polymerisation, followed by

- a step of applying at least one continuous layer of at least one polyelectrolyte covering the upper face (2) of said support (1) and the surface of said mould made of absorbable polymer (22), to form the 3D nanostructured porous membrane (5), by

- a step of dissolving said mould made of absorbable polymer to obtain said lower face (7) of said 3D nanostructured porous membrane (5) positioned in a rigidly connected manner on said upper face (2) of said support (1) and said inner face (10) of the at least one protrusion (8) positioned totally facing said at least one perforation (4),

said absorbable polymer (22) being in particular chosen from chitosan, agarose and alginate.

15. Method for manufacturing a microfluidic cell culture chip according to claim 1, said upper face (6) of the 3D nanostructured membrane (5) of the microfluidic chip being positioned in a rigidly connected manner on said lower face (3) of said support (1), wherein the manufacturing of the central module comprising the central unit comprises:

(i) a step of assembling a support part (I, i), consisting of a lateral frame (213), of an open upper face (214) and of a solid lower face (215) including a cutout (216) in the format of the central unit, and a mould (H, H1, H2, h1, h2), having the shapes and dimensions of said support part (I, i), comprising at least one moulded 3D nanostructure (208) on the upper face (209), said mould being in particular made of plastic,

(ii) a step of casting a solution of absorbable polymer (22) on said upper face (209) of said mould (H, H1, H2, h1, h2), (figure 61) followed by

(iii) a step of polymerisation of said solution of absorbable polymer (22) to rigidify said solution of absorbable polymer and form a matrix made of absorbable polymer (210) including at least one negative mould of said at least one 3D nanostructure (211), followed by

(iv) a step of removing said mould (H, H1, H2, h1, h2), to obtain said matrix made of absorbable polymer (210) including at least one negative mould of said at least one 3D nanostructure (211) formed in said cutout (216) of the solid lower face (215) of said support part (I, i), followed by

(v) a step of assembling said support part (I, i), with a perforated part (G, G1, G2, g1, g2), comprising a support consisting of a non-absorbable membrane (1) perforated by at least one perforation (4), integrated into a base (106),

said perforated part (G, G1, G2, g1, g2) having the shapes and dimensions of said support part (I, i), and the number of perforations (4) of said perforated part (G, G1, G2, g1, g2) being identical to the number of moulded 3D nanostructures (211) in said mould (H, H1, H2, h1, h2) used in step (i),

so that said at least one perforation (4) of said support (1) is alignment with said at least one negative mould of said at least one 3D nanostructure (211),

followed by

(vi) a step of applying at least one continuous layer of at least one polyelectrolyte onto the continuous surface consisting of the lower face (3) of said support (1) and of the lower face (212) of said matrix made of absorbable polymer (210) including at least one negative mould of said at least one 3D nanostructure (211) at said at least one perforation (4) of said support (1),

to form a 3D nanostructured porous membrane (5) including at least one protrusion (8), followed by

(vii) a step of dissolving said matrix made of absorbable polymer (210) including at least one negative mould of said at least one 3D nanostructure (211),

(viii) a step of removing the support part (I, i) to obtain said perforated part (G, G1, G2, g1, g2) comprising on its lower face (3) a 3D nanostructured porous membrane (5), and on its upper face side said at least one protrusion.

## Figure 1

## Figure 2

## Figure 3

## Figure 4

## Figure 5

## Figure 6

## Figure 7

## Figure 8

**Figure 9**

**Figure 10**

## Figure 11

## Figure 12

## Figure 13

*(x)*

*(w)*

## Figure 14

*(y)*

*(t)*

## Figure 15

## Figure 16

## Figure 17

## Figure 18

## Figure 19

## Figure 20

## Figure 21

## Figure 22

EP 3 455 342 B1

Figure 23

Figure 24

66

## Figure 25

## Figure 26

## Figure 27

## Figure 28

## Figure 29

## Figure 30

## Figure 31

# Figure 32

## Figure 33

## Figure 34

Figure 35

## Figure 36

## Figure 37

## Figure 38

## Figure 39

**104**

204

204

**105**

**106**

207

204

204

## Figure 40

**105**

**8**

**106**

EP 3 455 342 B1

## Figure 41

107
205
201
201
109
206
108
201
201

## Figure 42

202
107
101
104
205

78

## Figure 43

## Figure 44

**Figure 45**

**Figure 46**

**Figure 47**

**Figure 48**

## Figure 49

## Figure 50

## Figure 51

## Figure 52

24

8

**Figure 53**

**Figure 54**

## Figure 55

## Figure 56

## Figure 57

## Figure 58

## Figure 59

## Figure 60

## Figure 61

## Figure 62

211
210
212
I

## Figure 63

211
210
I
G
1
2
3
4

## Figure 64

## Figure 65

## Figure 66

## Figure 67

**Figure 68**

F :

214

216

215

213

217

I

106

4

G2

208

218

H2

**Figure 69**

## Figure 70

## Figure 71

A

B

Gl

F

C

D

## Figure 72

218 · g1 4 106

208 214 216 215

218 213

218 i 217

h1

# Figure 73

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8647861 B2,  de Ingber

- US 20140038279 A1

**Littérature non-brevet citée dans la description**

- **KIM et al.** Gut-on-a-Chip microenvironment induces human intestinal cells to undergo villus differenciation. *Integr. Biol.,* 2013, vol. 5, 1130

- **MARCH et al.** *Differentiating Intestinal Stem Cells in a 3D Niche,* 2014